# EUROPEAN PATENT SPECIFICATION

(11) **EP 4 076 245 B1**
(45) Date of publication and mention of the grant of the patent: **27.05.2026**
(21) Application number: 20839237.3
(22) Date of filing: 15.12.2020
(51) Int. Cl.: A61B 18/24, A61B 18/26, A61B 18/00

(54) **CATHETER SHEATH FOR ACOUSTICALLY AMPLIFYING LASER-INDUCED PRESSURE WAVES**
KATHETERHÜLLE ZUR AKUSTISCHEN VERSTÄRKUNG VON LASERINDUZIERTEN DRUCKWELLEN
GAINE DE CATHÉTER POUR AMPLIFICATION ACOUSTIQUEMENT D'ONDES DE PRESSION INDUITES PAR LASER

(30) Priority: 20.12.2019 US 201962951331 P
(43) Date of publication of application: 26.10.2022
(73) Proprietor: Koninklijke Philips N.V., 5656 AE Eindhoven (NL)
(72) Inventor: TSCHIDA, Adam, 5656 AE Eindhoven (NL)
(74) Representative: Philips Intellectual Property & Standards
(86) International application number: PCT/EP2020/086059
(87) International publication number: WO 2021/122491

(56) References cited:
- US-A1- 2015 105 714
- US-A1- 2017 333 132
- US-A1- 2019 346 649

## Description

### FIELD

The present disclosure relates generally to the use of medical devices for the treatment of vascular conditions. In particular, the present disclosure provides materials and systems for using laser-induced pressure waves to disrupt vascular blockages and to deliver therapeutic agents to the blockage area.

### BACKGROUND

Arterial disease is a common disease that affects millions of Americans. Coronary artery disease (CAD) most often results from a condition known as atherosclerosis, which generally manifests as the accumulation of a waxy substance on the inside of a subject's coronary arteries. This substance, called plaque, is made of cholesterol, fatty compounds, calcium, and a blood-clotting material called fibrin. Similarly, peripheral artery disease (PAD) often results from the accumulation of plaque on the inside of a subject's peripheral arteries, such as the arteries in a patient's arms, hands, legs and/or feet.

As the plaque builds up in either coronary arteries, peripheral arteries and other arteries, the corresponding artery narrows and/or becomes stenotic, thereby making it more difficult for blood to flow through the arteries. As the size of the stenosis increases and the blockage worsens, blood flow slows and upon the formation of a total vascular occlusion, blood flow through the corresponding artery completely stops, which in turn may cause pain in the extremities and, in severe cases, gangrene, which may ultimately require amputation.

Balloon angioplasty and other transluminal medical treatments are well-known and have been proven efficacious in the treatment of stenotic lesions at the core of CAD and/or PAD, as long as the artery is only partially blocked and not totally blocked. In a typical angioplasty procedure to treat CAD, a catheter is inserted into the groin or arm of a subject and guided forward through the aorta and into the coronary arteries of the heart. The angioplasty catheter includes a balloon, which when placed within the partial occlusion, can be inflated, thereby dilating the obstruction or restriction and increasing the size of the diameter of the artery to provides more typical blood flow therethrough.

Over time, a vascular occlusion, particularly a total occlusion, may calcify and/or becomes fibrous, thereby decreasing the balloon's ability to dilate the vascular occlusion. Certain types of catheters, such as electrically-induced shockwave balloon catheters, may be used to break the calcified tissue. An electrically-induced shockwave balloon catheter may include a liquid filled balloon and a one or more pairs of electrodes within the balloon. Upon creating a discharge across the electrodes, plasma is produced, which results in the formation of one or more vapor bubbles. The vapor bubbles created within the balloon cause the balloon to expand and contract. The expansion and contraction of the balloon creates a hydraulic force that transfers energy to the vascular occlusion and/or to the walls of the vessel in an amount sufficient to disrupt intraluminal calcium as well as calcium within the tissue layer of the vasculature (for example, calcium deposits). In addition to producing vapor bubbles being upon the formation of plasma generated by the electrical reaction in the liquid, shockwaves are also produced. The shockwaves are transferred through the balloon and to the calcified vascular occlusion, and the shockwaves modify the calcified occlusion.

In the event a total stenotic occlusion forms, it may be difficult for the balloon to enter the stenosis. Additionally, if a total occlusion calcifies and/or become fibrous, thereby increasing the hardness of occlusion, it may become even more difficult, if not impossible, to penetrate the occlusion and insert a balloon catheter. For example, the proximal and/or distal ends of the occlusion may become calcified to the point that "caps" or "calcified caps" are created, such that even an electrically-induced shockwave balloon catheter may be unable to penetrate the calcified total occlusion because the balloon must be within and adjacent the occlusion in order to operate. And because the balloon within an electrically-induced shockwave balloon catheter is typically proximal the distal end of the electrically-induced shockwave balloon catheter, it is unable to be inserted into or through the calcified cap of the total occlusion.

It is also known to use a laser catheter surrounded by a sheath to create a laser-induced pressure waves to disrupt the calcified cap or calcified tissue within the blood vessel to increase vessel compliance. Using a laser catheter to create the pressure waves for disrupting calcium, however, has its challenges, particularly when using a laser catheter to create pressure waves in smaller-sized blood vessels, such as the peripheral vasculature below a patient's knee. Again, it is desirable for the laser catheter to emit a sufficient amount of energy, which will translate into creating a predetermined pressure wave that will disrupt the calcium in the blood vessel itself or the various blood vessel layers. But smaller-sized blood vessels require a laser catheter having a reduced diameter for vessel entry, and the smaller-sized laser catheters may have limitations as to the amount of energy that can transfer. That is, the reduced sized laser catheters may have less optical fibers or smaller diameter optical fibers in comparison to larger sized laser catheters, thereby limiting the amount of energy that the reduced sized laser catheters can transfer before causing damage to the optical fibers. That is, if the optical fibers attempt to transfer too much energy, they will be damaged.

One way to compensate for the reduction in energy transferred through and emitted by the laser catheter includes increasing the amount of contrast in the liquid medium within the outers sheath. Transmitting pulses of light energy into the liquid medium produces vapor bubbles. Upon emitting light from an emitter, such as a laser catheter, within a sheath that contains an absorptive liquid medium, vapor bubbles may be produced within the interior of the sheath and/or exterior to the sheath. Assuming that the vapor bubbles are created on the interior of the sheath, it may be desirable to limit some or all of the potential expansion of the relevant portion of the sheath caused by the vapor bubbles. That is, it may be desirable to reduce the size of the vapor bubbles created within the sheath so as to reduce or prevent the sheath from applying a hydraulic force or pressure to the vascular occlusion and/or to the walls of the vessel. Also, assuming that the vapor bubbles are created on exterior of the sheath within the vessel wall, it may be desirable to reduce and/or prevent the formation of such vapor bubbles so as to reduce or prevent the cavitation event and the formation of the vapor bubbles themselves from applying a hydraulic force or pressure to the vascular occlusion and/or to the walls of the vessel. But increasing the amount of contrast may create an undesirably sized vapor bubble, either within or on the outside of the sheath, thereby potentially damaging the blood vessel.

In other words, the size of the vapor bubble created is dependent at least in part on the degree of absorption of the light energy by the liquid medium as well as total energy deposited by the light source. Generally, the greater the absorption of the light energy by the liquid medium, the greater the force generated by the laser-induced pressure waves. Also, the greater the amount of the light energy delivered to the liquid medium, the greater the force generated by the laser-induced pressure waves. But if the liquid medium absorbs too much energy, an undesirably-sized vapor bubble will form and potentially damage the blood vessel. In US2015/0105714 devices and methods are discussed directed to the use of a low profile laser ablation catheter for use in laser ablation removal of arterial plaque blockages to restore blood flow in the treatment of arteriovenous fistulas. Also discussed are devices and methods directed to packaging, long term storage and sterilization of liquid core ablation catheters.

### SUMMARY

What is needed is a device and method for penetrating a calcified and/or fibrous vascular occlusion, particularly a calcified cap(s), and disrupting at least a portion of the vascular occlusion as the device penetrates and traverses the total occlusion. What is also needed is a device that is capable of delivering laser-induced pressure waves to the vascular occlusion in order to disrupt the calcified and/or fibrous portions without applying a hydraulic force thereto. These and other needs are addressed by the various aspects, embodiments, and configurations of the present disclosure. For example, the present disclosure discusses a sheath that has multiple layers to increase the reflective index of the pressure waves without increasing the size of the undesirable vapor bubble. The sheath has a composite structure that mimics anisotropic metamaterial to increase the amplitude of the pressure waves that transmit through the sheath.

The object of the present invention is solved by the subject-matter of the independent claim; further embodiments are incorporated in the dependent claims.

An example of a method for improving the compliance of a blood vessel within a subject comprises locating a calcified portion in the media of the blood vessel of the subject, positioning a laser catheter within the vasculature of the subject, the catheter comprising a proximal end a distal end, and at least one emitter disposed adjacent the distal end, positioning a sheath over the laser catheter within the vasculature of the subject, wherein the sheath comprises a proximal end and a distal end, wherein the sheath comprises a first layer forming a lumen, a plurality of pairs of attenuating members and intermediate layers radially exterior the first layer, wherein the attenuating members are constructed of a first material having a first thickness and first durometer, and the intermediate layers are constructed of a second material having a second thickness and a second durometer, wherein the first durometer is greater than the second durometer, wherein the first thickness and the second thickness are different, positioning the sheath within the vasculature such that the attenuating member is disposed adjacent a portion of the calcified portion in the media of the blood vessel, positioning the laser catheter within the vasculature such that the at least one emitter is positioned within the attenuating member and adjacent the portion of the calcified portion in the media of the blood vessel, introducing a liquid medium into the sheath and to the at least one emitter, and emitting a plurality of pulses of light energy from the at least one emitter into the liquid medium, wherein the plurality of pulses of light energy react with the liquid medium and generate a plurality of propagating laser-induced pressure waves that disrupt the calcified portion of media, thereby improving the compliance of the blood vessel.

Another example is the method of the previous paragraph further comprising the step of re-positioning the sheath such that the attenuating member is adjacent another calcified portion of the media.

Another example is the method of any of the previous two paragraphs further comprising the step of re-positioning the laser catheter within the sheath such that the one or more emitters is adjacent another calcified portion of the media.

Another example is the method of any of the previous three paragraphs, wherein the laser catheter is re-positioned within the attenuating member.

Another example is the method of any of the previous four paragraphs further comprising the step of re-positioning the laser catheter within sheath.

Another example is the method of any of the previous five paragraphs, wherein the laser catheter is re-positioned within the attenuating member.

Another example is the method of any of the previous six paragraphs further comprising the steps of removing the laser catheter from the vasculature and removing the sheath from the vasculature.

An example of a method for performing an atherectomy within a subject having a vasculature occlusion within the subject's vasculature comprises inserting a guidewire through a vascular occlusion within the vasculature, introducing a laser catheter into the vasculature and over the guidewire, wherein the laser catheter comprises at least one emitter, ablating at least a portion of the vascular occlusion with the laser catheter, introducing a sheath into the vasculature and over the laser catheter, wherein the sheath comprises a proximal end and a distal end, wherein the sheath comprises a first layer forming a lumen, a plurality of pairs of attenuating members and intermediate layers radially exterior the first layer, wherein the attenuating members are constructed of a first material having a first thickness and first durometer, and the intermediate layers are constructed of a second material having a second thickness and a second durometer, wherein the first durometer is greater than the second durometer, wherein the first thickness and the second thickness are substantially equal, positioning the sheath within the vasculature such that the attenuating member is disposed radially adjacent a calcified portion within the vasculature, positioning the laser catheter within the vasculature such that the at least one emitter is positioned within the attenuating member and radially adjacent the calcified portion, introducing a liquid medium into the sheath and to the at least one emitter, and emitting a plurality of pulses of light energy from the at least one emitter into the liquid medium, wherein the plurality of pulses of light energy react with the liquid medium and generate a plurality of propagating laser-induced pressure waves that disrupt the calcified portion.

Another example is the method of the previous paragraph, further comprising extending the laser catheter distally of the sheath and ablating another portion of a second vascular occlusion, positioning the sheath within the vasculature such that the attenuating member is disposed radially adjacent a second calcified portion of the second vascular occlusion, positioning the laser catheter within the vasculature such that the at least one emitter is positioned within the attenuating member and radially adjacent the second calcified portion, introducing a liquid medium into the sheath and to the at least one emitter, and emitting a plurality of pulses of light energy from the at least one emitter into the liquid medium, wherein the plurality of pulses of light energy react with the liquid medium and generate a plurality of propagating laser-induced pressure waves that disrupt the second calcified portion.

An example of a catheter system of the present disclosure comprises a laser catheter comprising a proximal end, a distal end and at least one emitter disposed adjacent the distal end, a sheath configured to be disposed over the laser catheter and configured to receive a liquid medium, the sheath comprising a proximal end and a distal end, wherein the sheath comprises a first layer forming a lumen, a plurality of pairs of attenuating members and intermediate layers radially exterior the first layer, wherein the attenuating members are constructed of a first material having a first thickness and first durometer, and the and the intermediate layers are constructed of a second material having a second thickness and a second durometer, wherein the first durometer is greater than the second durometer.

Another example is the catheter system of a previous paragraph, wherein the first thickness and the second thickness are different.

Another example is the catheter system of a previous paragraph, wherein the first thickness and the second thickness are substantially equal.

Another example is the catheter system of a previous paragraph, wherein a difference between the first durometer and the second durometer is about Shore 10D.

Another example is the catheter system of a previous paragraph, wherein a difference between the first durometer and the second durometer is about Shore 20D.

Another example is the catheter system of a previous paragraph, wherein a difference between the first durometer and the second durometer is about Shore 30D.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, Yi and Zₒ).

It is to be noted that the term "a" or "an" entity refers to one or more of that entity. As such, the terms "a" (or "an"), "one or more" and "at least one" can be used interchangeably herein. It is also to be noted that the terms "comprising," "including," and "having" can be used interchangeably.

The term "about" when used in conjunction with a numeric value shall mean plus and/or minus ten percent (10%) of that numeric value, unless otherwise specifically mentioned herein.

The term "attenuating member" as used herein is any component which alters a cavitation event, and/or vapor bubble. An example of an attenuating member is an element which minimally affects the laser induced pressure wave, yet alters the cavitation event and/or the vapor bubble. An example of an attenuating member is a porous attenuating member. The attenuating member, however, does not need to be porous and may include a solid configuration.

The term "catheter" as used herein generally refers to a tube that can be inserted into a body cavity, duct, lumen, or vessel, such as the vasculature system. In most uses, a catheter is a relatively thin, flexible tube ("soft" catheter), though in some uses, it may be a larger, solid, less flexible-but possibly still flexible-catheter ("hard" catheter). In some uses a catheter may contain a lumen along part or all of its length to allow the introduction of other catheters or guidewires. An example of a catheter is a sheath.

The term "balloon catheter" as used herein generally refers to the various types of catheters which carry a balloon for containing fluids. Balloon catheters may also be of a wide variety of inner structure, such as different lumen design, of which there are at least three basic types: triple lumen, dual lumen and co-axial lumen. All varieties of internal structure and design variation are meant to be included by use of the term "balloon catheter" herein. In some uses, balloon catheters can be used to perform angioplasty.

The term "cavitation event" as used herein describes the rapid fluid movement that leads to collapse of a vapor bubble to its smallest radius. In some cases, a cavitation event may include the generation of a pressure wave.

The terms "coupler" or "fiber optic coupler" refers to the optical fiber device with one or more input fibers or emitters and one or several output fibers or emitters. Fiber couplers are commonly special optical fiber devices with one or more input fibers or emitters for coupling these fibers or emitters to an energy source. The energy source can be another optical energy carrying fiber or emitter which is coupled to one or more additional fibers or emitters.

The term "emitter" as used herein refers to a fiber or an optical component (including any portion thereof, such as the end of a fiber) that emits light from a distal end of device, such as a catheter, towards a desired target. In some uses, this target can be tissue, or an absorptive media, such as contrast fluid. An emitter can be the output end of any device that transports light from an optical energy source to a target or treatment area. These optical energy transport devices can include glass or fused silica fiber optics, plastic fiber optics, air or gas light guides, and liquid light guides. As described herein, an emitter or emitters can be used to emit light of any wavelength. An emitter or emitters can emit light, including but not limited to, laser light, white light, visible light, infrared light, and ultraviolet light.

According to the present disclosure, the catheter contains at least one emitter, which may comprise glass or fused silica fiber optics, plastic fiber optics, air or gas light guides, and liquid light guides. Examples of a liquid light guide, or a catheter that contain a liquid light guide can be seen in U.S. Application Ser. No. 11/923,488, filed Oct 24, 2007, published as US 2009/0112198 A1, and U.S. Application Ser. No. 12/254,254, filed Oct 20, 2008, published as US 2009/0254074 A1.

The term "flexible structure" as used herein shall mean a structure that is able to bend or otherwise conform to the shape of the vasculature as it passes therethrough. The term "radial flexible structure" shall include a flexible structure that is also able to expand and/or contract in the radial direction upon a laser induced pressure wave passing therethrough.

A "laser emitter" as used herein refers to an end portion of a fiber or an optical component that emits laser light from a distal end of the catheter towards a desired target. In some uses, this target can be tissue, or an absorptive media, such as contrast fluid.

The term "laser-induced pressure wave" as used herein is a pressure wave caused by a reaction between laser light and an absorptive material. The laser-induced pressure wave may be generated in a gas, liquid (e.g., saline that may or may not include a contrast medium) or solid.

The term an "optical fiber" (or laser active fiber) as used herein refers to a flexible, transparent fiber made of an optically transmissive material, such as glass (silica) or plastic, which functions as a waveguide, or "light pipe", to transmit light between the two ends of the fiber.

The term "porous attenuating member" as used herein shall mean an attenuating member constructed of a rigid member or semi-rigid member having openings therein. Examples of a rigid member and a semi-rigid member include a member constructed of coils, braids, laser-cut tubing, reinforced polymer extrusions, patterned plastics, metals and ceramics. Specific materials used to construct such rigid member and a semi-rigid member may include nitinol (which is a nickel-titanium alloy), stainless steel, titanium, silver, aluminum, cobalt, chromium, nylon, pebax, silicone, urethane, polyethylene and derivatives, nylons, polytetrafluoroethylene and derivatives, polyethylene terephthalate, polypropylene, poly(ether ether ketone), hydroxyapatite, alumina, tricalcium phosphate, silicates or other biocompatible metals, ceramics or polymers. Possible configurations for the porous attenuating member include, but are not limited to, spiral cuts, interrupted spiral cut, honeycomb, lattice structures as found commonly in vascular stents, slots, offset slots, helices, slits that are either longitudinal, radial, circumferential, or a combination thereof, openings that are shaped cutouts. The scope of this disclosure also encompasses the "porous attenuating member" being constructed of a flexible structure and/or a radial flexible structure, although it may be preferable for the attenuating to be constructed of a rigid member or semi-rigid member.

The term "rigid structure" as used herein shall mean a structure that is able to bend or otherwise conform to the shape of the vasculature as it passes therethrough but is substantially unable to expand and/or contract in the radial direction upon a laser induced pressure wave passing therethrough.

The term "semi-rigid structure" as used herein shall mean a structure that is partly rigid with an additional degree of flexibility as it passes through the vasculature but is substantially unable to expand and/or contract in the radial direction upon a laser induced pressure wave passing therethrough.

The term "sheath" as used herein generally refers to a tube that can be inserted into a body cavity duct, lumen, or vessel, such as the vasculature system that allows for the introduction of catheters and the introduction of fluid along its length. An example of a catheter that can be introduced into a sheath is a laser catheter. An example of fluid that can be introduced into a sheath is an absorptive fluid such as contrast. The sheath can have a closed end or an open end. Because the sheath is a tube that can be inserted into a body cavity, duct, lumen, or vessel, such as the vasculature system, the sheath may also be considered a catheter. Accordingly, a catheter, such as a laser catheter, can be introduced into another catheter.

The term "therapeutic agent" as used herein generally refers to any known or hereafter discovered pharmacologically active agent that provides therapy to a subject through the alleviation of one or more of the subject's physiological symptoms. A therapeutic agent may be a compound that occurs in nature, a chemically modified naturally occurring compound, or a compound that is chemically synthesized. The agent will typically be chosen from the generally recognized classes of pharmacologically active agents, including, but not necessarily limited to, the following: analgesic agents; anesthetic agents; antiarthritic agents; respiratory drugs, including antiasthmatic agents; anticancer agents, including antineoplastic drugs; anticholinergics; anticonvulsants; antidepressants; antidiabetic agents; antidiarrheals; antihelminthics; antihistamines; antihyperlipidemic agents; antihypertensive agents; anti-infective agents such as antibiotics and antiviral agents; antiinflammatory agents; antimigraine preparations; antinauseants; antiparkinsonism drugs; antipruritics; antipsychotics; antipyretics; antispasmodics; antitubercular agents; antiulcer agents; antiviral agents; anxiolytics; appetite suppressants; attention deficit disorder (ADD) and attention deficit hyperactivity disorder (ADHD) drugs; cardiovascular preparations including calcium channel blockers, CNS agents; beta-blockers and antiarrhythmic agents; central nervous system stimulants; cough and cold preparations, including decongestants; diuretics; genetic materials; herbal remedies; hormonolytics; hypnotics; hypoglycemic agents; immunosuppressive agents; leukotriene inhibitors; mitotic inhibitors; restenosis inhibitors; muscle relaxants; narcotic antagonists; nicotine; nutritional agents, such as vitamins, essential amino acids and fatty acids; ophthalmic drugs such as antiglaucoma agents; parasympatholytics; psychostimulants; sedatives; steroids; sympathomimetics; tranquilizers; and vasodilators including general coronary, peripheral and cerebral.

The term "vapor bubble" as used herein is a gaseous cavity created within a liquid.

The terms "vasculature" and "vascular" as used herein refer to any part of the circulatory system of a subject, including peripheral and non-peripheral arteries and veins. Vasculature can be comprised of materials such as nucleic acids, amino acids, carbohydrates, polysaccharides, lipids fibrous tissue, calcium deposits, remnants of dead cells, cellular debris and the like.

The term "vascular occlusion" or "occlusion" refers to buildup of fats, lipids, fibrin, fibro-calcific plaque, thrombus and other atherosclerotic tissue within the lumen or within the intima of an artery that either narrows or completely obstructs the inner lumen the artery thereby restricting or blocking normal blood flow through the artery segment. The occlusion may partially or totally occlude the vasculature. Accordingly, the term "vascular occlusion" or "occlusion" shall include both a total occlusion and a partial occlusion. Alternatively, a vascular occlusion or occlusion may also be referred to as a vascular obstruction (or obstruction) or a vascular restriction (or restriction). A vascular obstruction may, therefore, be referred to as a total obstruction or a partial obstruction, and a vascular restriction may be referred to as a total restriction or a partial restriction.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 illustrates an exemplary ablation system, including a laser generator and a laser induced pressure wave emitting catheter sheath;
FIG. 2 is a perspective view of a laser catheter and a guidewire, according to one embodiment of the present disclosure;
FIG. 3A is a perspective view of a kit within the vasculature of a patient, wherein the kit includes a laser catheter radially disposed within a sheath and over a guidewire, according to one embodiment of the present disclosure, wherein the kit and guidewire are located proximally of a vascular occlusion;
FIG. 3B is a perspective view of a kit within the vasculature of a patient, wherein the kit includes a laser catheter radially disposed within a sheath and over a guidewire, according to one embodiment of the present disclosure, wherein the kit is located proximally of a vascular occlusion, and the guidewire has penetrated the vascular occlusion;
FIG. 3C is a perspective view of a kit within the vasculature of a patient, wherein the kit includes a laser catheter radially disposed within a sheath and over a guidewire, according to one embodiment of the present disclosure, wherein the sheath is located proximally of a vascular occlusion, and the laser catheter and guidewire have penetrated the vascular occlusion;
FIG. 3D is a perspective view of a kit within the vasculature of a patient, wherein the kit includes a laser catheter radially disposed within a sheath and over a guidewire, according to one embodiment of the present disclosure, wherein the kit and guidewire have penetrated the vascular occlusion;
FIG. 4 is a representative flow diagram of a method of treating a subject using a laser catheter and sheath, according to an example;
FIG. 5 is a perspective view of an outer sheath comprising an attenuating member, according to one embodiment of the present disclosure;
FIG. 5A is a side elevation view of an attenuating member comprising a plurality of square-shaped openings, according to one embodiment of the present disclosure;
FIG. 5B is a side elevation view of an attenuating member comprising a plurality of diamond-shaped openings, according to one embodiment of the present disclosure;
FIG. 5C is a side elevation view of an attenuating member comprising a plurality of openings formed by a helical structure wound in a particular direction, according to one embodiment of the present disclosure;
FIG. 5D is a side elevation view of an attenuating member comprising a plurality of openings formed by a helical structure wound in a particular direction, according to one embodiment of the present disclosure;
FIG. 5E is a side elevation view of an attenuating member comprising a plurality of openings formed by a helical wound ribbons, according to one embodiment of the present disclosure;
FIG. 5F is a side elevation view of an attenuating member comprising a plurality of hexagon-shaped openings, according to one embodiment of the present disclosure; and
FIG. 6 is a representative flow diagram of a method of treating a subject using a laser catheter and sheath, according to an example.
FIG. 7 is a cross-sectional view of an arterial wall taken along a direction perpendicular to the longitudinal axis of the arterial wall.
FIG. 7A is reduced version of the cross-sectional view of the arterial wall in FIG. 7.
FIG. 8A is a longitudinal-sectional view of a healthy arterial wall taken along a direction parallel to the longitudinal axis of the arterial wall.
FIG. 8B is a longitudinal-sectional view of an arterial wall taken along a direction parallel to the longitudinal axis of the arterial wall, wherein the arterial wall includes fat and/or lipids.
FIG. 8C is a longitudinal-sectional view of an arterial wall taken along a direction parallel to the longitudinal axis of the arterial wall, wherein the arterial wall includes plaque and calcium in the intima.
FIG. 8D is a longitudinal-sectional view of an arterial wall taken along a direction parallel to the longitudinal axis of the arterial wall, wherein the arterial wall includes calcified plaque and lipids in the intima.
FIG. 8E is a longitudinal-sectional view of an arterial wall taken along a direction parallel to the longitudinal axis of the arterial wall, wherein the arterial wall has ruptured.
FIG. 8F is a longitudinal-sectional view of an arterial wall taken along a direction parallel to the longitudinal axis of the arterial wall, wherein the artery includes an occlusion.
FIG. 8G is a longitudinal-sectional view of an arterial wall taken along a direction parallel to the longitudinal axis of the arterial wall with a laser catheter removing the occlusion depicted in FIG. 8F.
FIG. 8H is a longitudinal-sectional view of an arterial wall taken along a direction parallel to the longitudinal axis of the arterial wall with a sheath and laser catheter located adjacent the remaining portion of the vascular occlusion depicted in FIG. 8G, wherein the end of the laser catheter, particularly its emitter(s) is disposed distally of the most distal end of the sheath.

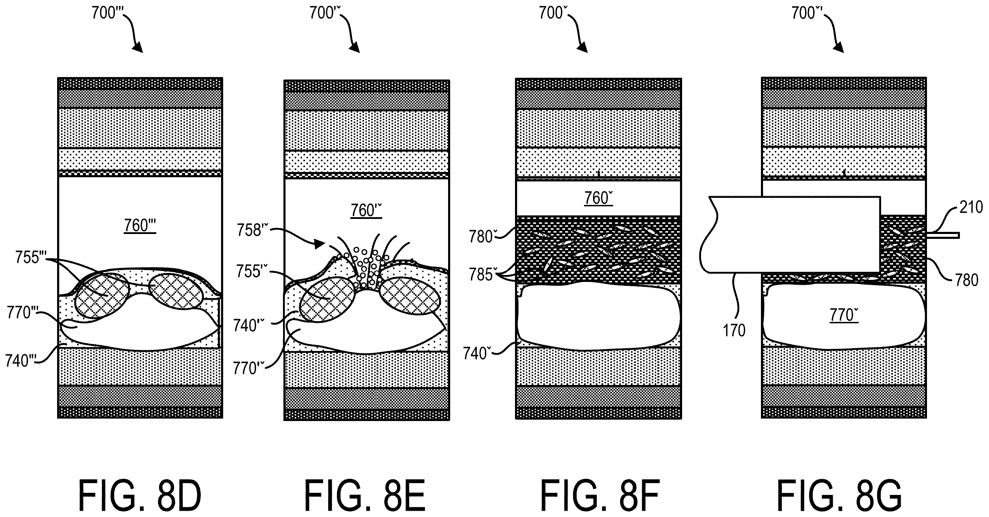

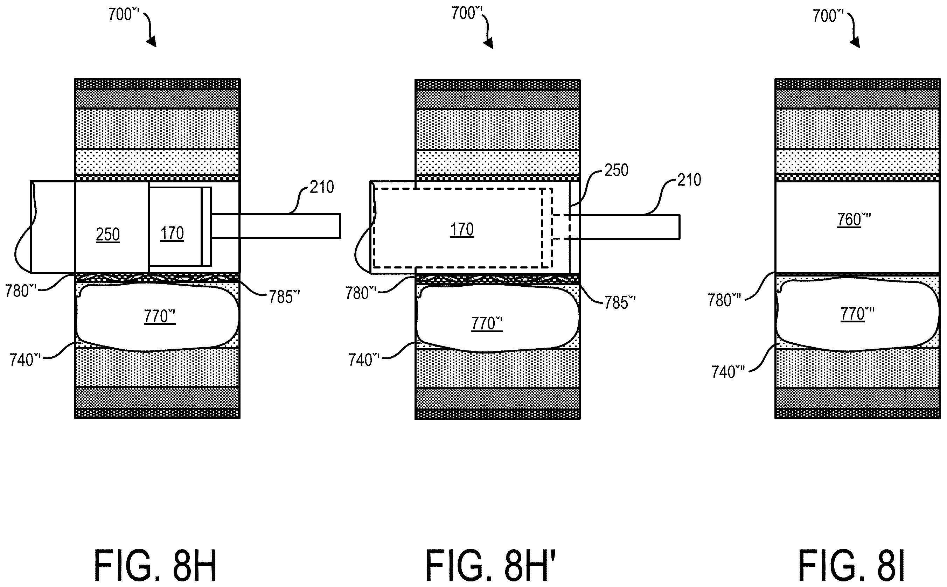

FIG. 8H' is a longitudinal-sectional view of an arterial wall taken along a direction parallel to the longitudinal axis of the arterial wall with a sheath and laser catheter located adjacent the remaining portion of the vascular occlusion depicted in FIG. 8G, wherein the end of the laser catheter, particularly its emitter(s), is disposed within and proximally of the most distal end of the sheath.
FIG. 8I is a longitudinal-sectional view of an arterial wall taken along a direction parallel to the longitudinal axis of the arterial wall after utilizing a sheath and laser catheter illustrated in FIG. 8H and or FIG. 8H'.

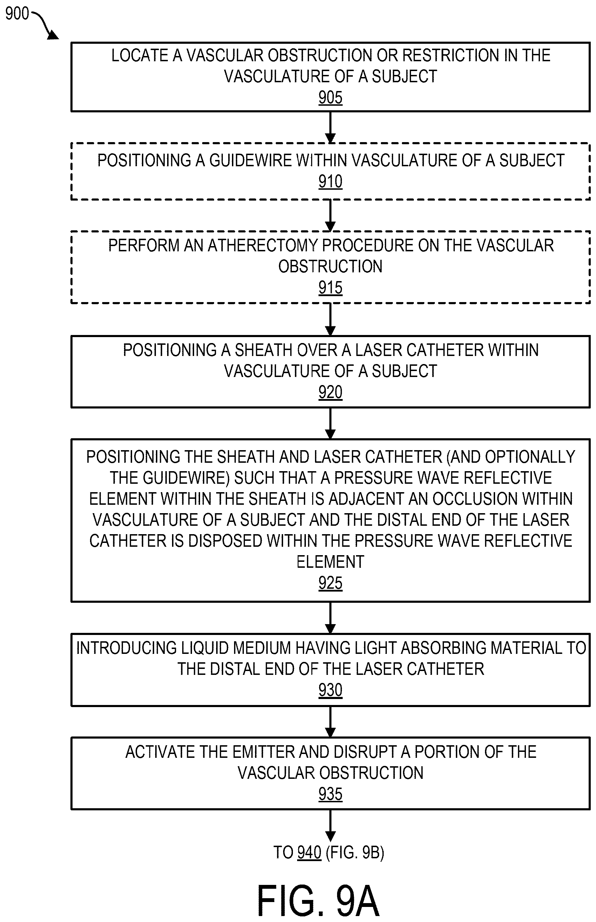

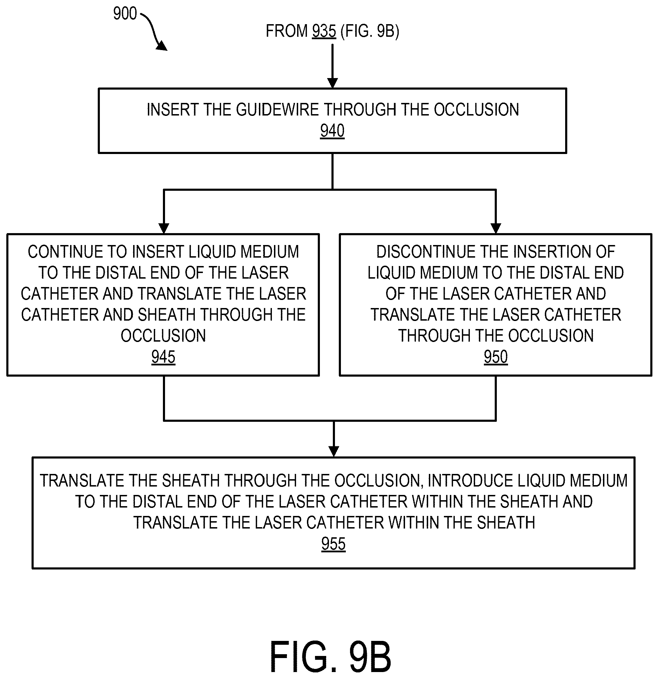

FIGS. 9A and 9B are a method example of removing restriction vascular occlusion and treating the remainder of the vascular occlusion within the intima with a sheath and laser catheter.

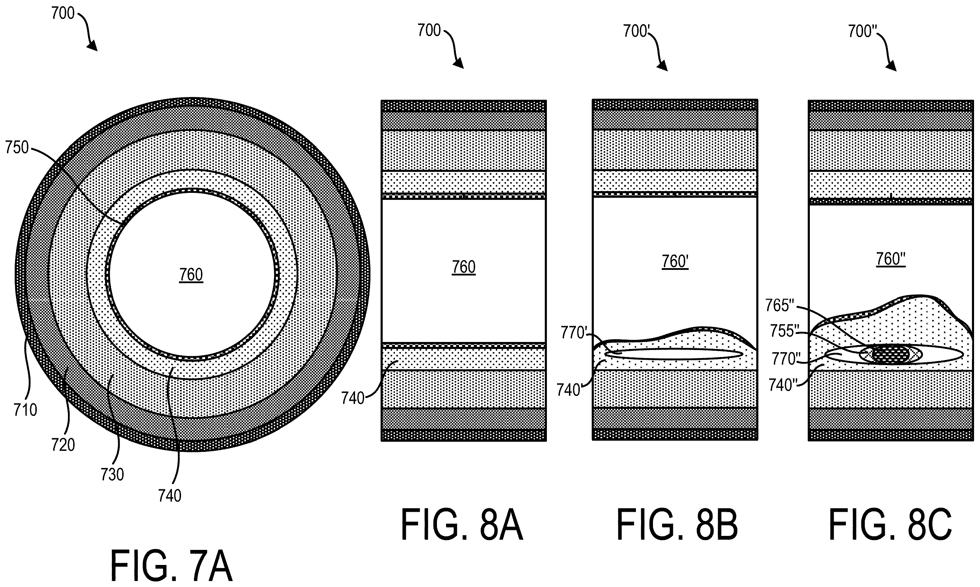

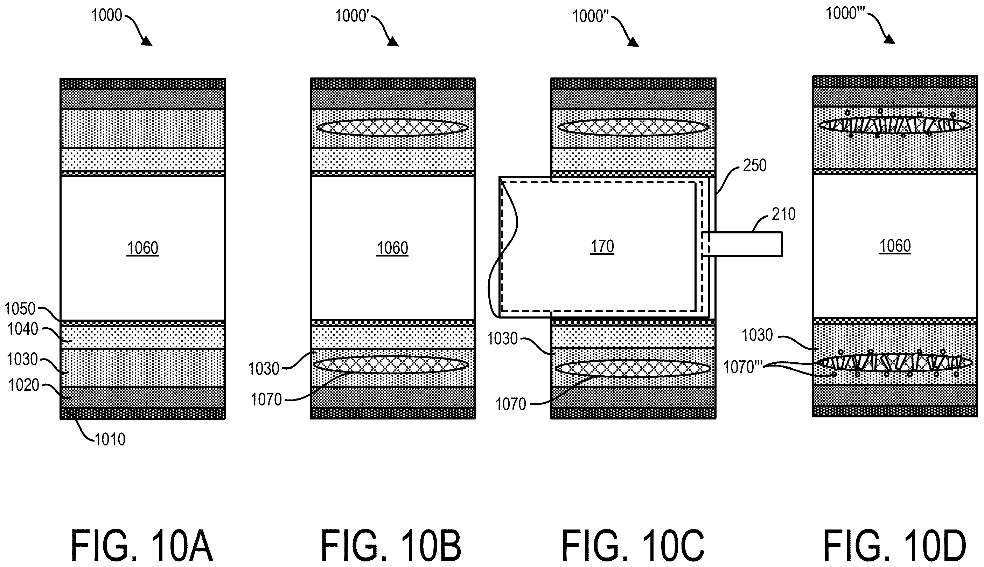

FIG. 10A is a longitudinal-sectional view of a healthy arterial wall taken along a direction parallel to the longitudinal axis of the arterial wall similar to the arterial wall depicted in FIG. 8A.
FIG. 10B is a longitudinal-sectional view of an arterial wall taken along a direction parallel to the longitudinal axis of the arterial wall with calcification of the media portion of the blood vessel.
FIG. 10C is a longitudinal-sectional view of an arterial wall taken along a direction parallel to the longitudinal axis of the arterial wall with a sheath and laser catheter located adjacent the portion of the arterial wall that includes the calcified media portion of the blood vessel.
FIG. 10D is a longitudinal-sectional view of an arterial wall taken along a direction parallel to the longitudinal axis of the arterial wall with a disrupted calcified media portion of the blood vessel.

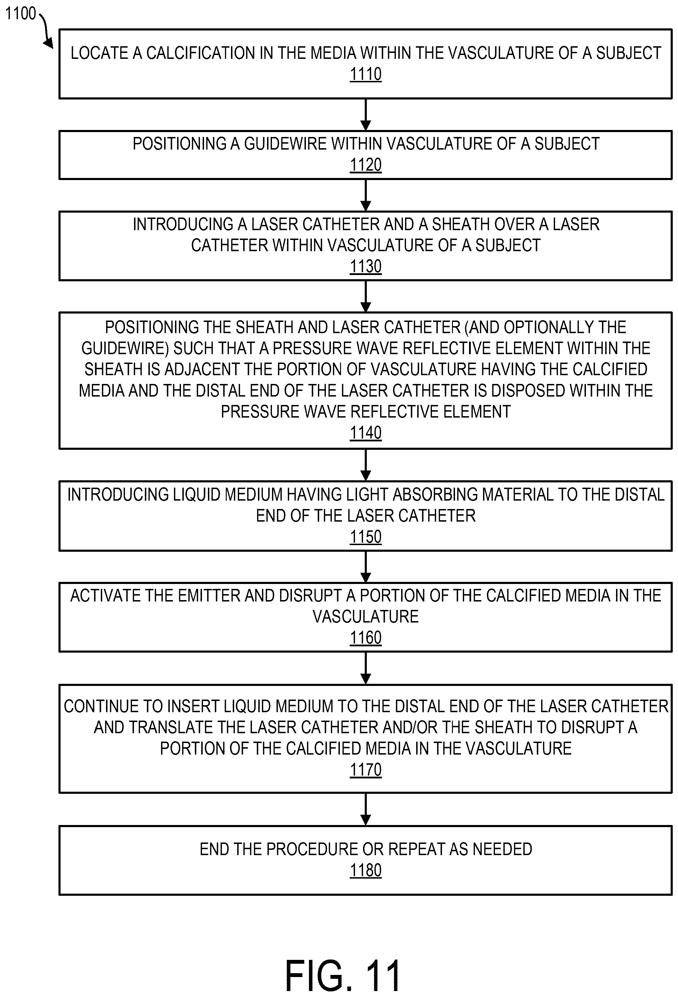

FIG. 11 is a method example of using a sheath and laser catheter to treat the calcified media portion of the blood vessel.

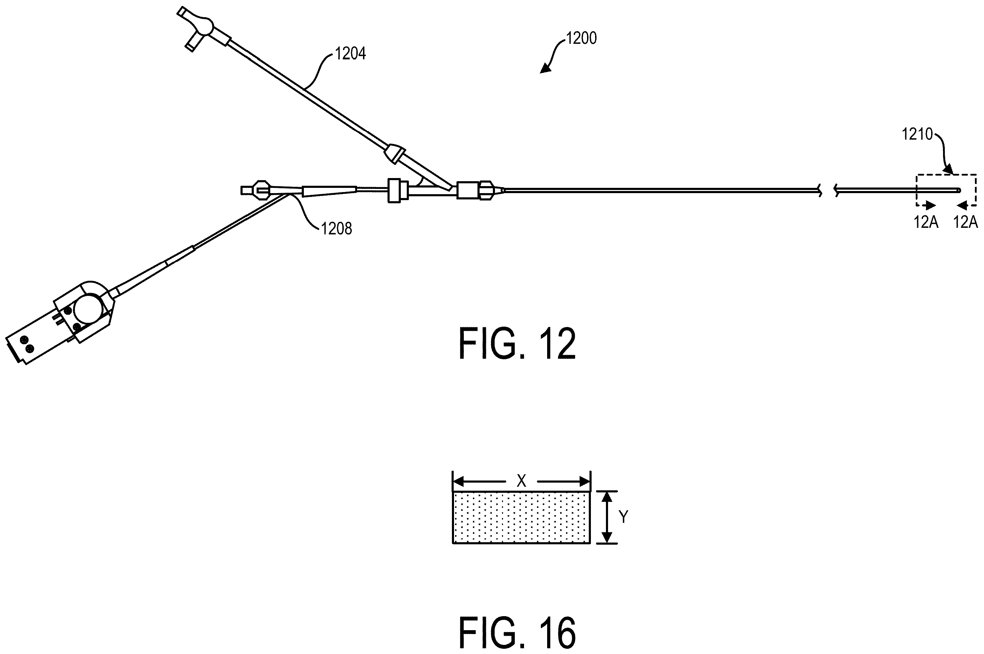

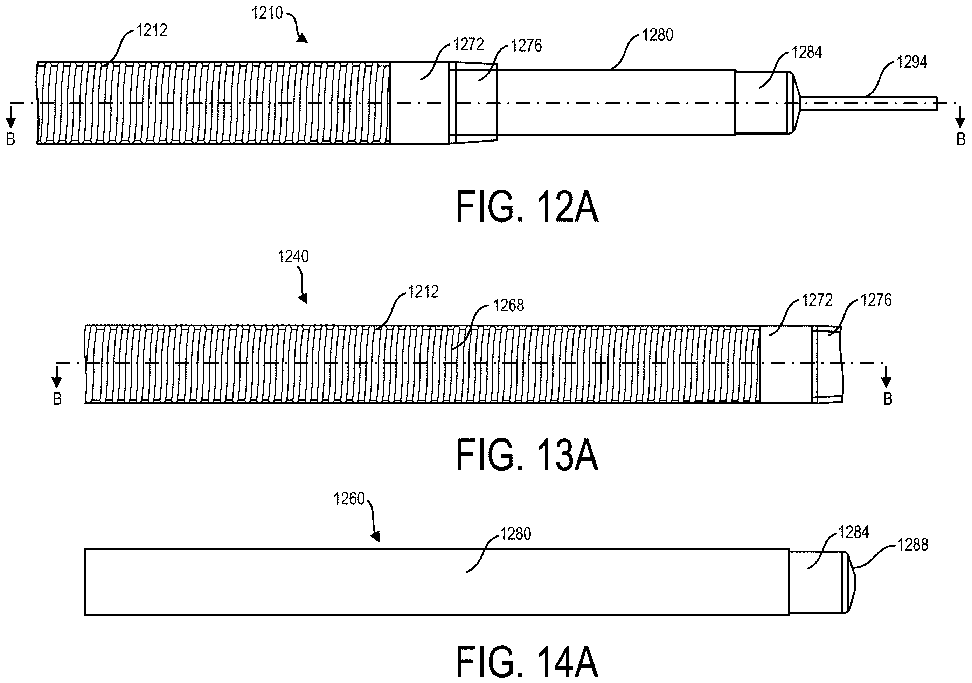

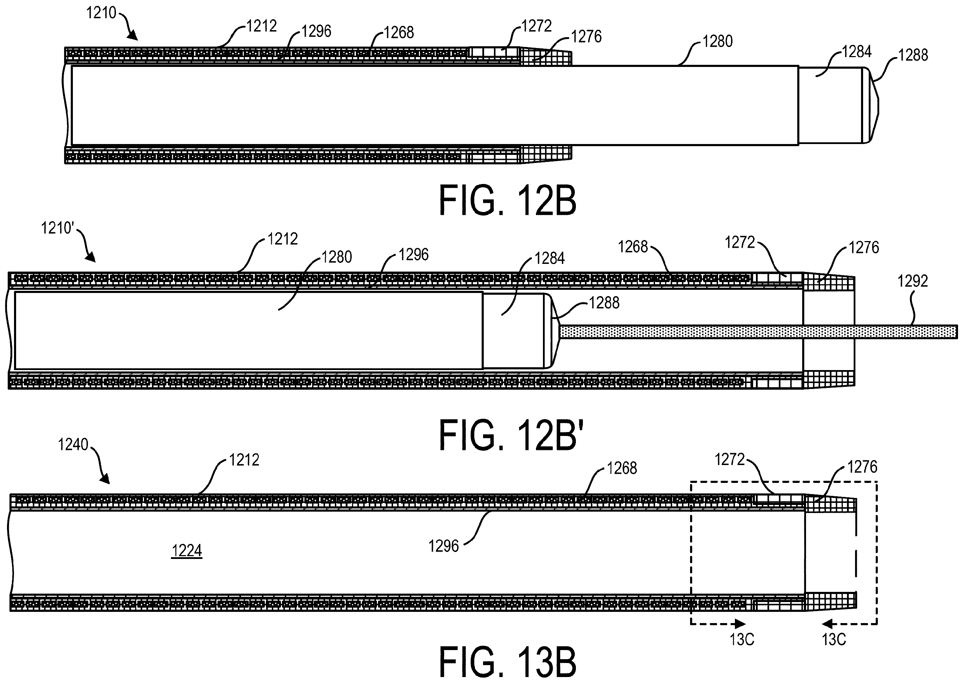

FIG. 12 is a kit that includes a laser catheter assembly and an outer sheath assembly.
FIG. 12A is an enlarged view of the distal portions of the laser catheter assembly and an outer sheath assembly within line 12A-12A of FIG. 12, wherein the laser catheter assembly extends beyond the distal end of the outer sheath assembly, and wherein a portion of the outer sheath assembly is illustrated in a translucent material for purposes of clarifying the figure.
FIG. 12B is an enlarged longitudinal sectional view of the distal portions of the laser catheter assembly and outer sheath assembly depicted in FIG. 12A taken along line B-B, wherein the laser catheter assembly is disposed within the outer sheath assembly and extends beyond the distal end of the outer sheath assembly.
FIG. 12B' is an enlarged longitudinal sectional view of the distal portions of the laser catheter assembly and outer sheath assembly depicted in FIG. 12A, wherein the laser catheter assembly is disposed within the outer sheath and the distal end of the laser catheter assembly is proximal of the distal end of the outer sheath assembly.

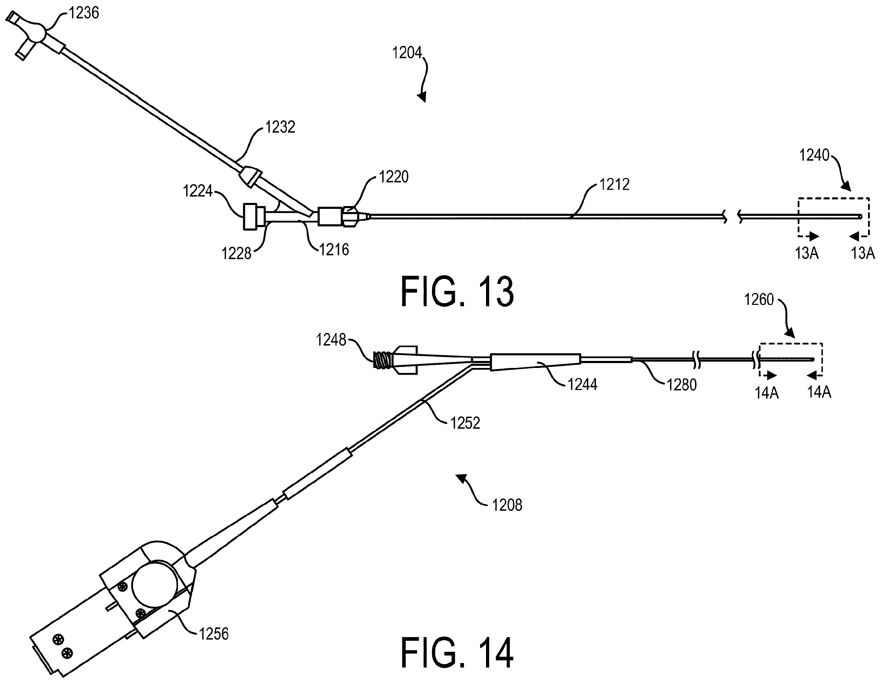

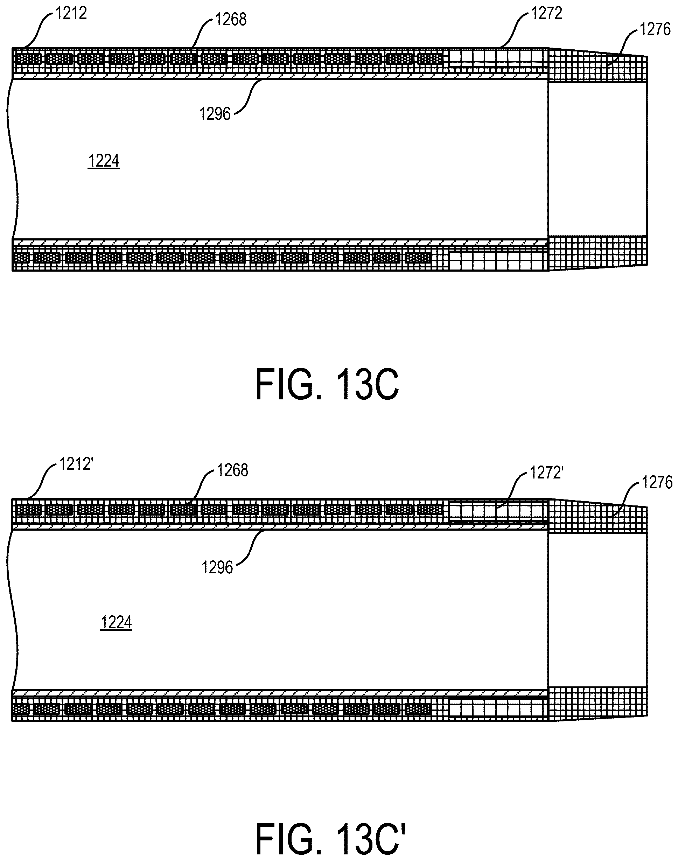

FIG. 13 is the outer sheath assembly depicted in FIG. 12.
FIG. 13A is an enlarged view of the distal portion of the outer sheath assembly within line 13A-13A of FIG. 12, wherein a portion of the outer sheath assembly is illustrated in a translucent material for purposes of clarifying the figure.
FIG. 13B is an enlarged longitudinal sectional view of the distal portion of the outer sheath assembly depicted in FIG. 13A taken along line B-B.
FIG. 13C is an enlarged longitudinal sectional view of the distal portion of the outer sheath assembly within line 13C-13C of FIG. 13B.
FIG. 13C' is an alternative embodiment of an enlarged longitudinal sectional view of the distal portion of the outer sheath assembly depicted in FIG. 13B.
FIG. 14 is the laser catheter assembly depicted in FIG. 12.
FIG. 14A is an enlarged view of a distal portion of the laser catheter assembly within line 14A-14A of FIG. 14.

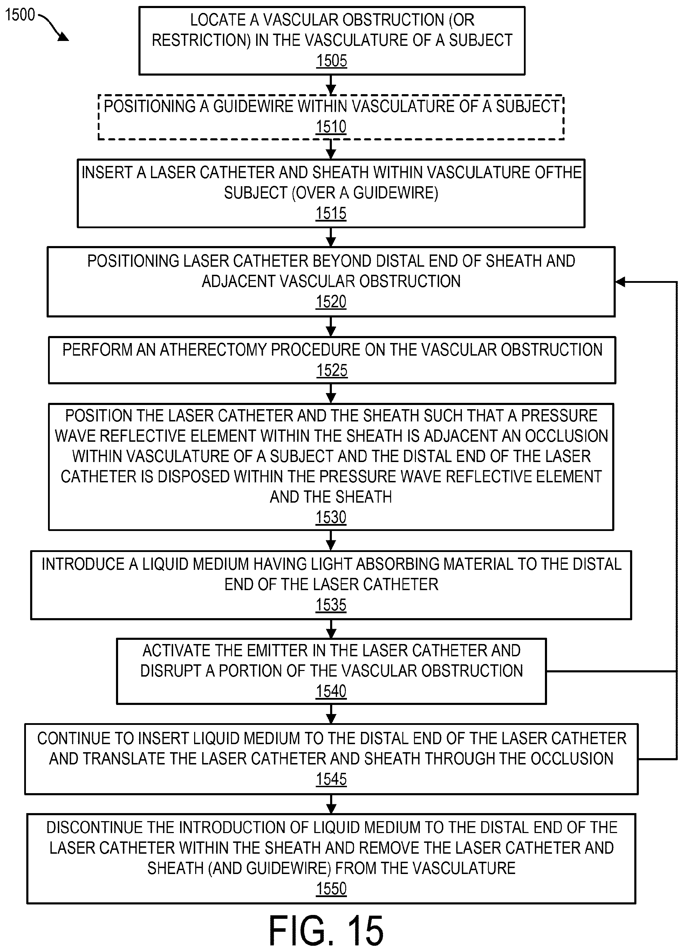

FIG. 15 is a flow chart illustrating the steps of a method example of using a kit that includes a laser catheter assembly and an outer sheath assembly.
FIG. 16 is an enlarged view of a flat wire disposed within one or more embodiments of the outer sheath assembly.

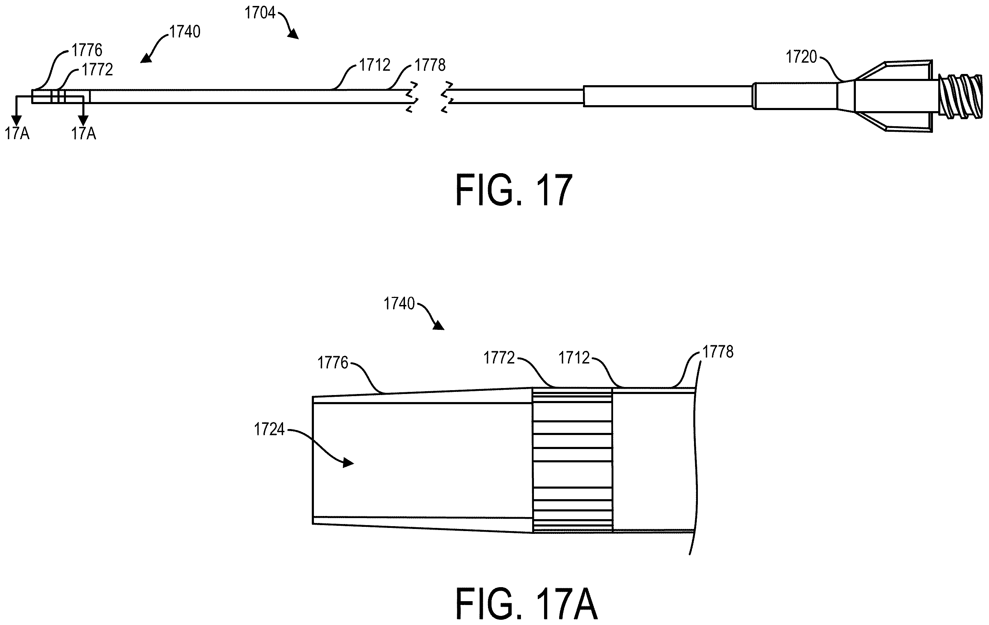

FIG. 17 is an outer sheath for a kit that also includes a laser catheter assembly.
FIG. 17A is an enlarged longitudinal sectional view of a distal portion of the outer sheath along line 17A-17A of FIG. 17.

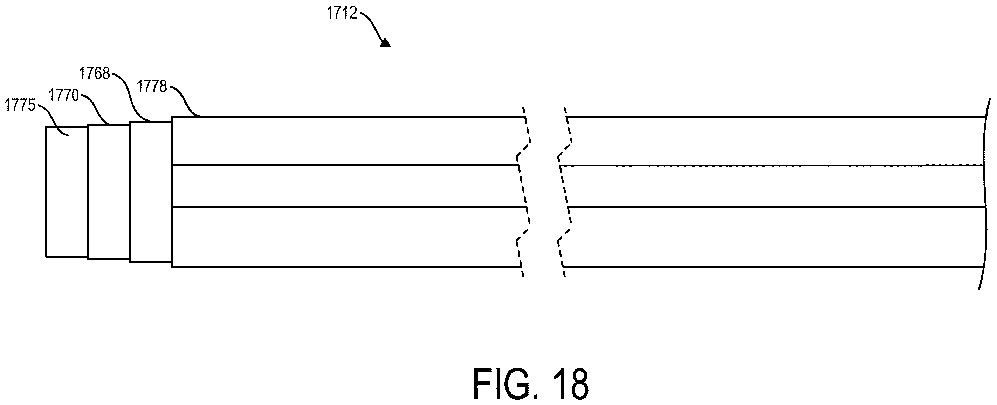

FIG. 18 is an enlarged partially exploded view illustrating various layers of the outer sheath of FIG. 17.

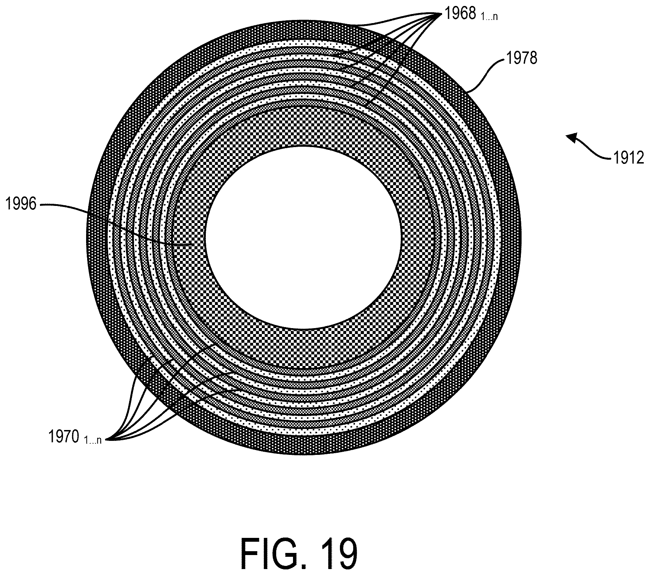

FIG. 19 is an enlarged cross-sectional view of an alternative configuration of an outer sheath.

### DETAILED DESCRIPTION

The present disclosure relates generally to the use of medical devices for the treatment of vascular conditions. In particular, the present disclosure provides materials and systems for using laser-induced pressure waves to disrupt vascular blockages and to deliver therapeutic agents to the blockage area and methods for exemplification.

Throughout the description French and Inch are used for dimensions of various parts and/or components of the embodiments, wherein 1 French is equal to 0.33 millimeters and 1 Inch is equal to 2.54 centimeters.

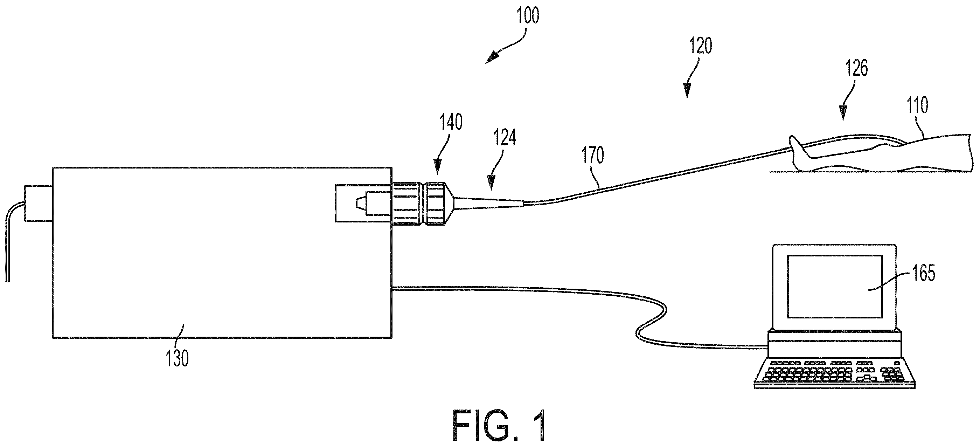

Referring to FIG. 1, there is depicted an exemplary ablation system 100 of the present disclosure. Ablation system 100 includes a laser apparatus 130 coupled to a laser controller 165. Controller 165 includes one or more computing devices programmed to control laser 130. Controller 165 may be internal or external to laser apparatus 130, such as a laser generator. Laser apparatus 130 may include an excimer laser or another suitable laser. In some embodiments, laser 130 produces light in the ultraviolet frequency range. In one embodiment, laser 130 produces optical energy in pulses.

Laser 130 is connected with the proximal end of a laser energy delivery system 120, illustratively a laser catheter 170 via coupler 140. Laser catheter 170 includes one or more optical energy transport devices which receive laser energy from laser 130 and transports the received laser energy from a first, proximal end 124 of laser energy catheter 170 towards a second, distal end 126 of laser catheter 170. The distal end of catheter 170 may be inserted into a vessel or tissue of a human body 110. In some embodiments, system 100 employs a plurality of light guides as the optical energy transport devices, such as optical fibers, that guide laser light from laser 130 through catheter 170 toward a target area in human body 110.

Exemplary laser catheter devices or assemblies may include laser catheters and/or laser sheaths. Examples of laser catheters or laser sheath are sold by The Spectranetics Corporation under the tradenames ELCA^{™} and Turbo Elite^{™} (each of which is used for coronary intervention or peripheral intervention, respectively, such as recanalizing occluded arteries, changing lesion morphology, and facilitating stent placement) and SLSII^{™} and GlideLight^{™} (which is used for surgically implanted lead removal). The working (distal) end of a laser catheter typically has a plurality of laser emitters that emit energy and ablate the targeted tissue. The opposite (proximal) end of a laser catheter typically has a fiber optic coupler 140 and an optional strain-relief member 124. The fiber optic coupler 140 connects to a laser system or generator 130. One such example of a laser system is the CVX-300 Excimer Laser System, which is also sold by the Spectranetics Corporation.

The laser controller 165 of FIG. 1 includes a non-transitory computer-readable medium (for example, memory (not shown)) that includes instructions that, when executed, cause one or more processors (not shown) to control laser 130 and/or other components of ablation system 100. Controller 165 includes one or more input devices to receive input from an operator. Exemplary input devices include keys, buttons, touch screens, dials, switches, mouse, and trackballs which providing user control of laser 130. Controller 165 further includes one or more output devices to provide feedback or information to an operator. Exemplary output devices include a display, lights, audio devices which provide user feedback or information.

A laser source of laser 130 is operatively coupled to laser controller 165. Laser source is operative to generate a laser signal or beam and provide the laser signal through a fiber optic bundle of catheter 170 to the human. Fiber optic bundle serves as delivery devices for delivering the laser signal to the target area of the human body 110.

FIG. 1 depicts the catheter 170 entering the leg, preferably through the femoral artery, of the human body. As discussed above, it may be desirable to treat either CAD or PAD. After entering the femoral artery, it the catheter 170 is intended to treat CAD, the catheter 170 will be directed through the patient's vasculature system and to the coronary arteries. Alternatively, if the catheter 170 is intended to treat PAD, the catheter 170 will be directed through the patient's vasculature system and to the peripheral arteries, such as the vasculature below the knee, particularly the vasculature in the patient's legs and/or feet. Unlike balloon catheters, the catheter 170 of the present disclosure is able to more easily navigate and enter smaller sized vasculature because the overall diameter of the sheath is smaller in comparison to balloon catheters, thereby allowing the catheter 170 of the present disclosure more easily treat PAD. That is, the increased size of a balloon of an electrically-induced shockwave balloon catheter and/or a typical dilation balloon catheter (in comparison to the catheter 170 of the present disclosure) may prevent or increase the difficulty of the balloon-type catheter from entering, penetrating and/or treating the peripheral vasculature, such the vasculature below the knee in the legs and/or feet.

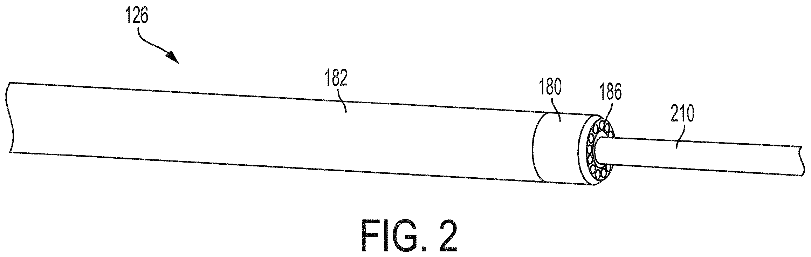

Referring to FIG. 2, there is depicted the distal end 126 of the laser catheter 170 and a guidewire 210 extending through the lumen of the catheter 170. The laser catheter 170 includes one or more layers of a plurality of optical fibers 186 surrounding a lumen extending therethrough, and a sheath 182 surrounds the layer(s) of optical fibers 186. The distal end of the laser catheter 170 may include a metal band 180, which improves the strength of the distal end and provides a radiopaque marker. That is, the catheter 170 of the present disclosure may include an outer sheath 182, an inner sheath 184 (not shown), one or more optical fibers 186, and a tip. The outer sheath 182, inner sheath 184, and one or more optical fibers 186 generally span the length of the catheter 170, and each have a proximal end and a distal end. The inner sheath 184 is disposed concentrically and/or radially within the outer sheath 182, and the one or more optical fibers 186 are disposed concentrically and/or radially within the inner sheath 184. Examples of laser catheters 170 or laser sheaths are sold by The Spectranetics Corporation under the tradenames ELCA^{™} and Turbo Elite^{™} (each of which is used for coronary intervention or catheterization such as recanalizing occluded arteries, changing lesion morphology, and facilitating stent placement) and SLSII^{™} and GlideLight^{™} (which is used for surgically implanted lead removal). Again, as illustrated in FIG. 10, the working (distal) end of a laser catheter typically has a plurality of laser emitters that emit energy and ablate the targeted tissue. The opposite (proximal) end of a laser catheter, which is not shown, typically has a fiber optic coupler that connects to a laser system or generator. One such example of a laser system is the CVX-300 Excimer Laser System, which is also sold by The Spectranetics Corporation.

The inner sheath 184, which is constructed of a biocompatible polymer has one or more lumens, which are used to deliver a liquid medium to the cavity, thereby partially or completely filling the cavity with the liquid medium. The liquid medium is introduced to the catheter 170 through one or more liquid medium ports (not shown) in fluid communication with the one or more lumens 190 (not shown) within the inner sheath 184 and disposed about the outer sheath 182. The liquid medium ports may also serve as a means for removing the liquid medium from the catheter 170.

The liquid medium is configured to absorb light energy and thereby produce laser-induced pressure waves in the liquid medium. The laser-induced pressure wave compresses the fluid surrounding its origin, thereby generating a vapor bubble. As the laser-induced pressure wave propagates away from its origin, the fluid surrounding the vapor bubble displaces inwardly, collapsing the vapor bubble and creating a cavitation event. The vapor bubble and subsequent cavitation event(s) are byproducts of the laser-induced pressure wave. And the subsequent cavitation event(s) produce additional resultant pressure waves that are transmitted to the tip 180 and/or the outer sheath 182 to disrupt a vascular occlusion.

Liquid medium can include contrast medium, including for example, iodine-containing contrast medium or gadolinium contrast medium, as well as contrast solutions comprising dye(s) and/or particle(s). Additionally, any liquid medium can be used, as long as the liquid medium is coupled with a light source, such as emitters coupled to the one or more optical fibers, which emits light at a suitable wavelength such that the liquid absorbs the light, produces laser-induced pressure waves, vapor bubbles, and cavitation events that produce additional resultant pressure waves. In some cases, the liquid medium can be contrast medium (for example, iodine-containing contrast medium or gadolinium contrast medium) and/or the liquid medium can be a contrast solution comprising a biocompatible fluid (for example, saline) in which a contrast dye(s) or particle(s) have been mixed at various concentrations.

As mentioned above, one or more optical fibers 186 are disposed within the inner sheath 184 extending from a proximal portion of the inner sheath 184 to the distal end of the inner sheath 184 and into the cavity. The proximal end of the one or more optical fibers is coupled to the laser generator 130. The distal end(s) of the one or more optical fibers 186 are proximate, at, or distal the distal end of the inner sheath 184. Again, one or more emitters are disposed at the distal end of the one or more optical fibers 186. The emitter(s) are in direct contact with the liquid medium, such that when laser light energy is emitted from the emitter(s), the liquid medium absorbs the emitted light, which in turn produces laser-induced pressure waves and generates vapor bubbles and cavitation events that produce additional pressure waves.

To treat a subject having a vascular occlusion, the tip 180 of the catheter 170 is positioned adjacent to the vascular occlusion. When the laser system 130 is activated, light energy travels through one or more optical fibers until the light energy is released from the emitter. As the liquid medium absorbs the light energy, laser-induced pressure waves are produced. Additionally, the liquid medium rapidly displaces outwards and inwards, creating vapor bubbles. The energy produced by the laser-induced pressure waves is captured within the cavity and converted to mechanical energy via moving the tip and/or transferred to the vascular occlusion through the tip. The transfer of the energy produced by the laser-induced pressure waves to the vascular occlusion is sufficient to disrupt vascular occlusion, particularly the calcified and/or fibrous (for example, calcium deposits) portions of a total occlusion. It is desirable for the mechanical energy created at the tip by the laser-induced pressure waves and resulting fluid displacement to be transferred to the occlusion. Accordingly, when the energy produced by the laser-induced pressure waves is captured within the cavity, it is desirable for the forces generated by the laser-induced pressure waves to propagate longitudinally, including in a forward (that is, parallel with the vessel) direction, thereby increasing the tip's ability to disrupt, destroy and/or penetrate the vascular occlusion. That is, as the laser-induced pressure waves are produced, the tip of the catheter rapidly moves (translates) forwards and backwards towards and away from, respectively, the occlusion. Pressure waves produced in this manner can also be used to increase vessel compliance prior to performing another procedure, such as a traditional balloon angioplasty or drug eluting balloon treatment.

In order to facilitate the direction in which the forces that are produced by the laser-induced pressure waves translate into the movement of the tip in a forward/backward longitudinal direction, the outer sheath 182 is not only flexible, but the outer sheath 182 also has the ability to expand and contract in a longitudinal direction. One example of such an outer sheath 182 includes a slotted or laser-cut hypotube constructed of a biocompatible material, such as stainless steel, or a biocompatible polymer. The hypotube has spring-like characteristics, which allow it to expand and contract in a longitudinal direction. Specifically, the slotted or laser cut pattern in the hypotube allows it to expand and contract. Another example of the outer sheath 182 may include of one or more spirally wound wires, thereby creating a coiled sheath, which also has the ability to expand and contract in a longitudinal direction.

The transfer of the energy produced by the creating a laser-induced pressure wave to the vascular occlusion and/or to the walls of the vessel is sufficient to disrupt intraluminal calcium as well as calcium within the tissue of the blood vessel, vascular occlusion (for example, calcium deposits). The forces generated by the laser-induced pressure wave can propagate radially, including in forward (that is, parallel to the vessel), upward (that is, perpendicular to the vessel), and backward (that is, proximally) directions. Laser-induced pressure waves produced in this manner can also be used to increase vessel compliance prior to performing another procedure, such as a traditional balloon angioplasty, drug-eluting balloon angioplasty and/or stent placement. That is, the laser-induced pressure wave disruption of the intraluminal calcium and/or calcium within the tissue of the blood vessel and the vascular occlusion, can improve the vasculature's ability to absorb drugs, particularly when such drugs are applied with a drug eluting balloon.

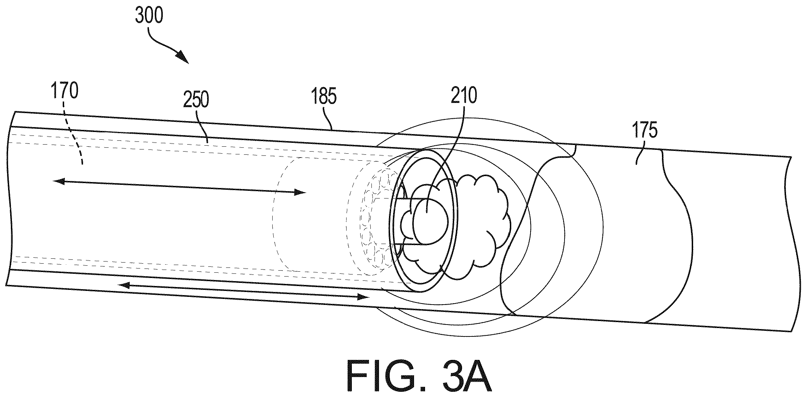

Referring again to FIGS. 3A-3D, the catheter system 300 includes the laser catheter 170 disposed within the sheath 250 and proximate the tip of the sheath 250, thereby allowing the laser-induced pressure waves to propagate radially from the sheath 250 and forward (that is, parallel to the vessel) from the tip.

The present disclosure envisions a two-piece catheter system or kit 300. Referring to FIG. 3A, the system 300 may include a laser catheter 170 radially disposed within a sheath 250. The system may optionally include a guidewire 210 disposed within a lumen of the laser catheter 170. A liquid medium is introduced into the sheath 250 distal to the laser catheter 170, particularly distal to the optical fibers/emitters of the laser catheter 170 such that when the laser is activated, the liquid absorbs the light and creates laser-induced pressure waves and/or vapor bubbles and additional resultant pressure waves and/or cavitation events. Although the liquid is not shown in this figure, the liquid may be introduced through a lumen in the laser catheter 170, a lumen in the sheath 250 and/or the lumen or space between the laser catheter 170 and the sheath 250. Regardless of which of these locations is used, one or more liquid medium ports located at or toward the proximal end of the catheter system will be also be used.

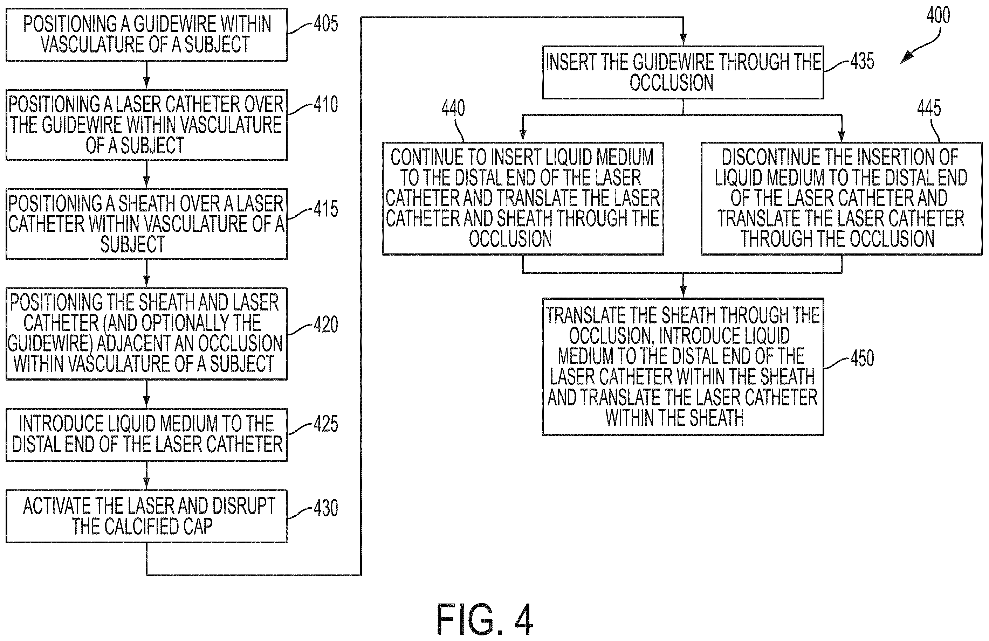

Referring to FIG. 4, there is depicted a representative flow diagram of a method 400 of removing restriction vascular occlusion using a laser catheter 170 to ablate a portion of the vascular occlusion, and/or using the laser catheter 170 in conjunction with the sheath 250 to create laser-induced pressure waves in the presence of a liquid medium and disrupt a portion of the vascular occlusion. The method 400 may include the step 405 of positioning a guidewire 210 within the vasculature 185 of a subject, the step 410 of positioning a laser catheter 170 over the guidewire 210 within the vasculature, the step 415 of positioning a sheath 250 over the laser catheter 170 within the vasculature and the step 420 of positioning the sheath 250 and laser catheter 170 (and optionally the guidewire 210) adjacent restriction vascular occlusion 175' within the vasculature 185 of a subject. Referring again to FIG. 3A, positioning the sheath 250 and laser catheter 170 adjacent the vascular occlusion 175 creates a cavity for the liquid medium to collect distally of the laser catheter 170, particularly distally of the emitters/optical fibers of the laser catheter 186.

FIG. 3A depicts the distal end of the laser catheter 170 proximal of the distal end of the sheath 250. However, it is envisioned that the distal end of the laser catheter 170 may be disposed at or distally of the distal end of the sheath 250, as long as there is liquid medium between the emitters/optical fibers of the laser catheter 170 and the vascular occlusion 175'. The axial locations of the laser catheter 170 and the sheath 250 may be adjusted by translating either or both components with respect to one another. In order to visualize the respective locations of the laser catheter 170 and the sheath 250 under fluoroscopy, the laser catheter 170 and the sheath 250 may include radiopaque markers at any corresponding locations along their lengths.

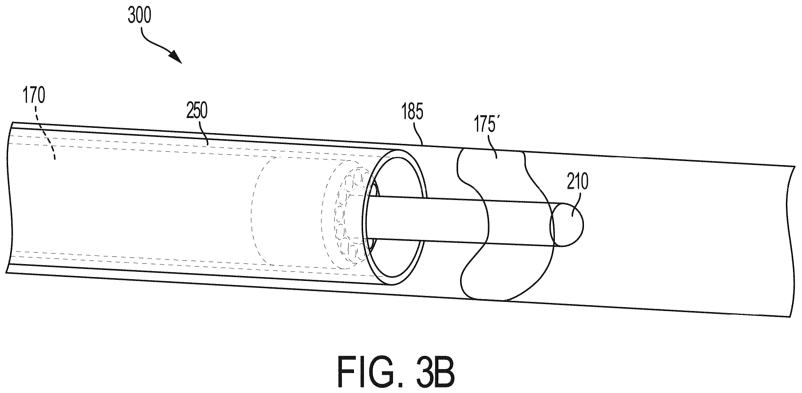

Continuing to refer to FIGS. 3A, once the sheath 250 and laser catheter 170 are disposed adjacent the vascular occlusion 175 within the vasculature (or blood vessel) 185, the liquid medium may be introduced to the distal end of the laser catheter as set forth in step 425 of FIG. 4. Continuing to refer to FIG. 4, step 430 includes activating the laser to create laser-induced pressure waves in the presence of the liquid medium and disrupting a portion of the vascular occlusion, particularly the calcified cap of the vascular occlusion. The laser catheter 170 and sheath 250 may be used to traverse the entire vascular occlusion 175, as set forth in step 440 of FIG. 4 (and optionally step 435 of FIG. 4), or only disrupt a portion of the vascular occlusion 175. If the laser catheter 170 and sheath 250 are only used to disrupt a portion of the vascular occlusion 175, then the guidewire 210 may penetrate and traverse the vascular occlusion 175. For example, FIG. 3B depicts the guidewire 210 penetrating and traversing the vascular occlusion 175'.

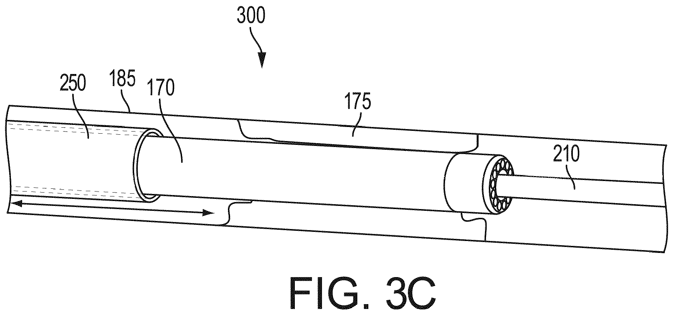

Referring to FIG. 3C, assuming that the laser catheter 170 and sheath 250 are only used to disrupt a portion of the vascular occlusion 175", the laser catheter 250 may be used to traverse the vascular occlusion 175" without the sheath 250. Referring to step 445 of FIG. 4, the insertion of the liquid medium may be discontinued and the laser catheter 170 may be used to ablate the vascular occlusion as the laser catheter 170 passes over the guidewire 210 through the vascular occlusion 175" while the sheath 250 remains proximal of the vascular occlusion.

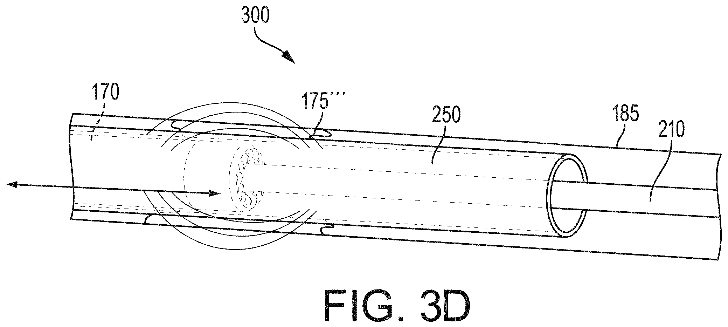

Once the entire vascular occlusion has been traversed by the laser catheter 170, the opening created by the laser catheter 170 should be large enough to translate the sheath 250 distally and through the vascular occlusion. At this point, both the distal end of the sheath 250 and the distal end of the laser catheter 170 should be distal of the vascular occlusion. At this point, referring to FIG. 3D, the laser catheter 170 is able to translate proximally while the sheath 250 remains stationary within the vascular occlusion. Upon introducing the liquid medium into the sheath 250 in front of the laser catheter 170, the laser may be activated, thereby creating laser-induced pressure waves in the presence of the liquid medium. At least a portion of the laser-induced pressure waves are directed radially, and as the laser catheter 170 translates proximally within the sheath 250, the laser-induced pressure waves transmit through the sheath 250 thereby disrupting the remainder of the vascular occlusion 175".

To ensure that the majority of the remainder of the vascular occlusion 175" is disrupted, and if desired, disrupt the intraluminal calcium and/or calcium within the tissue layers (e.g., medial layer) of the blood vessel, and the vascular occlusion, the laser catheter 170 may be repeatedly translated distally and proximally within the sheath 250. As discussed above, disruption of the intraluminal layer and/or tissue layers (e.g., medial layer) of the blood vessel and the vascular occlusion, can improve the vasculature's ability to absorb drugs, particularly when such drugs are applied with a drug eluting balloon. Also, it is contemplated that prior to, during and/or after any step in the process outlined in FIG. 4, the laser catheter 170 may be used individually to ablate a portion of the vascular occlusion, or the laser catheter 170 may be used in conjunction with the sheath 250.

Laser-induced pressure waves generally have different characteristics in comparison to ultrasound. Ultrasound typically consists of periodic oscillations with limited bandwidth. Laser-induced pressure waves are single, mainly positive pressure pulses that are followed by comparatively small tensile wave components. Ultrasound applies an alternating high frequency load to tissue, with a frequency range of several megahertz, and can thus lead to heating, tissue tears and cavitation at high amplitudes. The effect of laser-induced pressure waves in comparison, however, largely involves radially directed energy, as described above, enabling the treatment of deep tissue as well as adjacent tissue with enhanced sensitivity.

The ability of the catheter of the present disclosure to generate laser-induced pressure waves for treating a vascular occlusion in a subject involves the suitable coupling of the light system and the liquid medium. Any wavelength of light can be used, including but not limited to, laser light, visible light, ultraviolet light and infrared light, as long as the light being emitted is coupled with a liquid medium capable of absorbing the light and producing laser-induced pressure waves. Additionally, any liquid medium can be used, as long as the liquid medium is coupled with a light source that emits light at a suitable wavelength such that the liquid absorbs the light and creates laser-induced pressure waves and/or vapor bubbles. In some cases, the liquid medium can be contrast medium (for example, iodine-containing contrast medium or gadolinium contrast medium) and/or the liquid medium can be a contrast solution comprising a biocompatible fluid (for example, saline) in which a contrast dye(s) or particle(s) have been mixed at various concentrations.

The force amplitude generated by the laser-induced pressure waves depends in part on the degree of absorption of the light energy by the liquid medium as well as total energy deposited by the light source. Generally, the greater the absorption of the light energy by the liquid medium, the greater the force generated by the laser-induced pressure waves. Also, the greater the amount of the light energy delivered to the liquid medium, the greater the force generated by the laser-induced pressure waves. For example, an excimer laser typically emits laser light at a wavelength of about 308 nanometers at pulse durations between about 120 nanoseconds and about 140 nanoseconds, at frequencies between about 25 pulses per second to about 80 pulses per second, and with a total energy output between about 1 to about 100 millijoules. In some cases, however, total energy output of a laser light system can range from greater than 0 to about 300 mJ. When emitted within contrast medium, such as iodine-containing contrast medium or gadolinium contrast medium, there will be a very high degree of absorption by the contrast medium, thus creating laser-induced pressure waves with sufficient force to treat a vascular occlusion in a subject.

Light energy can be emitted at any suitable wavelength capable of generating laser-induced pressure waves. Light energy can be emitted between about 1 nanometer and about 1 millimeter. In some cases, light can be emitted from about 10 nanometers to about 5000 nanometers. In some cases, light can be emitted from about 100 nanometers to about 1000 nanometers. In some cases, light can be emitted from about 250 nanometers to about 750 nanometers. In some cases, light can be emitted from about 300 nanometers to about 600 nanometers. In still other cases, light can be emitted from about 300 nanometers to about 350 nanometers.

Light energy can be emitted at any suitable pulse duration capable of generating laser-induced pressure waves. In some cases, light can be emitted at pulse durations between about 1 femtosecond to about 1 second. In some cases, light can be emitted at pulse durations between about 10 nanoseconds to about 500 nanoseconds. In some cases, light can be emitted at pulse durations between about 100 nanoseconds to about 150 nanoseconds. In still other cases, light can be emitted at pulse durations between about 120 nanoseconds and about 140 nanoseconds.

Light energy can be emitted at any suitable pulse repetition frequency (PRF), or pulses per second, capable of generating vapor bubbles and producing resultant pressure waves that propagate through the surrounding vasculature. In some cases, light can be pulsed at a frequency of between about 1 pulse to about 5000 pulses per second. In some cases, light can be pulsed at a frequency of between about 10 pulses to about 2500 pulses per second. In some cases, light can be pulsed at a frequency of between about 10 pulses to about 1500 pulses per second. In some cases, light can be pulsed at a frequency of between about 100 pulses to about 1000 pulses per second. In other cases, light can be pulsed at a frequency of between about 50 pulses to about 500 pulses per second. In other cases, light can be pulsed at a frequency of between about 50 pulses to about 150 pulses per second. In other cases, light can be pulsed at a frequency of between about 50 pulses to about 100 pulses per second. In still other cases, light can be pulsed at a frequency of between about 25 pulses to about 80 pulses per second.

The total number of pulses administered during a particular treatment period depends on a variety of factors, including patient characteristics, the type of condition being treated, and the specific characteristics of the vascular occlusion, as one of ordinary skill in the art would readily appreciate based on the present disclosure. In some cases, the total number of pulses administered during a treatment period can range from a single pulse to any number of pulses generated in a 10 second treatment period, a 15 second treatment period, a 20 second treatment period, a 25 second treatment period, a 30 second treatment period, up to a 1 minute treatment period. Treatment periods can be repeated depending on the extent of the vascular occlusion remaining after initial treatment.

For example, a generator and/or one or more emitters may be configured to emit laser light energy at wavelengths of between about 150 nanometers to about 400 nanometers, at pulse durations between about 1 femtosecond to about 1 second, and at frequencies between about 1 pulse per second to about 5000 pulses per second. In some cases, the generator and/or the emitter(s) may be configured to emit laser light energy at wavelengths of between about 400 nanometers to about 800 nanometers, at pulse durations between about 1 femtosecond to about 1 second, and at frequencies between about 1 pulse per second to about 5000 pulses per second. In other cases, the generator and/or the emitter(s) may be configured to emit laser light energy at wavelengths of between about 800 nanometers to about 3,000 nanometers, at pulse durations between about 1 femtosecond to about 1 second, and at frequencies between about 1 pulse per second to about 5000 pulses per second. In other cases, the generator and/or the emitter(s) may be configured to emit laser light energy at wavelengths of between about 3,000 nanometers to about 12,000 nanometers, at pulse durations between about 1 femtosecond to about 1 second, and at frequencies between about 1 pulse per second to about 5000 pulses per second. In other cases, the generator and/or the emitter(s) may be configured to emit laser light energy at wavelengths of between about 300 nanometers to about 360 nanometers, at pulse durations between about 1 femtosecond to about 1 second, and at frequencies between about 1 pulse per second to about 5000 pulses per second.

The degree of force generated by the laser-induced pressure waves can be modulated by using lasers that produces laser light energy at different wavelengths and at different pulse durations, as would be appreciated by one of ordinary skill in the art based on the present disclosure. For example, different degrees of force may be required to break apart a vascular occlusion, as compared to the degree of force required to deliver a therapeutic agent to vascular tissue. In some embodiments, a laser having a holmium source, referred a Holmium laser, can emit laser light energy at a wavelength of about 2,100 nanometers (nm) and can be coupled with various light absorbing materials, including an aqueous or saline-based medium, to treat a vascular occlusion in a subject.

Several other additional sources of laser light energy can be paired with corresponding light absorbing materials to generate laser-induced pressure waves to treat a vascular occlusion. For example, YAG crystal lasers can produce wavelengths of infrared light, which is highly absorptive in aqueous solutions. Aqueous solutions can be used as light absorbing material or medium to generate laser-induced pressure waves. Aqueous solutions include, but are not limited to, saline, dextrose, radio-opaque contrast, lactated ringer's, and electrolyte solutions. In some cases, YAG wavelengths can be doubled to generate visible spectrum light of 532 nm wavelength. Materials or medium capable of absorbing light of this wavelength include, but are not limited to, gold nanospheres, nitrite solutions, potassium permanganate solutions, copper salts, aluminum solutions, aluminon, ammonia salts, and dyes such as hemotoxylin and propidium iodide. Light absorbing materials such as these can be part of a solution, such as an aqueous solution as described above, and/or they can be applied as coatings on various surfaces within a device.

In some embodiments, a Holmium YAG laser can emit laser light energy at a wavelength of about 2,120 nm and can be coupled with various light absorbing materials, including an aqueous or saline-based medium, to treat a vascular occlusion in a subject. In some embodiments, a thulium laser, such as a Thulium YAG laser, can emit laser light energy at a wavelength of about 2,013 nm and can be coupled with various light absorbing materials, including an aqueous or saline-based medium, to treat a vascular occlusion in a subject. In some embodiments, a thulium laser, such as a Thulium Fiber laser, can emit laser light energy at a wavelength of about 1,908 nm and can be coupled with various light absorbing materials, including an aqueous or saline-based medium, to treat a vascular occlusion in a subject. In some embodiments, an Nd-YAG laser can emit laser light energy at a wavelength of about 1,064 nm and can be coupled with various light absorbing materials to treat a vascular occlusion in a subject. In some embodiments, a doubled YAG laser can emit laser light energy at a wavelength of about 532 nm and can be coupled with various light absorbing materials to treat a vascular occlusion in a subject. In some embodiments, an alternative band YAG laser can emit laser light energy at a wavelength of about 1,319 nm and can be coupled with various light absorbing materials to treat a vascular occlusion in a subject. In still other embodiments, an Er-YAG laser can emit laser light energy at a wavelength of about 2,940 nm and can be coupled with various light absorbing materials to treat a vascular occlusion in a subject.

Carbon dioxide (CO₂) lasers can emit infrared light that is highly absorptive in aqueous solutions. CO₂ lasers are common surgical lasers and are highly absorptive in tissues due to their high water content. Light absorbing materials that can be coupled with CO₂ lasers that emit infrared light, such as light emitted at a 10.6 micron wavelength, to generate laser-induced pressure waves include, but are not limited to, aqueous solutions such as saline, dextrose, radio-opaque contrast, lactated ringer's, and electrolyte solutions.

Nitrogen lasers can be used to produce low frequency, high energy laser pulses. Nitrogen lasers can emit light in the UV spectrum can emit laser light energy at a wavelength of about 337 nm and can be coupled with various light absorbing materials to generate laser-induced pressure waves, including but not limited to, radio-opaque contrast as well as metals and oxides such as aluminum, silver, gold, copper, nickel, cerium, zinc, titanium, and dyes such as hydroxycoumarin and aminocoumarin.

Other medically useful lasers that can be used to generate a laser-induced pressure wave to treat a vascular occlusion include Ti-Sapphire lasers, which can emit laser light energy at wavelengths of about 800 nm; Ruby lasers, which can emit laser light energy at wavelengths of about 694 nm; and Alexandrite lasers, which can emit laser light energy at about 755 nm. These medical lasers emit laser light energy in the near infrared light spectrum, and can be used for laser-induced pressure wave generation. Light absorbing material or medium that can be coupled with these laser include, but are not limited to, dyes and colorants which could be used in solution, suspension, or coating on another material or surface within a device. Various materials capable of absorbing laser light energy in these wavelengths include aqueous copper, copper salts, and cupric sulfate, and materials such as fluorophores that are used in fluorescent microscopy (for example, methylene blue).

Dye lasers can also be used to generate laser-induced pressure waves to treat a vascular occlusion. In some cases, dye lasers can be tuned to output a specific wavelength of light in the visible spectrum, which can allow for the optimization of the laser for a certain light absorbing material, as an alternative or in addition to, using a material which is highly absorptive of a specific wavelength of light. In this way, the light absorbing material can be any of the previously mentioned materials, as well as dyes, colorants, and visible light chromophores.

For certain applications, it may be desirable to increase the amount and/or the size of vapor bubbles produced along with a laser-induced pressure wave that is generated by emitting laser light energy into a corresponding light absorbing liquid medium. For example, when entering smaller diameter sized blood vessels, the size of the catheter may be limited. In some cases, the force that vapor bubbles exert on tissue (for example, a vascular occlusion) may be proportional to the size of the individual vapor bubbles created, as the bubbles expand and contract after laser light energy is emitted into liquid medium and a pressure wave is generated. That is, the strength of the initial laser-induced pressure wave and/or the size of the vapor bubble may be limited with the use of a non-gas saturated liquid medium. One manner by which the size of individual vapor bubbles can be increased (for example, to impart greater amount of force on a particular tissue) is to saturate the liquid medium with gaseous substances so that the gas within the liquid medium exhibits a higher vapor pressure as compared to that of the liquid medium without such gas. Suitable gaseous substances that may be used to create gas-saturated liquid medium include, but are not limited to, ambient air, carbon dioxide, iodine gas, oxygen, nitrogen, compressed air, nitrous oxide, and combinations of these.

The higher vapor pressure of the gaseous substance added to the liquid medium will cause the gaseous substance to return to a gaseous state faster (under smaller pressure fluctuations) than the liquid medium. In other words, less pressure is required to cause the saturated gaseous substances to come out of solution, resulting in the creation of larger vapor bubbles, and concomitantly, a greater amount of force. In some cases, the use of gas-saturated liquid medium allows for the use of laser light energy at decreased intensities, or decreased pulses or pulse durations, without any accompanying decrease in the overall force generated by the vapor bubbles (as each vapor bubble is larger). This can enhance both the safety and efficacy of the procedure being performed.

The gaseous substances can be imparted to the liquid medium through various means, including under pressure, through mechanical agitation, and/or by bubbling the gas into the liquid medium. In some cases, gas-saturated liquid medium can be prepared prior to a procedure and then delivered to the distal end of a catheter prior to performing the procedure. Additionally or alternatively, gaseous substances can be delivered into that liquid medium that is already present in the catheter.

The gases and/or gaseous substances may be dissolved and quantified by the amount of gases present in a 1 kg of the liquid medium. The maximum amount of gas that will dissolve in the liquid medium is dependent on the solubility of the particular gas in that liquid medium, the pressure, and the temperature as described by Henry's law of gas solubility. For example, carbon dioxide may be dissolved into water at a concentration of 1.25 g/kg of water or less at 30 degrees C under atmospheric pressure. And upon dissolving carbon dioxide into water or saline, an overall concentration between 0.25-3.5 g/kgH₂O is produced. The concentrations of other dissolved gases in a kilogram of liquid medium ranges from 1mg-1g/kg for iodine, 5-80mg/kg for oxygen, 5-40mg/kg for nitrogen, 5-500mg/kg for room air, and 0.1-4g/kg for nitrous oxide.

The gases and/or gaseous substances may be dissolved in quantities above the theoretical limit, which is known as super saturation. The theoretical limit is described by Henry's law as mentioned previously. By dissolving the gases under increased pressure or decreased temperature and then returning it to normal atmospheric conditions, it is possible to dissolve a larger quantity of gas then is possible at atmospheric conditions. For example, 2.5g of carbon dioxide may be dissolved into 30 degrees C water under 2 atm of pressure, and then returned to atmospheric pressure. For any dissolved gas, the saturation percentage is defined by the concentration of gas over the theoretical maximum concentration. For any of the previously mentioned gases in a supersaturated solution, the saturation percentage can range from 100-300 percent.

The use of a gas saturated liquid medium or super saturated liquid medium may also increase the initial laser-induced pressure wave caused by the interaction of the laser light and the liquid medium. That is, the gas saturated liquid medium or super saturated liquid medium may contain larger potential energy, which when activated by the laser light, may create a larger initial laser-induced pressure wave in comparison to a laser-induced pressure wave created by the interaction of laser light and a non-gas saturated liquid medium.

Additionally or alternatively, methods described as examples also include activating at least one proximal laser emitter enclosed within the sheath assembly to send pulses of laser light energy through the liquid medium and propagating laser-induced pressure waves to assist in stent deployment. Pressure waves generated from vapor bubbles can assist in seating or expanding the stent to its full diameter as part of a medical procedure.

As discussed above, activating one or more emitters and transmitting pulses of light energy into the liquid medium produces vapor bubbles. Upon emitting light from an emitter, such as a laser catheter, within a sheath that contains an absorptive liquid medium, the vapor bubbles may be produced within the interior of the sheath and/or exterior to the sheath. Assuming that the vapor bubbles are created on the interior of the sheath, it may be desirable to limit some or all of the potential expansion of the relevant portion of the sheath caused by the vapor bubbles. That is, it may be desirable to reduce or prevent the sheath's ability to expand and contract upon creation of the vapor bubbles therein so as to reduce or prevent the sheath from applying a hydraulic force or pressure to the vascular occlusion and/or to the walls of the vessel. Also, assuming that the vapor bubbles are created on exterior of the sheath within the vessel wall, it may be desirable to reduce and/or prevent the formation of such vapor bubbles so as to reduce or prevent the cavitation event and the formation of the vapor bubbles themselves from applying a hydraulic force or pressure to the vascular occlusion and/or to the walls of the vessel.

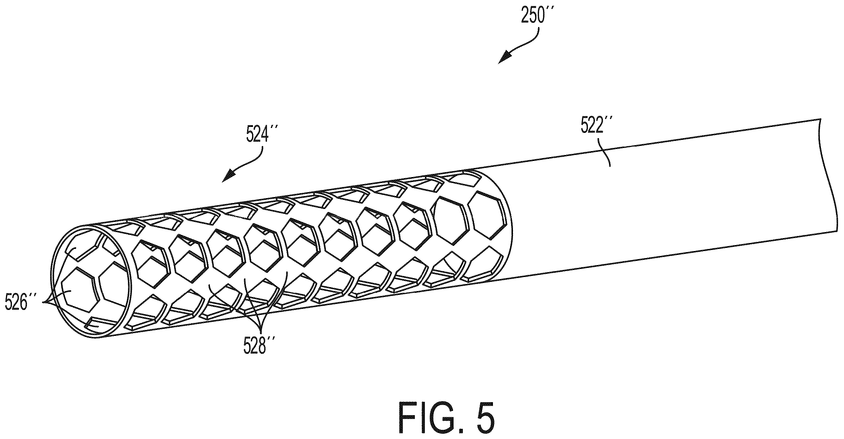

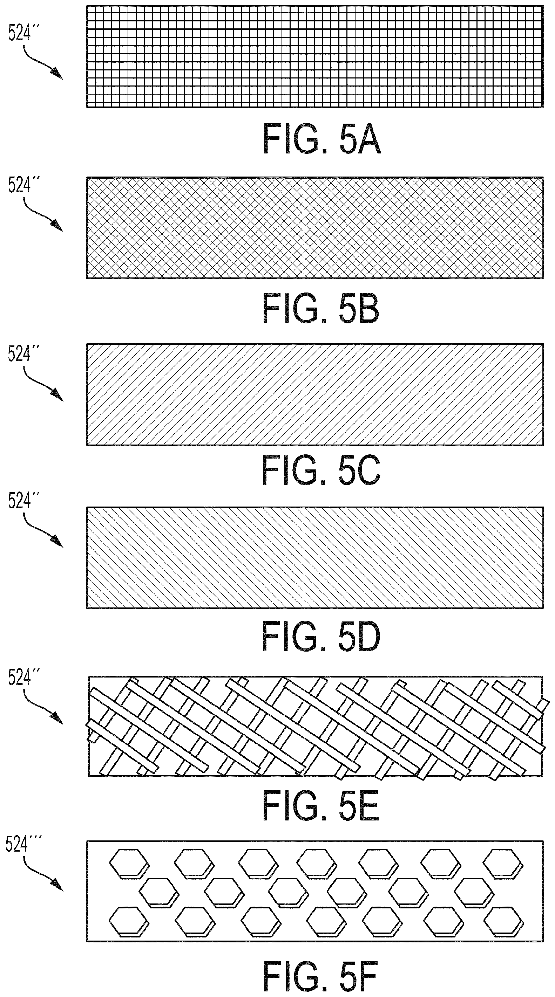

Referring to FIG. 5, there is depicted a perspective view of biocompatible sheath 250" that can be used in conjunction with a laser catheter or any of the earlier embodiments to perform a method of treating a subject, such as removing or treating a vascular occlusion. The sheath 250" may include a sleeve or jacket 522" and an attenuating member 524". FIG. 5 illustrates the attenuating member 524" as being exposed and coupled to the distal end of the sleeve 522" via an adhesive. The attenuating member 524", however, can alternatively be integrally disposed within the sleeve 522", disposed on the exterior of the sleeve 522" and/or disposed on the interior of the sleeve 522". Additionally, if the attenuating member 524" is coupled to the distal end of the sleeve 522" or the attenuating member 524" is integrally disposed within the sleeve 522" or disposed on the interior of the sleeve 522", the entire attenuating member 524" may be covered (unexposed) by the sleeve 522", the entire attenuating member 524" may be exposed, or a portion (for example, distal portion) of the attenuating member 524" may be exposed and another portion (for example, proximal portion) may be covered.

FIG. 5 also illustrates the attenuating member 524" as being disposed at the distal end of the sheath 250". The attenuating member 524", however, may alternatively and/or additionally as be disposed at the proximal end of the sheath 250", the central portion of the sheath 250", any location or multiple locations between the proximal end and distal end of the sheath 250", or in the entire length or substantially the entire length of the sheath 250".

The attenuating member 524" has two purposes. One purpose is to reinforce the sleeve 522" and/or the sheath 250", and the other purpose is to reduce the size or prevent the likelihood formation of vapor bubbles exterior of the attenuating member 524", the sleeve 522" and/or the sheath 250". Regarding the reinforcing the sleeve 522", coupling the attenuating member 524" with the sleeve 522" may reduce or prevent the sheath's ability to expand and contract upon creation of the vapor bubbles therein so as to reduce or prevent the sleeve 522" from applying a hydraulic force or pressure to the vascular occlusion and/or to the walls of the vessel. Reinforcing the sleeve 522" may minimize and/or prevent the sleeve from bulging, splitting, or delaminating (in the event the sleeve comprises multiple layers), as well as minimize and/or prevent a hole from forming within the sleeve. In the event of one or more occurrences, the difficulty of subsequent translation of the sleeve through the patient's vasculature and/or translation relative to the laser catheter may be increased.

Both the attenuating member 524" and the sleeve 522" are constructed of biocompatible materials. Coupling the attenuating member 524" with the sleeve 522" forms a rigid or semi-rigid structure within the sheath 250" such that it applies a small hydraulic force or it does not apply a hydraulic force to the vascular occlusion and/or to the walls of the vessel upon formation of vapor bubbles therein. It may be desirable that the majority or only force(s) applied to the vascular occlusion and/or to the walls of the vessel are a result of the laser-induced pressure waves that pass through the 250", thereby allowing more precise control over the laser-induced pressure waves.

Regarding the other purpose of the attenuating member 524", which is to reduce or prevent the formation of vapor bubbles exterior of the attenuating member 524", the sleeve 522" and/or the sheath 250" and continuing to refer to FIG. 5, the openings 526" within the attenuating member 524" may prevent the formation and propagation of vapor bubbles on the sheath 250". The openings 526" not only allow the laser-induced pressure waves to pass therethrough, but the quantity and size of the openings 526", particularly with respect to the remainder of the structural mass (or portions thereof 528") of the sleeve 522", may also limit the size of the vapor bubbles that can form on the exterior of the sheath 250". The relationship between the open area and the closed area (or the ratio of the open area to the overall area) within the attenuating member 524" should be such that a sufficient amount of the laser-induced pressure waves pass through the attenuating member 524". And the size of the openings 526" should allow the laser-induced pressure waves to pass therethrough, while also limiting the size of the vapor bubbles that can form on the exterior of the sheath 250". Accordingly, it may be desirable for the ratio of the open area to the overall area of the attenuating member 524" to be between 1 percent-99 percent, including any increment therebetween such as 2 percent, 3 percent, 4 percent, 5 percent, 6 percent, 7 percent, 8 percent, 9 percent, 10 percent, . . ., 15 percent . . ., 20 percent, . . ., 25 percent, . . ., 30 percent, . . ., 35 percent, . . ., 40 percent, . . ., 45 percent, . . ., 50 percent, . . ., 55 percent, . . ., 60 percent, . . ., 65 percent, . . ., 70 percent, ..., 75 percent, ..., 80 percent, ..., 85 percent, . . ., 90 percent, 91 percent, 92 percent, 93 percent, 94 percent, 95 percent, 96 percent, 97 percent, and 98 percent. It may also be desirable for the ratio of the open area to the overall area of the attenuating member 524" to be within a particular range such as between 5 percent to 95 percent, 10 percent to 90 percent, 15 percent to 85 percent, 20 percent to 80 percent, 25 percent to 75 percent, 30 percent to 70 percent, 35 percent to 65 percent, 40 percent to 60 percent, and 45 percent to 55 percent. Additionally, for any of the above listed ratios it may be desirable for each opening to have a particular size, such as between 10 microns to 10,000 microns (1 millimeter), including any increment therebetween such as 10 microns, . . ., 12.5 microns, ..., 15 microns, 17.5 microns,... 20 microns,... 30 microns,... 40 microns,...50 microns,...75 microns,...100 microns, . . ., 125 microns, . . ., 150 microns, 175 microns, . . ., 200 microns, . . ., 300 microns, . . ., 400 microns, . . ., 500microns, . . ., 600 microns, ..., 700 microns, ..., 800 microns, . . ., 900microns, . . ., 1000 microns, .. 2000 microns , . . ., 3000microns, . . ., 4000 microns, . . ., 5000 microns, . . ., 6000microns, . . ., 7000 microns, ..., 8000 microns, . . ., 9000microns, . . ., 9100microns, . . ., 9200microns, . . ., 9300microns, . . ., 9400microns, . . ., 9500microns, . . ., 9600microns, . . ., 9700microns, . . ., 9800 microns, . . ., 9900 microns, ..., and 10,000 microns. It may also be desirable for the size openings 526" within the attenuating member 524" to be within a particular range such as between 1000 to 9000 microns, 2000 to 8000 microns, 3000 to 7000 microns, 4000 to 6000 microns, and 4500 to 5500 microns.

The attenuating member's ability to reduce or prevent the formation of vapor bubbles exterior of the attenuating member 524", the sleeve 522" and/or the sheath 250" potentially reduces the existence and/or the size of the vapor bubbles formed on the exterior of the attenuating member 524", the sleeve 522" and/or the sheath 250", which in turn reduces the likelihood that vapor bubbles will be created and expand and contract between the attenuating member 524", the sleeve 522" and/or the sheath 250" and the vasculature wall. And reducing or preventing expansion and contraction of vapor bubbles between the sleeve 522", and/or the sheath 250", and the vasculature wall prevent or reduce the likelihood that a hydraulic force or pressure will be applied to the vascular occlusion and/or to the walls of the vessel, thereby preventing and/or minimizing potential damage to the vasculature itself.

Regarding the attenuating member's ability to reinforce the sleeve 522" and/or the sheath 250", the attenuating member 524" may reduce or prevent the sleeve's ability and/or the sheath's ability to expand and contract upon creation of the vapor bubbles therein. Reducing the sleeve's ability and/or the sheath's ability to expand and contract upon the formation of vapor bubbles therein, reduces or prevents the sleeve 522" and/or the sheath 250" from applying a hydraulic force or pressure to the vascular occlusion, restriction and/or to the walls of the vessel.

The openings 526" in the attenuating member 524" depicted in FIG. 5 are shown as hexagons, which are disposed around the circumference of the attenuating member 524", as well as along its length. Although the openings 526" in the attenuating member 524" are illustrated as hexagons, the openings may have an alternate shape, such as a circle, oval, triangle, square, rectangle, helix, polygon, diamond, pentagon, heptagon, octagon, nonagon, and decagon. For example, FIG. 5A illustrates a side view of a attenuating member 524" comprising a plurality of square-shaped openings; FIG. 5B is a side view of a attenuating member 524" comprising a plurality of diamond-shaped openings, FIG. 5C is a side view of a attenuating member 524" comprising a plurality of openings formed by a helical structure wound in a particular direction (for example, clockwise or left to right) while FIG. 5D is a side view of a attenuating member 524" comprising a plurality of openings formed by a helical structure wound in an alternate direction (for example, counter-clockwise or right to left). Additionally, the two helically formed attenuating members 524" may be combined to form the attenuating member 524" depicted in FIG. 5E. The attenuating member 524" depicted in FIG. 5E is similar to the attenuating member 524" depicted in FIG. 5B, but the attenuating member 524" depicted in FIG. 5B is braided and the attenuating member 524" depicted in FIG. 5E is wound or formed by one or two hypotubes. Additionally, the structural mass (or portions thereof) of the attenuating member 524" depicted in FIG. 5E is larger than the structural mass (or portions thereof 528") of the attenuating member 524" depicted in FIG. 5B because braided materials are generally smaller in size. Referring to FIG. 5F, the structural mass (or portions thereof) of the attenuating member 524" are substantial in comparison to the size of the hexagonal openings.

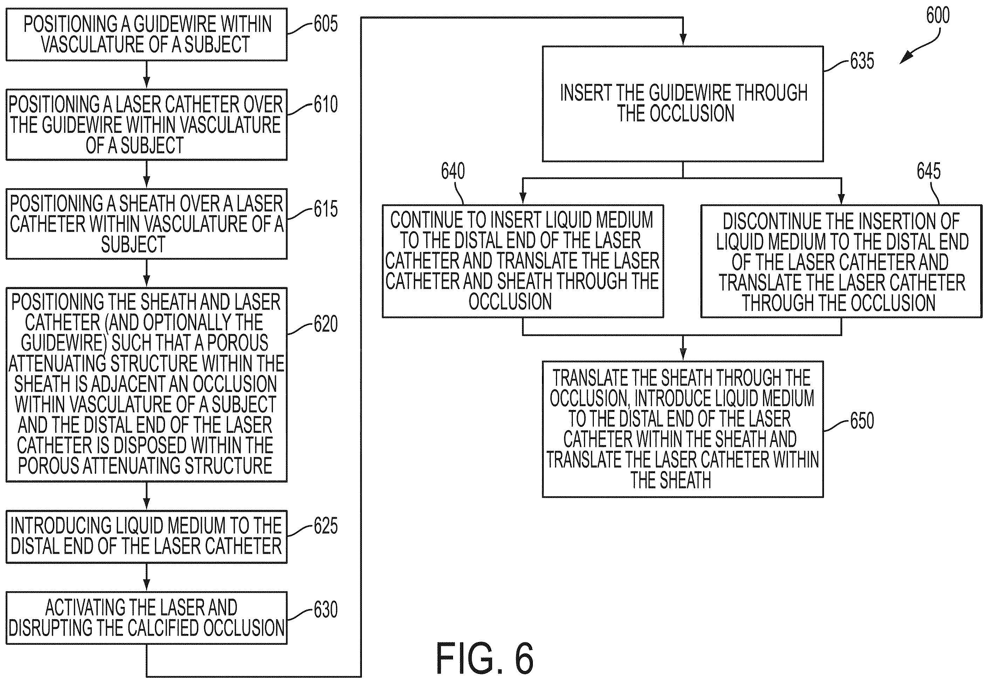

Referring to FIG. 6, there is depicted a representative flow diagram of a method 600 of treating a subject using a laser catheter 170 (depicted in FIG. 2) and the sheath 250" (depicted in FIG. 5), and/or using the laser catheter 170 in conjunction with the sheath 250" to ablate a vascular occlusion and/or create laser-induced pressure waves in the presence of a liquid medium and disrupt a portion of the vascular occlusion as depicted in FIGS. 3A-3D. The method 600 may include the step 605 of positioning a guidewire 210 within the vasculature 185 of a subject, the step 610 of positioning a laser catheter 170 over the guidewire 210 within the vasculature 185, the step 615 of positioning a sheath 250" over the laser catheter 170 within the vasculature and the step 620 of positioning the sheath 250" and laser catheter 170 (and optionally the guidewire 210) adjacent a vascular occlusion 175' within the vasculature 185 of a subject. Referring again to FIG. 3A, positioning the sheath 250 and laser catheter 170 adjacent the vascular occlusion 175 creates a cavity for the liquid medium to collect distally of the laser catheter 170, particularly distally of the emitters/optical fibers of the laser catheter 170.

FIG. 3A depicts the distal end of the laser catheter 170 proximal of the distal end of the sheath 250. However, it is envisioned that the distal end of the laser catheter 170 may be disposed at or distally of the distal end of the sheath 250, as long as there is liquid medium between the emitters/optical fibers of the laser catheter 170 and the vascular occlusion 175. The axial locations of the laser catheter 170 and the sheath 250 may be adjusted by translating either or both components with respect to one another. In order to visualize the respective locations of the laser catheter 170 and the sheath 250 under fluoroscopy, the laser catheter 170 and the sheath 250 may include radiopaque markers at any corresponding locations along their lengths.

Continuing to refer to FIGS. 3A, once the sheath 250 and laser catheter 170 are disposed adjacent the vascular occlusion 175, the liquid medium may be introduced to the distal end of the laser catheter as set forth in step 625 of FIG. 6. Continuing to refer to FIG. 6, step 630 includes activating the laser to create laser-induced pressure waves in the presence of the liquid medium and disrupting a portion of the vascular occlusion, particularly the calcified cap of the vascular occlusion. The laser catheter 170 and sheath 250 may be used to traverse the entire vascular occlusion 175, as set forth in step 640 of FIG. 6 (and optionally step 635 of FIG. 6), or only disrupt a portion of the vascular occlusion 175'. If the laser catheter 170 and sheath 250 are only used to disrupt a portion of the vascular occlusion 175, then the guidewire 210 may penetrate and traverse the vascular occlusion 175. For example, FIG. 3B depicts the guidewire 210 penetrating and traversing the vascular occlusion 175'.

Referring to FIG. 3C, assuming that the laser catheter 170 and sheath 250 are only used to disrupt a portion of the vascular occlusion 175", the laser catheter 250 may be used to traverse the vascular occlusion 175" without the sheath 250. Referring to step 645 of FIG. 6, the insertion of the liquid medium may be discontinued and the laser catheter 170 may be used to ablate vascular occlusion as the laser catheter 170 passes over the guidewire 210 through the vascular occlusion 175" while the sheath 250 remains proximal of the vascular occlusion.

Referring to step 650 of FIG. 6 and once the entire vascular occlusion has been traversed by the laser catheter 170, the opening created by the laser catheter 170 should be large enough to translate the sheath 250 distally and through the vascular occlusion. At this point, both the distal end of the sheath 250 and the distal end of the laser catheter 170 should be distally of the vascular occlusion. At this point, referring to FIG. 3D, the laser catheter 170 is able to translate proximally while the sheath 250 remains stationary within the vascular occlusion. Upon introducing the liquid medium into the sheath 250 in front of the laser catheter 170, the laser may be activated, thereby creating laser-induced pressure waves in the presence of the liquid medium. At least a portion of the laser-induced pressure waves are directed radially, and as the laser catheter 170 translates proximally within the sheath 250, the laser-induced pressure waves transmit through the sheath 250 and/or the sheath 250 itself expands and contracts, thereby disrupting the remainder of the vascular occlusion 175"".

To ensure that the majority of the remainder of the vascular occlusion 175' is disrupted, and if desired, disrupt the intraluminal layer and/or the tissues of the blood vessel and the vascular occlusion, the laser catheter 170 may be repeatedly translated distally and proximally within the sheath 250. As discussed above, disruption of the intraluminal layer and/or tissues of the blood vessel and the vascular occlusion, can improve the vasculature's ability to absorb drugs, particularly when such drugs are applied with a drug eluting balloon. Also, it is contemplated that prior to, during and/or after any step in the process outlined in FIG. 6, the laser catheter 170 may be used individually to ablate a portion of the vascular occlusion, or the laser catheter 170 may be used in conjunction with the sheath 250.

As discussed above, transmitting pulses of light energy from an emitter into a liquid medium creates laser-induced pressure waves and/or vapor bubbles and cavitation events resulting in additional pressure waves that disrupt at least a portion of a vascular occlusion. The catheter may include a guidewire lumen through which a guidewire can pass and cross the vascular occlusion. It may also be desirable to excite and vibrate the guidewire to increase the guidewire's ability to pierce and cross the vascular occlusion. Accordingly, the present disclosure also contemplates directing the laser light energy emitted by the emitter into the liquid medium in a direction which causes the liquid medium to propagate pressure waves toward the guidewire lumen and/or guidewire such that the pressure waves excite and vibrate the guidewire.

In some embodiments, the devices of the present disclosure and methods given as exemplification can also be used deliver laser-induced pressure waves to ablate a vascular occlusion using a substantially solid light absorbing material instead of liquid medium. In some circumstances, pairing a laser that emits a specific wavelength of light with a light absorbing material designed to absorb light at that wavelength can significantly increase the energy efficiency of the resultant laser-induced pressure waves produced by the reaction. The use of such pairings can ultimately reduce the energy input required to treat a vascular occlusion, which can increase the safety of the procedure and reduce costs. For example, the catheters according to embodiments of the present disclosure can be filled with air or a substantially inert liquid medium (for example, saline) instead of contrast medium, which can significantly reduce the amount and size of vapor bubbles produced along with the laser-induced pressure waves. Because the laser-induced pressure waves can propagate outside of the catheter to ablate a vascular occlusion, it can be advantageous in some circumstances to reduce (for example, by filling the catheter with saline) or eliminate (for example, by filling the catheter with air or inert gas) the production of vapor bubbles. In other cases, liquid medium delivered to the distal end of the catheter can be pre-treated to remove the amount of gas dissolved in it using methods known to one of ordinary skill in the art based on the present disclosure, as this can also reduce the amount of vapor bubbles generated along with the laser-induced pressure waves.

Suitable light absorbing material can be any agent capable of absorbing light energy and producing a laser-induced pressure wave. For example, the light absorbing material can contain an aromatic hydrocarbon with iodine bonded to it, such as iodinated x-ray contrasts. Low osmolar, non-ionic, iodinated, and radio-opaque contrasts are also suitable light absorbing materials that can be used to produce laser-induced pressure waves. Other light absorbing materials include, but are not limited to, iodinated contrasts such as Diatrizoic acid, Metrizoic acid, lodamide, lotalamic acid, loxitalamic acid, loglicic acid, Acetrizoic acid, locarmic acid, Methiodal, Diodone, Metrizamide, lohexol, loxaglic acid, lopamidol, lopromide, lotrolan, loversol, lopentol, lodixanol, lomeprol, lobitridol, loxilan, lodoxamic acid, lotroxic acid, loglycamic acid, Adipiodone, lobenzamic acid, lopanoic acid, locetamic acid, Sodium iopodate, Tyropanoic acid, Calcium iopodate, lopydol, Propyliodone, lofendylate, Lipiodol, non-iodinated contrasts such as Barium sulfate, MRI contrast agents such as Gadobenic acid, Gadobutrol, Gadodiamide, Gadofosveset, Gadolinium, Gadopentetic acid, Gadoteric acid, Gadoteridol, Gadoversetamide, Gadoxetic acid, Ferric ammonium citrate, Mangafodipir, Ferumoxsil, and Ferristene Iron oxide nanoparticles, Perflubron, Glucose and other carbohydrates, Albumen and other proteins, Nitroglycerin or other vasodilators, Hydrocarbons such as Oils, Alcohols, or other organic functional groups (Amines, Alkanes, Carboxyl, and the like), blood/tissue products such as Platelet Rich Plasma (PRP), packed red cells, plasma, platelet, fat, Charcoal, biocompatible materials such as stainless steel, biopolymers, and bioceramics, or other pharmacological agents which contain a combination of aromatic carbon rings and functional groups such as Salicylic acid, Acetylsalicylic acid, Methyl salicylate, Mesalazine, Aspirin, Acetaminophen, Ibuprofen, Clopidogrel, or other pharmacological and/or biological agents which may be compatible with the medical procedures described herein.

Suitable light absorbing material can also include those materials capable of absorbing wavelengths in the UV spectrum. For example, light absorbing materials can include, but are not limited to, PABA, Padimate 0, Phenylbenzimidazole sulfonic acid, Cinoxate, Dioxybenzone, Oxybenzone, Homosalate, Menthyl anthranilate, Octocrylene, Octyl methoxycinnamate, Octyl salicylate, Sulisobenzone, Trolamine salicylate, Avobenzone, Ecamsule, 4-Methylbenzylidene camphor, Tinosorb M, Tinosorb S, Tinosorb A2B, Neo Heliopan AP, Mexoryl XL, Benzophenone-9, Uvinul T 150, Uvinul A Plus, Uvasorb HEB, Parsol SLX, or Amiloxate, Silicon and its various atomic structures, Cadmium telluride, Copper indium gallium selenide, Gallium arsenide, Ruthenium metalorganic dye, Polyphenylene vinylene, Copper phthaloncyanine, Carbon fullerenes and derivatives, Carbon compounds such as Graphite, Graphene, Diamond, Charcoal, Titianium and oxides, Nickel and oxides, Gold, Silver, Zinc and oxides, Tin and oxides, Aluminum and oxides, or alloys or ceramics of the preceding metals.

Light absorbing material may be combined with various other compounds to facilitate their attachment to a substrate. For example, light absorbing materials may be combined with various compounds (for example, solubilizing agents) that aid in the generation of a solution or mixture comprising the light absorbing material, which can be used to coat the substrate. In some embodiments, a biodegradable and biocompatible hydrophobic polymer may be used as a light absorbing material. For example, the biodegradable and biocompatible hydrophobic polymer may be poly(glycerol sebacate acrylate) (PGSA), or variations and combinations thereof, which can be crosslinked using ultraviolet light. Ultraviolet light may be emitted from the distal end of a catheter, which may be disposed within or outside of a sheath, to activate the PGSA, for example.

Other light absorbing material can also include agents having adhesive-like properties, and in some cases, the light absorbing properties of these agents can be in addition to, or independent of, their use as adhesives. For example, light absorbing materials can include, but are not limited to, cyanoacrylates, bovine serum albumin (BSA)-glutaraldehyde, fibrin sealants, gelatin matrix thrombin, gelatin sponge, oxidized cellulose, collagen sponge, collagen fleece, recombinant factor VIIa, and the like. In some embodiments, the light absorbing material may comprise hydrophobic functional groups, such as hexanoyl (Hx; C6), palmitoyl (Pam; C16), stearoyl (Ste; C18), and oleoyl (Ole; C18 unsaturated) groups, so as to resist being washed out or disengaged from their substrate in predominately aqueous environments (for example, vascular tissue). Such light absorbing materials can include, but are not limited to, 10Ole-disuccinimidyl tartrate, 10Ste-disuccinimidyl, and variations and combinations thereof.

Light absorbing material can be configured to exhibit high absorption of light energy from an emitter. Light energy can be emitted at any suitable wavelength capable of generating laser-induced pressure waves. Light energy can be emitted between about 1 nanometer and about 1 millimeter. In some cases, light can be emitted from about 10 nanometers to about 5000 nanometers. In some cases, light can be emitted from about 100 nanometers to about 1000 nanometers. In some cases, light can be emitted from about 250 nanometers to about 750 nanometers. In some cases, light can be emitted from about 300 nanometers to about 600 nanometers. In still other cases, light can be emitted from about 300 nanometers to about 350 nanometers.

In general, the light absorbing material can be located anywhere within a catheter, so long as it generally intersects with the path of light emitted from the optical fibers. In some embodiments, the light absorbing material may be substantially solid (for example, stable in a generally solid state, such as metals and metal alloys). Substantially solid light absorbing material can be used to construct various portions of the components of the catheter, and/or substantially solid light absorbing material can be used to construct a separate structure that is independent of another catheter component.

In some embodiments, the light absorbing material can be applied to a separate supporting structure (that is, a support structure that is not predominately made of light absorbing material, or a support structure that is not being used as a light absorbing material) and used to generate laser-induced pressure waves using the devices of the present disclosure and methods given as exemplification. In some embodiments, the light absorbing materials are stable only in liquid, gel, or semi-liquid forms. In these embodiments, the light absorbing material can be included as part of a formulation or coating that is suitable for application to a support structure, such as impregnated in hydrogel or other solid support matrix. In some embodiments, the light absorbing materials can be part of a formulation or coating containing other agents that facilitate their placement on and/or adherence to a support structure. For example, solid absorbing materials can be formulated with coating agents, thickening agents, adhesive agents, and/or other pharmaceutical or biological agents that are suitable for use with the devices of the present disclosure and methods given as exemplification.

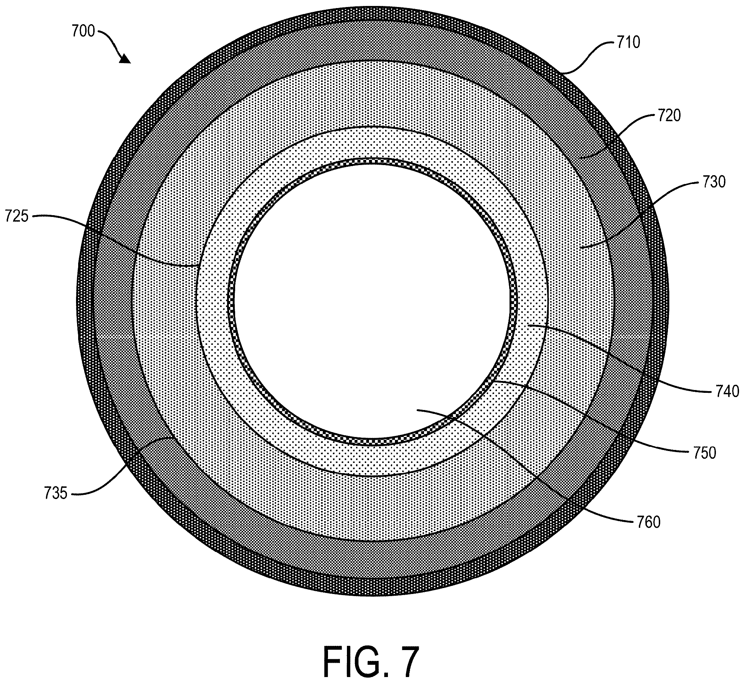

As discussed above, the present disclosure discusses using a laser catheter to ablate 170 at least a portion of the vascular occlusion or restriction in the vessel of the subject prior to using the combination of the laser catheter 170 (depicted in FIG. 2) and a sheath 250" (depicted in FIG. 5) to create laser-induced pressure waves in the presence of a liquid medium and disrupt a portion of the vascular occlusion. FIGS. 7-8F are included to illustrate the formation of a vascular occlusion within the vasculature of a subject that is treated with the laser catheter 170 and the sheath 250. Referring to FIG. 7, there is depicted a cross-sectional view of a healthy arterial wall 700 taken along a direction perpendicular to the longitudinal axis of the arterial wall. A healthy arterial wall 700, or vascular wall, typically includes an outer layer referred to as the "adventitia" or "adventicia" which is shown in FIG. 7 as layer 720. There may be additional layers, such as layer 710 of the arterial wall 700, on the outside of the adventitia. A healthy arterial wall 700 also includes a middle or central layer referred to as the media 730. The media 730 is located radially inward of and adjacent to the inner portion of the adventitia 720. The media 730 has a layers of smooth muscle cells and layers of elastin fiber that allows the artery to expand and contract. A healthy arterial wall 700 also includes an inner layer referred to as the intima 740. The intima 740 is located radially inward of and adjacent to the media 730. A healthy arterial wall 700 also includes an endothelium layer 750, which is located on the inner most surface of the intima 740 and creates the boundary for the passageway (or inner lumen) 760.

As mentioned above, the media 730 is located radially inward of and adjacent to the inner portion of the adventitia 720. Specifically, an external elastic membrane, commonly referred to as the external elastic lamina, 735 separates the media 730 from the adventitia 720. As also mentioned above, the intima 740 is located radially inward of and adjacent to the inner portion of the media 730. An internal elastic membrane 725, commonly referred to as the internal elastic lamina, separates the intima 740 from the media 730.

Referring to FIG. 7A, there is depicted a smaller version of the structure of the healthy arterial wall 700 depicted in FIG. 7. Also, FIG. 8A is a longitudinal-sectional view of the healthy arterial wall 700 taken along a direction parallel to the longitudinal axis of the arterial wall. Specifically, FIG. 8A is a longitudinal-sectional view of the structure of the healthy arterial wall 700 taken along line B-B of FIG. 7A.

Referring to FIG. 8B, over time, fat and/or lipids 770' may start to collect and/or deposit in the intima 740' of the arterial wall 700' as a result of buildup of fat and lipids in the blood. This disease process is commonly referred to as atherosclerosis and occurs in the arteries of the body including the coronary and peripheral arteries. It is this collection of fat and/or lipids 770' in the intima 740' that will lead to the formation of a vascular occlusion that can reduce or completely obstruct blood flow in the passageway 760'. Over time this buildup of fat and lipids 770" becomes a heterogeneous mix 765" (commonly referred to as plaque) of many constituents including but not limited to fats, lipids, fibrin, fibro-calcific plaque, calcium crystals, thrombus, etc. For example, a portion of the fat and/or lipids 770' may turn into plaque 765" and even become calcified, which is depicted as 755" in FIG. 8C. As the fat and/or lipids 770" collect, turn into plaque 765" and/or become calcified 755", the intima 740" starts to inflame and expand, thereby decreasing the cross-sectional area of the passageway 760".

Referring to FIG. 8D, as atherosclerotic disease progresses, and the condition of the arterial wall 700‴ is left untreated, the plaque 765‴ (not shown) continue to collect, and the intima 740‴ continues to expand and decrease the cross-sectional area of the passageway 760‴. However, upon the intima 740‴ of the arterial wall 700‴ reaching its limit to expand further, the intima 740 and the endothelium can rupture 758 and release the plaque contents previously contained in the thickened lipids and enlarged intima white blood cells into the passageway 760, as depicted in FIG. 8E. Platelets and fibrin collect within the passageway 760 of the arterial wall 700 to try and repair the rupture, and in doing so form a vascular occlusion 780, which may have calcified portions 785, as depicted in FIG. 8F. This figure also illustrates that formation of the vascular occlusion 780 further decreases the size of the passageway 760 and in some instances fully obstructs flow.

Referring to FIG. 8D and FIG. 8G and 8H, a laser catheter 170 may be used to debulk or remove plaque buildup 770 contained behind the intima or within the vascular occlusion 780 or a portion thereof from the passageway of the arterial wall 700. After debulking the plaque buildup or the occlusive disease, the combination of the laser catheter 170 and the sheath 250 of the present disclosure may be used to treat the remaining portion of the plaque buildup 770 or the vascular occlusion 780, particularly by disrupting the calcified portions 785 as depicted in FIG. 8H and/or FIG. 8H' by creating laser-induced pressure waves as described previously, to treat the condition of the arterial wall 700 as depicted in FIG. 8I.

FIG. 8I illustrates the arterial wall 700 with an enlarged passageway 760, the majority of the plaque 770‴ or vascular occlusion 780 removed, and the calcification of the remaining portion of the vascular occlusion, along with the calcification of the intima 740, fractured or modified making it more amenable to dilation at lower atmospheric pressures. FIGS. 7, 7A and 8A-8I use similar numeric values, but the different figures include different indicators, such as ' and " and combinations thereof, for the numeric values due to the changes occurring within the arterial wall as the vascular occlusion is formed and treated, which is progressively illustrated from one figure to the next. For purposes of brevity, certain layers of the arterial wall 700 are omitted from the discussion of particular figures, and numeric values of certain items of the arterial wall 700 are omitted from the particular figures. Nevertheless, one should consider the layers of the arterial wall 700, and the formations therein, to have the same numeric values even if omitted from FIGS. 7, 7A and 8A-8I.

Referring to FIGS. 9A and 9B, there is a method 900 of removing plaque buildup or occlusive disease by performing an atherectomy procedure and treating the remainder of the vascular occlusion within the intima using a laser catheter 170 (depicted in FIG. 2) in conjunction with the sheath 250 (depicted in FIG. 5) to create laser-induced pressure waves in the presence of a liquid medium and disrupt a portion of the vascular occlusion. This method 900 may be used to treat coronary arteries and/or peripheral arteries including but not limited to arteries of the vasculature of the legs, the renal arteries, subclavian arteries, etc.

The method 900 in FIGS. 9A and 9B includes locating a vascular occlusion in the vessel of a subject at step 905. The next step 910, which is optional, includes locating a guidewire at the vascular occlusion and/or inserting a guidewire through the vascular occlusion or through the passageway past the vascular occlusion. Step 915 includes performing an atherectomy procedure to remove the plaque or vascular occlusion or a portion thereof. One type of atherectomy device is an ablation catheter, such as a laser ablation catheter 170 discussed herein, which is capable of ablating at least a portion of the vascular occlusion as depicted in FIG 8G. Other types of ablation catheters include radiofrequency ablation catheters, microwave ablation catheters, and cryoablation catheters. Atherectomy devices other than ablation catheters, such as mechanical atherectomy devices, may also be used to remove the vascular occlusion.

After the vascular occlusion (or a portion thereof) is removed from the vasculature, step 920 may then be performed. Step 920 includes positioning a sheath 250 of the present disclosure over a laser catheter 170 within vasculature of a subject, as depicted in FIG. 8H, followed by step 925, which includes positioning the sheath 250 and laser catheter 170 adjacent the vascular occlusion, as depicted in FIG. 8H'. For example, if a clinician uses a catheter 170, which has a guidewire lumen, and a sheath 250, the catheter 170 may be slid over the guidewire 210 and into the vasculature, and the sheath 250 is subsequently slid over the catheter 170, which is then coupled to the sheath 250. Step 925 also includes is positioning the sheath 250 and laser catheter 170 adjacent to the vascular occlusion (or remainder thereof). The axial locations of the laser catheter 170 and the sheath 250 may be adjusted be translating either or both components with respect to one another. Particularly, the sheath 250 may be translated from the position depicted in FIG. 8H, which illustrates the laser catheter 170 extending beyond the distal end of the sheath 250, to the axial locations of the laser catheter 170 and the sheath 250 depicted in FIG. 8H', wherein the emitters of the laser catheter 170 are within the attenuating member of the sheath 250 and axially aligned with both the attenuating member and the remainder of the vascular occlusion, and the corresponding portions of the sheath and attenuating member are adjacent the vascular occlusion (or remainder thereof).

Once the sheath 250 and laser catheter 170 are disposed adjacent the vascular occlusion, such that the emitters and the attenuating member are axially aligned adjacent the vascular occlusion as depicted in FIG. 8H', the liquid medium may be introduced to the distal end of the laser catheter as set forth in step 930 of FIGS. 9A and 9B. Continuing to refer to FIGS. 9A and 9B, step 935 includes activating an energy source, such as a laser, to create laser-induced pressure waves in the presence of the liquid medium and disrupting a portion of the vascular occlusion. The laser catheter 170 and sheath 250 may be used to traverse the entire vascular occlusion or only disrupt a portion of the vascular occlusion. This is, the laser catheter 170 and sheath 250 may move axially with respect to another (between the positions shown in FIG. 8H and FIG. 8H') and/or together, while emitting laser-induced pressure waves to disrupt a portion of the vascular occlusion. If the laser catheter 170 and sheath 250 are used to disrupt a portion of the vascular occlusion, then the guidewire 210 may penetrate and traverse the vascular occlusion as set forth in step 940.

Activating at least one energy source coupled to at least one emitter of the laser catheter, which is surrounded by the sheath 250, to emit and send pulses of laser light energy into and/or to react with the liquid medium to produce propagating laser-induced pressure waves and disrupt a remaining portion of the vascular occlusion. Disrupting the remaining portion of the vascular occlusion, particularly any calcified portions within the vascular occlusion, produces cracks in the calcified portions and/or reduces the size of the calcified portions because the laser-induced pressure waves disrupt the calcified portions, thereby cracking the calcified portions and/or fragmenting the size of the calcified particles such that the contiguous area is reduced. In some cases, the method 900 may include an additional step (not shown) of activating at least one energy source coupled to at least one emitter enclosed within the sheath to emit and send pulses of laser light energy into and/or to react with the liquid medium to produce propagating laser-induced pressure waves to deliver a therapeutic agent from the sheath to a remaining portion of the vascular occlusion and/or the vascular tissue near the obstruction or restriction.

One of the benefits of the present disclosure is that the catheter 170 and sheath 250 depicted in FIG. 8H and FIG. 8H' may optionally include an attenuating member, such as the attenuating member depicted in the sheath 250" of FIGS. 5 above. The attenuating member 524", or the alternatives illustrated in FIGS. 5A-5F, may reduce or prevent the formation of vapor bubbles on the exterior of the attenuating member and/or the sheath 250" and/or reinforces the sheath such as to minimize or prevent sheath expansion. Reinforcing the sheath and/or reducing or preventing the formation of vapor bubbles on the exterior of the attenuating member (or sheath) reduces or prevents the outward and inward fluid displacement from dilating the arterial wall while simultaneously allowing the laser-induced pressure wave to penetrate the arterial wall and disrupt calcified portions in the vascular occlusion and/or intima. That is, incorporating an attenuating member, reinforces the sheath, reduces or preventing the formation of vapor bubbles on the exterior of the attenuating member, potentially inhibits displacement of the soft tissue within the arterial wall and possibly reduces or prevents delamination of the layers of the arterial wall.

Referring again to FIGS. 9A and 9B, after performing step 935 (and possibly 940), step 945 may be performed. Step 945 includes the continued insertion of the liquid medium into the gap between the combination of the laser catheter 170 and the sheath 250, thereby continuing to disrupt the vascular occlusion with laser-induced pressure waves, while the laser catheter 170 remains within the sheath 250 proximal its distal end. Alternatively, the insertion of the liquid medium may be discontinued and the laser catheter 170 may be used to ablate vascular occlusion as the laser catheter 170 passes over the guidewire 210 through the vascular occlusion while the sheath 250 remains proximal of the vascular occlusion as set forth in step 950. That is, introduction of the liquid medium between the laser catheter 170 and the sheath 250 may be terminated, and the laser catheter 170 may extend beyond the distal end of the sheath 250 so that the laser catheter 170 can perform additional atherectomy. After the additional atherectomy procedure is performed, the distal end of the laser catheter 170 may return to a position within the distal end of the sheath 250, and the liquid medium may again be supplied to the distal end of the laser catheter 170 within the sheath 250, thereby once more creating laser-induced pressure waves to disrupt the remainder of the vascular occlusion recently ablated by the laser catheter.

To ensure that the majority of the remainder of the vascular occlusion is disrupted, and if desired, disrupt the intraluminal layer and/or tissues of the blood vessel and the vascular occlusion, the laser catheter 170 may be repeatedly translated distally and proximally within the sheath 250, as in step 945 and/or step 955. As discussed above, disruption of the intraluminal layer and/or tissues of the blood vessel and the vascular occlusion, can improve the vasculature's ability to absorb drugs, particularly when such drugs are applied with a drug eluting balloon. Also, it is contemplated that prior to, during and/or after any step in the process outlined in FIG. 6, the laser catheter 170 may be used individually to ablate a portion of the vascular occlusion, or the laser catheter 170 may be used in conjunction with the sheath 250.

As discussed above, transmitting pulses of light energy from an emitter into a liquid medium creates laser-induced pressure waves and/or vapor bubbles and additional resultant pressure waves that disrupt at least a portion of a vascular occlusion. The catheter may include a guidewire lumen through which a guidewire can pass and cross the vascular occlusion. It may also be desirable to excite and vibrate the guidewire to increase the guidewire's ability to pierce and cross the vascular occlusion. Accordingly, the present disclosure also contemplates directing the laser light energy emitted by the emitter into the liquid medium in a direction which causes the liquid medium to propagate laser-induced pressure waves toward the guidewire lumen and/or guidewire such that the laser-induced pressure waves excite and vibrate the guidewire.

Although the method illustrated in FIGS. 9A and 9B depicts steps 905 through 955 of method 900 as being performed serially, any or all of the steps within the method 900 may in any order and/or in parallel with any of the other steps. For example, certain steps can be performed without performing other steps. Upon completing step 935 and/or step 940, the combined laser catheter and sheath can optionally be repositioned within the vasculature and adjacent another portion thereof. Similarly, upon completing step 935 and/or step 945, the emitter(s) can optionally be repositioned within the sheath. The sheath can be repositioned within the vasculature and/or the emitter(s) can be repositioned within the sheath. The method 900 also includes ending the procedure when the desired therapeutic outcome is obtained, or repeating any of steps 905 through 945 as may be necessary to treat a subject having a vascular occlusion.

Furthermore, a drug eluting (coated) balloon (DEB or DCB) catheter may be used to deliver drugs to the remnants of the vascular occlusion. Disrupting the remaining portion of the vascular occlusion with the laser-induced pressure waves prior to utilizing a DEB may increase the effectiveness of the drugs being applied to the vascular occlusion because the laser-induced pressure waves disrupt calcium formed in the intima layer, as well as in tissues within the blood vessel, thereby creating a pathway for the drug to enter the intima and tissues within the blood vessel and/or vascular occlusion.

The present disclosure also contemplates using the laser-induced sheath with conventional angioplasty balloons, as well as with DEBs. For example, a surgical procedure may include performing an atherectomy with a laser catheter, using the sheath in combination with the laser catheter to treat the calcified portions of the vasculature as set forth in FIGS. 9A and 9B above, and then inserting an angioplasty balloon (or DEB) into the vasculature adjacent the relevant portion of the vasculature and expanding the angioplasty balloon to dilate the relevant portion of the vasculature.

As discussed above, the laser-induced pressure waves created by the laser catheter and sheath of the present disclosure not only disrupt a vascular occlusion and/or calcium in the intima layer, the laser-induced pressure waves created by the catheter of the present disclosure can also disrupt calcification of the tissues within the vessel wall(s) That is, the laser-induced pressure waves may be used to fracture or modify calcified tissue regardless of whether the vasculature includes an occlusion. For example, patients with medial artery calcification, which is also known as Mönckeberg's sclerosis, could potentially benefit from being treated with the catheter of the present disclosure.

Referring to FIG. 10A, there is depicted a healthy arterial wall 1000 similar to the arterial wall depicted in FIG. 8A. For example, reference numerals 710, 720, 730, 740, 750 and 760 of FIG. 8A correspond to reference numerals 1010, 1020, 1030, 1040, 1050 and 1060 of FIG. 10A. That is, reference numerals 1010 and 1020 are the externa, reference numeral 1030 is the media, reference numeral 1040 is the intima, reference numeral 1050 is the endothelium, and reference numeral 1060 is the passageway.

Referring to FIG. 10B, there is depicted is a cross-sectional view of an arterial wall 1000' that includes calcium deposits 1070 formed in the media 1030. The calcium deposits begin as crystal aggregates and typically aggregate along the elastin fibrin layers within the media. As Mönckeberg's sclerosis (commonly referred to as medial calcification) progresses, multiple layers of calcium can form that involve up to the full circumference of the vessel. The calcium can also extend radially into the adventitia and intima layers. Mönckeberg's sclerosis (medial calcification) is caused by a recruitment of calcium by the smooth muscle cells and is attributed but not limited to common comorbidities found in patients suffering from vascular disease including diabetics, kidney disease patients and other metabolic or hormonal imbalances. The media 1030 includes smooth muscle cells and elastin fiber, which allow the artery to expand and contract. Upon formation of calcium deposits 1070, however, the artery's ability to expand and contract is reduced. That is, formation of calcium deposits 1070 in the media 1030 reduces the compliance of the artery 1000', which in turn potentially reduces the amount of blood flow through such arteries and can potentially negatively affect other health conditions, such as diabetes. This condition can occur with atherosclerotic disease as described previously or be an isolated condition without the narrowing of the lumen of the artery.

The combined catheter 170 and sheath 250 of the present disclosure are able to create laser-induced pressure waves, which fracture or disrupt the calcium deposits 1070 in the media 1030 of the arterial wall 1000" as shown in FIG. 10C, thereby increasing the compliance of the arterial wall 1000" and blood flow therethrough while minimizing or preventing dilation of the arterial wall. That is, one of the benefits of the present disclosure is that the sheath depicted in FIG. 10C, may optionally include an attenuating member, such as the attenuating member 524" depicted in FIG. 5 above. The attenuating member 524", or the alternatives illustrated in FIGS. 5A-5F, may reduce or prevent the formation of vapor bubbles on the exterior of the attenuating member and/or reinforce the sheath such as to minimize or prevent its expansion. Reinforcing the sheath and/or reducing or preventing the formation of vapor bubbles on the exterior of the attenuating member reduces or prevents outward and inward fluid displacement from expanding and contracting the arterial wall while simultaneously allowing the laser-induced pressure wave to penetrate the arterial wall and disrupt calcium deposits in the tissue (e.g., media) and/or tissue layers (e.g., media layer) of the blood vessel.

FIGS. 10A-10D use similar numeric values, but the different figures include different indicators, such as ' and " and combinations thereof, for the numeric values due to the changes occurring within the arterial wall as the calcium is formed and treated, which is progressively illustrated from one figure to the next. For purposes of brevity, certain layers of the arterial wall 1000 are omitted from the discussion of particular figures, and numeric values of certain items of the arterial wall 1000 are omitted from the particular figures. Nevertheless, one should consider the layers of the arterial wall 1000, and the formations therein, to have the same numeric values even if omitted from FIGS. 10A-10D.

Referring to FIG. 11, there is depicted a method 1100 of using a catheter to generate laser-induced pressure waves to treat the calcium deposits in the tissue (e.g., media) and/or tissue layers (e.g., media layer) of the blood vessel by disrupting the calcium deposits to increase vasculature compliance, thereby increasing blood flow therethrough. This method 1100 may be used to treat calcium deposits in the tissue(s) of coronary arteries and/or peripheral arteries. The method 1100 in FIG. 11 includes locating a calcification of the tissue (e.g., media) and/or tissue layers (e.g., media layer) within the vasculature of a subject at step 1110. The next step 1120, which is optional, includes positioning a guidewire within the vasculature of a subject.

After locating the calcified portion(s) of the tissue (e.g., media) and/or tissue layers (e.g., media layer) within the vasculature, step 1130 may then be performed. Step 1130 includes introducing to the vasculature a laser catheter of the present disclosure and a sheath of the present disclosure over the laser catheter. Step 1140 includes positioning within the vasculature the sheath and the laser catheter (and optionally the guidewire) such that an attenuating member within the sheath is adjacent the portion of vasculature having the calcification, and the distal end of the laser catheter is disposed within the attenuating member adjacent the vasculature that includes the calcified portion(s). For example, if a clinician uses a laser catheter 170 described herein, which has a guidewire lumen, the laser catheter may be slid over the guidewire and into the vasculature such that the emitter(s) of the laser catheter are positioned adjacent to the vasculature that has the calcification. A sheath 250 of the present disclosure is then slid over the laser catheter 170 to a position within the vasculature such that the attenuating member is adjacent the vasculature that includes the calcified portion(s). Accordingly, the emitter will be disposed within the portion of the sheath comprising the attenuating member.

The method 1100 also includes step 1150, which comprises introducing the liquid medium (for example, contrast medium) having light absorbing material to the distal end of the laser catheter, wherein the laser catheter is disposed within the sheath, and preferably within the portion of the sheath comprising the attenuating member. At step 1160, the emitters on the laser catheter are activated, thereby initiating the formation of laser-induced pressure waves a portion of which at least pass through the sheath, including the attenuating member, thereby disrupting the calcium in the vasculature. That is, the laser-induced pressure waves crack the calcified portion(s) and/or break the calcified portions of the tissue (e.g., media) and/or tissue layers (e.g., media layer) into smaller particles. Disrupting the calcified portion(s) within the tissue(s) of the blood vessel cracks the calcified portion(s) because the laser-induced pressure waves are absorbed by the calcified portions, thereby increasing the arterial wall's compliance, which in turn leads to improved blood flow and positive implications for other health conditions.

Step 1170 of method 1100 may include continuing to insert liquid medium to the distal end of the laser catheter and axially translate the laser catheter and/or the sheath to disrupt a portion of the calcified tissues of the blood vessel in the same portion or other portions of the vasculature. And any of the steps of method 1100 may be repeated until a sufficient amount of calcium is disrupted and the arterial wall's compliance is satisfactorily increased, as set forth in step 1180.

Additionally, although it is not disclosed in FIG. 11, in some cases, the method 1100 may include the step of activating at least one energy source coupled to at least one emitter enclosed within the sheath to emit and send pulses of laser light energy into and/or to react with the liquid medium to produce propagating laser-induced pressure waves to deliver a therapeutic agent from the exterior of the sheath to through the cracks in the calcified portions through the cracks in the calcified portions and/or through (or to) potentially.

Referring to FIG. 12, there is depicted a kit 1200 that includes a laser catheter assembly 1208 and a sheath assembly 1204. Sheath assembly 1204 may also be referred to as outer sheath assembly 1204 due to its disposition with respect to the laser catheter assembly 1208. FIG. 13 depicts the sheath assembly 1204 shown in FIG. 12, and FIG. 14 depicts the laser catheter assembly 1208 shown in FIG. 12. The sheath assembly 1204 may include a proximal end portion, a distal end portion 1240 and a sheath 1212 having a working length of about between 50 cm and 200 cm, including 140 cm, and a lumen 1224 extending between such ends. The sheath 1212 may also be referred to as the outer sheath 1212 due to its disposition with respect to the laser catheter assembly 1208. The distal end portion 1240 is shown in further detail in FIG. 13A. The proximal end of the sheath assembly 1204 may include a bifurcate 1216 (or Y connector) that is coupled to the sheath 1212 by a Luer fitting 1220. The bifurcate 1216 may comprise a tube 1228 extending in one direction (e.g., an axial direction) and another tube 1232 that extends in a direction offset from tube 1228. The tube 1228 may have an opening 1224 through which a guidewire (not shown) may enter the proximal end of the sheath assembly 1204. The tube 1228 may also comprise a hemostasis valve at or adjacent the opening 1224. The guidewire is capable of extending from the proximal end of the sheath assembly 1204 to the distal end of the sheath assembly 1204 through a lumen therein. The tube 1232 may include a stopcock 1236 through which the liquid medium may enter the sheath assembly 1204.

Referring to FIG. 13A, FIG. 13B, 13C and 13C' there are depicted enlarged views of distal end portion 1240 of the sheath assembly 1204 having a lumen 1224 there though. The distal end portion 1240 of the sheath assembly 1204 may comprise a sheath 1212 (or outer jacket), an inner liner 1296 radially or concentrically disposed within the sheath 1212, an outer band 1272 disposed about the distal end of the sheath 1212 (FIG. 13C), and a tapered tip 1276 disposed distally of the outer band 1272. The outer band 1272' may alternatively be disposed integrally within the distal end of the sheath 1212' such that the outer sheath (or jacket) 1212' covers the (FIG. 13C') outer band 1272'. The distal end portion 1240 of the sheath 1212 may also have an attenuating member formed therein. The attenuating member may be included within the entire length of the sheath 1212 or only at the distal end portion 1240. Assuming that the length of the sheath 1212 is 140 centimeters, the distal end portion 1240 that includes the attenuating member may be between 0.010 and 10.0 centimeters in length, which represents between 0.05 and 20.0 percent of the length of the sheath 1212.

The attenuating member may include a coil or coils 1268 integrally formed from flat wire within the sheath 1212. An example of the flat wire is shown in FIG. 16, wherein the cross-sectional width (X) of the flat wire is 0.005 inches, and the cross-sectional height (Y) of the flat wire is 0.001 inches, wherein 1 inch is 2.54 centimeters. The flat wire may be constructed of stainless steel, such as 304 stainless steel, or other type of metal or metal alloy. Additionally, it may be preferable for the flat wire to be constructed with alternative dimensions, such as a cross-sectional width (X) of between 0.001 and 0.010 inches and a cross-sectional height (Y) of between 0.0005 and 0.015 inches. Alternatively, it may be preferable to use a round wire having a diameter between 0.0005 and 0.015 inches in lieu of a flat wire.

As discussed herein, it may be desirable for the ratio of the open area for the attenuating member in comparison to the overall area of the attenuating member to be within a certain range, such as between 30 percent to 70 percent, and possibly between 40 percent and 60 percent, and more possibly between 45 percent and 55 percent, such as 45, 46, 47, 48, 49, 50, 51, 52, 53, 54 or 55 percent. Although round wire may be used to create the coil(s) 1268, using flat wire to construct the coil(s) 1268 may provide the attenuating member with an overall thinner longitudinal cross-sectional profile while decreasing the amount of open area per wrap of the wire because the height of the flat wire may be smaller than the width of the flat wire. The width of the flat wire provides the desired ratio of open area for the attenuating member in comparison to the overall area of the attenuating member, while the height of the material increases the coil(s) strength to withstand the laser-induced pressure wave without breaking. In other words, a smaller longitudinal cross-sectional profile reduces the overall diameter of the sheath assembly, thereby allowing the kit to enter smaller sized vasculature, while maintaining sufficient strength and rigidity to absorb and attenuate the laser-induced pressure wave.

There are three factors in determining the percentage of open area in the attenuating member: (1) the width of the flat wire (or diameter of the round wire); (2) the number of wraps of the wire; and (3) the gap between each wrap of wire. Once two of these factors are determined for a desired percentage of open area in the attenuating member, the third factor can be solved. In order to maintain a certain percentage of open area in the attenuating member, there is an inverse relationship between the number of wraps and the width of the flat wire (or diameter of the round wire). That is, for a certain percentage of open area in the attenuating member, the necessary wraps per inch decreases with a wire having a larger width, and the necessary wraps per inch increases with a wire having a smaller width. Additionally, there is also an inverse relationship between the number of wraps per inch and the gap between the wire wraps. That is, for a certain percentage of open area in the attenuating member using a predetermined wire size, the wraps per inch increases with a smaller gap between the wire(s). Moreover, there is a direct relationship between the size of the gap between the wire and the amount of open area in the attenuating member. That is, for a given wire width, the greater the gap between each winding, the larger the open area in the attenuating member, and the smaller the gap between each winding, the smaller the open area in the attenuating member.

For example, assuming that the attenuating member is constructed of 0.005 inch wide by 0.001 tall inch flat wire with a desired open area between 30 percent and 70 percent, the attenuating member may include between about 75 and 125 wraps (or revolutions) per inch. Specifically, a gap of about 0.008 inches between each wrap of 0.005 inch wide flat wire produces an open area of about 61.5 percent, and a gap of about 0.003 inches between each wrap of 0.005 inch wide flat wire produces an open area of about 37.5 percent. Additionally, a gap of about 0.005 inches between each wrap of 0.005 inch wide flat wire produces an open area of about 50 percent. The attenuating member, therefore, may be constructed from the flat wire such that the attenuating member includes between 75 and 125 wraps (or revolutions) per inch of the flat wire, between 80 and 120 wraps per inch of the flat wire, between 85 and 115 wraps per inch of the flat wire, between 90 and 110 wraps per inch of the flat wire depending upon the amount of open area within the attenuating member, the size (i.e., width) of the wire and the gap between each wrap of the wire. As such, it may also be preferable for the attenuating member to include about 75, 80, 85, 90, 95, 100, 105, 110, 115, 120 or 125 wraps per inch of the flat wire, wherein the flat wire is wound such that the width (X) of the flat wire is parallel with the longitudinal axis of the sheath 1212, and the height (Y) of the flat wire is perpendicular with the longitudinal axis of the sheath 1212. Additionally, if it desirable for the attenuating member to have a desired open area between 30 percent and 70 percent using a 0.004 inch wide flat wire, the gap between windings may be between, 0.0017 and 0.0093 inches respectively. These are examples, which shall not limit the scope of this disclosure because it may be desirable to have a flat wire with dimensions of 0.0002 to 0.010 inches wide and 0.0005 to 0.002 inches high, as well as round wire having a diameter between 0.0005 to 0.010 inches. For these ranges of wire size, the gap between the wire winding(s) and the wraps per length (inch) can be adjusted accordingly to produce the desired open area in the attenuating member.

Upon forming the sheath 1212 with the internally disposed attenuating member, the sheath 1212 may have an inner diameter between 0.010 and 0.200 inches and an outer diameter of about 0.014 inches such that the wall thickness of the sheath 1212 is between 0.002 and 0.015 inches. The sheath 1212 may be constructed of a polymeric material, such as Nylon-12. As mentioned above, distal end portion 1240 may also include an inner liner 1296 radially or concentrically disposed within the sheath 1212. The inner liner 1296 may be constructed of a polymer such as a polyimide having a thickness between 0.0005 and 0.010 inches, such as 0.0005, 0.0006, 0.0007, 0.0008, 0.0009, 0.001, 0.0015, 0.002, 0.00025, 0.003, 0.0035, 0.004, 0.0045, 0.005, 0.0055, 0.006, 0.0065, 0.007, 0.0075, 0.008, 0.0085, 0.0090, 0.0095 and 0.010 inches.

Again, the distal end portion 1240 of the sheath assembly 1204 may comprise an outer band 1272, which may also be referred to as a marker band, disposed about the distal end of the sheath 1212 and a tapered tip 1276 disposed distally of the outer band 1272. The outer band 1272 may be constructed of a highly radiopaque material, such as platinum iridium alloy or polymers doped with radiopaque materials such as barium sulfate, bismuth subcarbonate, bismuth trioxide, or tungsten. The tapered tip 1276 may be constructed of the same material as the sheath 1212 or an alternative material, such as nylon, pebax, polysulfone, HDPE, LDPE, UHMWPE, polypropylene, polyolefins, carbothane, polyurethane, Suralyn, ionomers, Estane, EPTFE, PTFE, or FEP. The tapered tip 1276 may, therefore, be formed integrally with the sheath 3012 or as a separate component. The circumference of the tapered tip 1276 may taper radially inward from its proximal end to its distal end between 1 degree and 10 degrees, such as 1, 2, 3, 4, 5, 6, 7, 8, 9 or 10 degrees.

It may also be preferable for the inner diameter of the tapered tip 1276 to be slightly less than the inner diameter of the inner liner 1296 of the sheath 1212, particularly if the inner liner 1296 is omitted from the distal end 1240 of the sheath assembly 1204. For example, it may be preferable for the tapered tip 1276 to seal the interface between the sheath assembly 1204 and the laser catheter assembly 1208 such that the escape of the liquid medium at the distal end of the kit 1200 is minimized or reduced. If a 5 French sized laser catheter 1280 of the laser catheter assembly 1208, in which 1 French is 0.33 millimeters, has an outer diameter of about 0.056 inches, then it may be preferable for the inner diameter of the tapered tip 1276 to be about 0.057 inches, thereby leaving a radial distance or gap of about 0.0005 inches between the laser catheter 1280 and the tapered tip 1276. For the purposes of this disclosure about 0.0005 inches means 0.0001 to 0.001 inches. Additionally, if a 6 French sized laser catheter 1280 of the laser catheter assembly 1208 has an outer diameter of about 0.069 inches, then it may be preferable for the inner diameter of the tapered tip 1276 to be about 0.070 inches, thereby leaving a distance or gap of about 0.0005 inches between the laser catheter 1280 and the tapered tip 1276. Although the embodiments described above includes a gap of about 0.0005 inches between the laser catheter 1280 and the tapered tip 1276, such gap may be between 0 and 0.002 inches and still perform a sufficient seal.

The gap between the laser catheter 1280 and the inner liner 1296 (of the sheath 1212) will preferably be greater than the gap between the laser catheter 1280 and tapered tip 1276, thereby allowing the liquid medium to enter such gap between the laser catheter 1280 and the inner liner 1296. For example, for a 5 French sized laser catheter 1280 of the laser catheter assembly 1208 having an outer diameter of about 0.056 inches, it may be preferable for the inner diameter of the inner liner 1296 to be about 0.0615 inches, thereby leaving a radial distance or gap of about 0.00275 inches between the laser catheter 1280 and the inner liner 1296, which is about five times the gap between the gap between the laser catheter 1280 and the tapered tip 1276. For a 6 French sized laser catheter 1280 of the laser catheter assembly 1208 having an outer diameter of about 0.069 inches, it may be preferable for the inner diameter of the inner liner 1296 to be about 0.0745 inches, thereby leaving a distance or gap of about 0.00275 inches between the laser catheter 1280 and the inner liner 1296, which is about five times the gap between the gap between the laser catheter 1280 and the tapered tip 1276. Accordingly, the diameter of the lumen in the inner liner 1296 is greater than the diameter of the lumen in the tapered tip 1276. Although the embodiments described above includes a radial gap of about 0.00275 inches between the laser catheter 1280 and the inner liner 1296, such radial gap may be between about 0.001 and 0.010 inches and still provide a sufficient amount of liquid medium to enter the radial gap and create the desired pressure wave upon exposure to the laser light energy.

Referring to FIG. 14 and FIG. 14A, there is depicted the laser catheter assembly 1208 shown in FIG. 12. An example of the laser catheter assembly 1208 is the Turbo-Elite^{™} laser atherectomy catheter produced by The Spectranetics Corporation. The laser catheter assembly 1208 may include a laser catheter 1280, a bifurcate 1244, a Luer adapter 1248, another sheath 1252 and a coupler 1256. Coupler 1256 is coupled to a laser system, such as the CVX-300 Excimer Laser System, which is also produced by The Spectranetics Corporation. The sheath 1252 is coupled to the coupler 1256, and sheath 1252 encapsulates the fiber optic bundle, which passes through the bifurcate 1244 and the laser catheter 1280. The coupler 1256 is configured to encapsulate or incorporate the fiber optic bundle. The bifurcate 1244 may be coupled to the proximal end of the sheath 1280. Additionally, Luer adapter 1248 may also be coupled to the proximal end of the bifurcate 1244, thereby providing an entry point to for a guidewire to enter into and pass through the laser catheter assembly 1208. The distal portion 1260 of the laser catheter assembly 1208 may include a radiopaque outer band 1284 and emitters 1288 disposed distally of the radiopaque outer band 1284, wherein the emitters 1288 are the distal ends of the optical fibers or coupled to the optical fibers.

Referring to FIG. 15, there is depicted a flow chart illustrating the steps of a method 1500 of using, for example, the kit 1200 (depicted in FIG. 12) that includes the laser catheter assembly 1208 (depicted in FIG. 14) and the sheath assembly 1204 (depicted in FIG. 13) to remove plaque buildup or occlusive disease by performing an atherectomy procedure and treating the remainder of the vascular occlusion within the intima using the laser catheter assembly 1208 in conjunction with the sheath assembly 1204 to create laser-induced pressure waves in the presence of a liquid medium and disrupt a portion of the vascular occlusion. This method 1500 may be used to treat coronary arteries and/or peripheral arteries including but not limited to arteries of the vasculature of the legs, the renal arteries, subclavian arteries, etc. The method 1500 in FIG. 15 includes locating a vascular occlusion in the vasculature (or blood vessel) of a subject at step 1505. The next step 1510, which is optional, includes locating the guidewire 1292 at the vascular occlusion and/or inserting the guidewire 1292 through the vascular occlusion or through the passageway past the vascular occlusion. Step 1515 includes inserting an atherectomy device over the guidewire 1292 and into the patient's vasculature. One type of atherectomy device is an ablation catheter, such as the laser catheter assembly 1208 discussed herein, which is capable of ablating at least a portion of the vascular occlusion. Other types of ablation catheters include radiofrequency ablation catheters, microwave ablation catheters, and cryoablation catheters. Atherectomy devices other than ablation catheters, such as mechanical atherectomy devices, may also be used to remove the vascular occlusion. Assuming that the laser catheter assembly 1208 is used as the atherectomy device, the sheath assembly 1204 may also be introduced to the patient's vasculature either simultaneously with the laser catheter assembly 1208 or sequentially, such as prior to or after introducing the laser catheter assembly 1208 into the patient's vasculature.

Once the laser catheter assembly 1208 and the sheath assembly 1204, particularly the laser catheter 1280 and the sheath 1212, respectively, are located within the patient's vasculature, the laser catheter 1280 is positioned beyond the distal end of the sheath 1212 and adjacent the vascular occlusion at step 1520. The clinician using the kit 1200 will be able to determine that the laser catheter assembly 1208, particularly the emitters 1288 is positioned beyond the distal end 1240 of the sheath assembly 1204 and adjacent the vascular occlusion because the radiopaque outer band 1284 of the laser catheter 1280 will be illustrated under fluoroscopy as being distal of the outer band 1272 of the sheath assembly 1204, as illustrated in FIG. 12A. At this point, step 1525 may be initiated by activating the emitters 1288, such as supplying laser light energy thereto, and ablating the vascular occlusion (or a portion thereof), as illustrated in FIG. 8G, FIG. 8H and/or FIG. 8H', wherein the laser catheter assembly 1208 and the sheath assembly 1204 replace the sheath 250 and the catheter 170, respectively, in FIG. 8G, FIG. 8H and/or FIG. 8H'.

After the vascular occlusion (or a portion thereof) is removed from the vasculature, step 1530 may then be performed. Step 1530 includes positioning the sheath assembly 1204 over the laser catheter assembly 1208, particularly the sheath 1212 over the laser catheter 1280, within vasculature of a subject and adjacent the vascular occlusion similar to the way in which the sheath 250 and the catheter 170 are situated in FIG. 8H'. The axial locations of the laser catheter 1280 and the sheath 1212 may be adjusted by translating either or both components with respect to one another back and forth between the positions illustrated in FIG. 8H and FIG. 8H' or any position(s) therebetween. For purposes of clarification, FIGS. 12A and 12B correspond to how the laser catheter assembly 1240 and the sheath assembly 1204, particularly the laser catheter 1280 and the sheath 1212, of those figures would be oriented in the vasculature if the catheter 170 and sheath 250 of FIG. 8H, and FIG. 12B' corresponds to how the laser catheter 1280 and the sheath 1212 of that figure would be oriented in the vasculature, if the catheter 170 and sheath 250 of FIG. 8H' would be replaced with the laser catheter 1280 and the sheath 1212. Particularly, the axial locations of the laser catheter 1280 and the outer sheath 1212 may be axially aligned such that the emitters 1288 are within the attenuating member 1268 of the sheath 1212, and the corresponding portions of the sheath 1212 and attenuating member 1268 are adjacent the vascular occlusion (or remainder thereof). That is, step 1530 includes positioning the sheath 1212 over a laser catheter 1280 within the vasculature of a subject and adjacent the vascular occlusion such that distal end 1260 of the laser catheter assembly 1208, including its emitters 1288, are within the distal end 1240 of the sheath assembly 1208 such that the emitters 1288 are within the attenuating member 1268 of the sheath assembly 1204, and the outer band 1284 of the laser catheter assembly 1208 is proximal of the radiopaque outer band 1272 of the sheath assembly 1204, as depicted if FIG. 12B' (and FIG. 8H').

Once the distal portions 1240, 1260 of the sheath assembly 1204 and laser catheter assembly 1208 are disposed adjacent the vascular occlusion, such that the emitters 1288 and the attenuating member 1268 are axially aligned adjacent the vascular occlusion, the liquid medium may be introduced to the distal end 1260 of the laser catheter assembly 1208 as set forth in step 1535 of FIG. 15. The liquid medium may be introduced to the kit 1500 through the tube 1232 and/or stopcock 1236 at the proximal end of the sheath assembly 1204. Continuing to refer to FIG. 15, step 1540 includes activating an energy source, such as a laser, to create laser-induced pressure waves in the presence of the liquid medium and disrupting a portion of the vascular occlusion. The distal ends 1240, 1260 of the laser catheter assembly 1208 and the sheath assembly 1204 may be used to traverse the entire vascular occlusion or only disrupt a portion of the vascular occlusion. That is, the laser catheter assembly 1208 and the sheath assembly 1204, particularly their respective distal portions 1240, 1260 may move axially with respect to another and/or together, while emitting laser-induced pressure waves to disrupt a portion of the vascular occlusion. While activating the emitters 1288, it may be desirable to translate the laser catheter assembly 1208 within the sheath assembly 1204 while the sheath assembly 1204 remains stationary, as the liquid medium continues to be introduced to the distal end 1210 of the kit 1200. For example, it may be desirable to retract the laser catheter assembly 1208 within the sheath assembly 1204 by axially moving the laser catheter assembly 1208 within the sheath assembly 1204 in a proximal direction at a rate of between 0.5 mm/second and 5 mm/second, particularly at a rate of less than or equal to 4.5, 4.0, 3.5, 3.0, 2.5, 2.0, 1.5 or 1 mm/second. In addition to moving the laser catheter assembly 1208 within the sheath assembly 1204 in a proximal direction, the laser catheter assembly 1208 may move in a distal direction within the sheath assembly 1204 while maintaining the emitters 1288 proximal of the distal end 1240 of the sheath assembly 1208. For the purposes of clarification, during emitter activation, as well as proximal and distal movement of the sheath assembly 1208 to disrupt the calcium, the emitters 1288 are within the distal end 1240 of the sheath assembly 1208 such that the emitters 1288 are within the attenuating member 1268 of the sheath assembly 1204, and the outer band 1284 of the laser catheter assembly 1208 is proximal of the radiopaque outer band 1272 of the sheath assembly 1204.

During disruption of the calcium, it may be desirable to adjust the settings of the laser system to which the laser catheter assembly 1208 is coupled such that the emitters 1288 produce a fluence between 30 and 80 mJ/mm², more preferably between 40 and 70 mJ/mm², and even more preferably at a fluence of 45, 50, 55, 60 mJ/mm². It may also be desirable for the repetition rate of the laser to be between 25 hertz and 80 hertz, including 25, 30, 35, 40, 45, 50, 55, 60, 65, 70, 75 and 80 hertz. It may also be desirable for the pulse width of the laser to be between 125 nanoseconds and 200 nanoseconds, including 125, 130, 135, 140, 145, 150, 155, 160, 165, 170, 175, 180, 185, 190, 195 and 200 nanoseconds. As also discussed herein, the wavelength of the laser light energy and that emitted by the emitters may include a variety of wavelengths, including a wavelength of between about 150 nanometers to about 400 nanometers such as 308 nanometers.

If the laser catheter assembly 1208 and the sheath assembly 1204 are continued to be used to disrupt a portion of the vascular occlusion, then laser catheter assembly 1208 and sheath assembly 1204 are used as set forth in step 1540. If, however, the clinician wishes to discontinue using the laser catheter assembly 1208 and the sheath assembly 1204 to disrupt a portion of the vascular occlusion and use the laser catheter assembly 1208 to perform additional ablation, then the clinician repeats step 1520 (as well as the additional subsequent steps), as depicted in FIG. 15, and the distal portion 1260 of laser catheter assembly 1208 is again extended beyond the distal portion 1240 of the sheath assembly 12, as illustrated in FIGS. 12A and 12B (and FIGS. 8G, FIG. 8H and/or FIG. 8H'). Upon satisfactory ablation and disruption of the vascular occlusion by repeating steps 1520 to 1540 or 1545, activation of the emitters and introduction of the liquid medium to the kit 1200 is discontinued and the kit is removed from the patient's vasculature.

As discussed above, transmitting pulses of light energy from an emitter into a liquid medium creates laser-induced pressure waves and/or vapor bubbles and additional resultant pressure waves that disrupt at least a portion of a vascular occlusion. The catheter may include a guidewire lumen through which a guidewire can pass and cross the vascular occlusion. It may also be desirable to excite and vibrate the guidewire to increase the guidewire's ability to pierce and cross the vascular occlusion. Accordingly, the present disclosure also contemplates directing the laser light energy emitted by the emitter into the liquid medium in a direction which causes the liquid medium to propagate laser-induced pressure waves toward the guidewire lumen and/or guidewire such that the laser-induced pressure waves excite and vibrate the guidewire.

Although the method illustrated in FIG. 15 depicts steps 1505 through 1550 of method 1500 as being performed serially, any or all of the steps within the method 1500 may in any order and/or in parallel with any of the other steps. For example, certain steps can be performed without performing other steps. Upon completing step 1535 and/or step 1540, the combined laser catheter and sheath can optionally be repositioned within the vasculature and adjacent another portion thereof. Similarly, upon completing step 1535 and/or step 1540, the emitter(s) can optionally be repositioned within the sheath. The sheath can be repositioned within the vasculature and/or the emitter(s) can be repositioned within the sheath.

Similar to how the laser catheter assembly 1208 and the sheath assembly 1204 of FIGS. 12-14 can replace the catheter 170 and sheath 250 shown in FIGS. 8G, 8H and FIG. 8H' to perform the method of FIG. 15, which is similar to the method of FIGS. 9A and 9B, to perform an atherectomy followed by disruption of the remaining portion of the vasculature occlusion, the laser catheter assembly 1208 and the sheath assembly 1204 of FIGS. 12-14, particularly the laser catheter 1280 and the sheath 1212, respectively, can replace the catheter 170 and sheath 250 shown in FIG. 10C to perform the method of FIG. 15, which is similar to the method of FIGS. 9A and 9B, to perform the method of depicted in FIG. 11. As set forth above, FIG. 11 illustrates a method using a kit 1200 to generate laser-induced pressure waves to treat the calcium deposits in the tissue (e.g., media) and/or tissue layers (e.g., media layer) of the blood vessel by disrupting the calcium deposits to increase vasculature compliance, thereby increasing blood flow therethrough.

Referring to FIGS. 17 and 17A, there is depicted an outer sheath assembly 1704 for a kit (not shown) that may further include a bifurcate (such as the bifurcate or Y connector) and a laser catheter assembly (such as the laser catheter assembly 1208). The sheath assembly 1704 may include a Luer fitting 1720 at a proximal end portion for detachably coupling to the bifurcate. The sheath assembly 1704 may further include a distal end portion 1740 and an outer sheath 1712 having a working length of about between 50 cm and 200 cm, including 140 cm, and a lumen 1724 extending between such ends. The outer sheath 1712 of FIGS. 17, 17A and 18 may be used in alternative to outer sheath 1212 in FIGS. 12 and 13 discussed hereinabove.

Referring now to FIGS. 17A and 18, there are depicted an enlarged view of the distal end portion 1740 of the sheath assembly 1704 and a partially exploded view illustrating various layers of the sheath 1712, respectively. The distal end portion 1740 of the sheath assembly 1704 may include a tapered tip 1776, an outer band 1772 disposed proximally from the tapered tip 1776 and disposed about the distal end of the sheath 1712, and the distal end of the sheath 1712.

The tapered tip 1776 may be constructed of various materials, such as nylon, pebax, polysulfone, high-density polyethylene (HDPE), low-density polyethylene (LDPE), ultra-high-molecular-weight polyethylene (UHMWPE), polypropylene, polyolefins, carbothane, polyurethane, Suralyn, ionomers, Estane, expanded polytetrafluoroethylene (EPTFE), polytetrafluoroethylene (PTFE), or fluorinated ethylene propylene (FEP). The tapered tip 1776 may be formed integrally with the sheath 1712 or as a separate component. The circumference of the tapered tip 1776 may taper radially inward from its proximal end to its distal end between 1 degree and 10 degrees, such as 1, 2, 3, 4, 5, 6, 7, 8, 9 or 10 degrees. It may be preferable for the tapered tip 1776 to have an inner diameter that facilitates sealing the interface between the sheath assembly 1704 and the laser catheter assembly such that the escape of the liquid medium at the distal end of the kit is minimized or reduced. If a 6 French sized laser catheter of the laser catheter assembly has an outer diameter of about 0.069 inches, then it may be preferable for the inner diameter of the tapered tip 1776 to be about 0.058 inches. If a 7 French sized laser catheter of the laser catheter assembly is to be used, it may be preferable for the inner diameter of the tapered tip 1776 to be about 0.071 inches. If an 8 French sized laser catheter of the laser catheter assembly is to be used, it may be preferable for the inner diameter of the tapered tip 1776 to be about 0.082 inches.

The outer band 1772, which may also be referred to as a marker band, may be constructed of a highly radiopaque material, such as platinum iridium alloy or polymers doped with radiopaque materials such as barium sulfate, bismuth subcarbonate, bismuth trioxide, or tungsten.

Referring specifically to FIG. 18, the sheath 1712 includes an outer jacket 1778 that covers the outer band 1772 (not shown). The sheath 1712 further includes an attenuating member 1768 radially or concentrically disposed within the outer band 1772, an intermediate layer 1770 radially or concentrically disposed within the attenuating member 1768, and an inner liner 1775 radially or concentrically disposed within the intermediate layer 1770. Proximally from the outer band 1772, the sheath 1712 includes a similar structure. Specifically, the attenuating member 1768 is radially or concentrically disposed within the outer jacket 1778, the intermediate layer 1770 is radially or concentrically disposed within the attenuating member 1768, and the inner liner 1775 is radially or concentrically disposed within the intermediate layer 1770. In some embodiments, the attenuating member 1768 is only present at the distal end portion 1740. Assuming that the length of the sheath 1712 is 140 centimeters, the distal end portion 1740 that includes the attenuating member 1768 may be between 0.010 and 10.0 centimeters in length, which represents between 0.05 and 20.0 percent of the length of the sheath 1712.

The outer jacket 1778 may be constructed of various materials, such as nylon, pebax, polysulfone, HDPE, LDPE, UHMWPE, polypropylene, polyolefins, carbothane, polyurethane, Suralyn, ionomers, Estane, EPTFE, PTFE, or FEP. If a 6 French sized laser catheter of the laser catheter assembly is to be used, the outer jacket 1778 may provide the sheath 1712 with an outer diameter between 0.058 and 0.098 inches, such as 0.058, 0.062, 0.066, 0.070, 0.074. 0.078, 0.082, 0.086, 0.090, 0.094, and 0.098 inches. If a 7 French sized laser catheter of the laser catheter assembly is to be used, the outer jacket 1778 may provide the sheath 1712 with an outer diameter between 0.071 and 0.111 inches, such as 0.071, 0.075, 0.079, 0.083, 0.087, 0.091, 0.095, 0.099, 0.103, 0.107, and 0.111 inches. If an 8 French sized laser catheter of the laser catheter assembly is to be used, the outer jacket 1778 may provide the sheath 1712 with an outer diameter between 0.082 and 0.122 inches, such as 0.082, 0.086, 0.090, 0.094, 0.098, 0.102, 0.106, 0.110, 0.114, 0.118, and 0.122 inches. The outer jacket 1778 may have a wall thickness between 0.0015 and 0.0035 inches, such as 0.0015, 0.0017, 0.0019, 0.0021, 0.0023, 0.0025, 0.0027, 0.0029, 0.0031, 0.0033, and 0.0035.

The attenuating member 1768 is a braided structure 1768. The braided structure 1768 may include between 4 and 28 carriers and more particularly between 12 and 20 carriers, such as 12, 13, 14, 15, 16, 17, 18, 19, or 20 carriers. Each carrier may include between 1 and 10 wires, such as 1, 2, 3, 4, 5, 6, 7, 8, 9, and 10 wires. Each wire may have a cross-sectional height between 0.0005 and 0.005, such as 0.0005, 0.0007, 0.0009, 0.001, 0.002, 0.003, 0.004, and 0.005. Each wire may be a flat wire having a cross-sectional width between 0.0005 and 0.003, such as 0.0005, 0.0007, 0.0009, 0.001, 0.002, and 0.003. Each wire may be constructed of stainless steel, such as 304 stainless steel, or other type of metal or metal alloy. The braided structure 1768 may have a braid density between 20 and 100 picks per inch (PPI) and particularly between 40 and 80 PPI, such as 40, 44, 48, 52, 56, 60, 64, 68, 72, 76, and 80 PPI. The relationship between the open area and the closed area (or the ratio of the open area to the overall area) within the braided structure 1768 should be such that a sufficient amount of laser-induced pressure waves pass through the braided structure 1768, and the open area should allow the laser-induced pressure waves to pass therethrough, while also limiting the size of the vapor bubbles that can form on the exterior of the sheath 1712. The braided structure 1768 may have an open area between 45 percent and 85 percent, and possibly between 55 percent and 75 percent, such as 55, 56, 57, 58, 59, 60, 61, 62, 63, 64, 65, 66, 67, 68, 69, 70, 71, 72, 73, 74, or 75 percent. The open area of the braided structure depends on the braid density, the number of carriers in the braided structure 1768, the number of wires in each carrier, and the dimensions of the wires.

The intermediate layer 1770 may be constructed of various materials, such as nylon, pebax, polysulfone, HDPE, LDPE, UHMWPE, polypropylene, polyolefins, carbothane, polyurethane, Suralyn, ionomers, Estane, EPTFE, PTFE, or FEP. The intermediate layer 1770 may have a wall thickness between 0.0005 and 0.0025 inches, such as 0.0005, 0.0007, 0.0009, 0.0011, 0.0013, 0.0015, 0.0017, 0.0019, 0.0021, 0.0023, and 0.0025.

The inner liner 1775 may be constructed of various materials, such as a polyamide or a fluoropolymer, specifically Neoflon FEP NP-101 available from Daikin America. If a 6 French sized laser catheter of the laser catheter assembly is to be used, the inner liner 1775 may provide the sheath 1712 with an inner diameter between 0.040 and 0.080 inches, such as 0.044, 0.048, 0.052, 0.056, 0.060, 0.064, 0.068, 0.072, 0.076 and 0.080 inches. If a 7 French sized laser catheter of the laser catheter assembly is to be used, the inner liner 1775 may provide the sheath 1712 with an inner diameter between 0.053 and 0.093 inches, such as 0.053, 0.057, 0.061, 0.065, 0.069, 0.073, 0.077, 0.081, 0.085, 0.089, and 0.093 inches. If an 8 French sized laser catheter of the laser catheter assembly is to be used, the inner liner 1775 may provide the sheath 1712 with an inner diameter between 0.064 and 0.104 inches, such as 0.064, 0.068, 0.072, 0.076, 0.080, 0.084, 0.088, 0.092, 0.096, 0.100, and 0.104 inches. The inner liner 1796 may have a wall thickness between 0.0005 and 0.0025 inches, such as 0.0005, 0.0007, 0.0009, 0.0011, 0.0013, 0.0015, 0.0017, 0.0019, 0.0021, 0.0023, and 0.0025.

Kits including the sheath assembly 1704 can be used to perform any of the methods described herein. In some embodiments, the sheath assembly 1704 is capable of resisting damage for a duration of at least 1, 2, 3, 4 or 5 minutes (or any duration therebetween) when laser pulses are emitted by a laser catheter during performance of such methods. More specifically, the sheath 1704 is capable of resisting damage when using Spectranetics 1.4, 1.7, and 2.0 Turbo-Elite^{™} and 2.0 Turbo-Power^{™} laser catheters to emit laser pulses at 60 fluence and 25 Hz into a contrast medium of fifty percent (50%) Optiray 320 contrast with saline, infused to the sheath 1704 at 5 mL/min. To facilitate this capability, one or both of the outer jacket 1778 and the intermediate layer 1770 may be integrally formed with the braided structure 1768 (that is, formed in manner that causes one or both of the outer jacket 1778 and the intermediate layer 1770 to occupy at least some of the open area of the braided structure). For example, the inner liner 1775 may be extruded, the intermediate layer 1770 may be over-extruded on the inner liner 1775, the braided structure 1768 may be positioned over the intermediate layer 1770, and the outer jacket 1778 may be over-extruded on the braided structure 1768. The sheath 1712 is laminated, coupled to the Luer fitting 1720, and the outer jacket 1778 may be stripped at the distal end of the sheath 1712.

In comparison to conventional unreinforced and/or reinforced sheaths, such as in comparison to sheath assemblies that include various or multiple laminated layers or sheath assemblies using other manufacturing processes, the sheath assembly 1704 disclosed and discussed herein with respect to FIGS. 17-18 is susceptible to less damage when used in conjunction with a laser catheter and laser pulses are emitted into a contrast medium within such sheath assemblies over short or extended durations, particularly durations in which continues emission of laser pulses occurs for more than 20 seconds, 40 seconds, 1 minute and/or 2 minutes. Minimizing and/or preventing the sleeve from such potential damage, such as bulging, splitting, or delaminating (in the event the sleeve comprises multiple layers), forming a whole within the sleeve, in turn, reduces the possibility of a surgeon experiencing increased difficulty while translating the sleeve through the patient's vasculature and/or translation relative to the laser catheter.

Referring to FIG. 19, there is depicted an enlarged cross-sectional view of an alternative configuration of an outer sheath 1912. The outer sheath 1912 shown in FIG. 19 differs from the sheath 1712 shown in FIG. 18 because the sheath 1712 shown in FIG. 18 includes one attenuating member 1768 and one intermediate layer 1770 disposed between the outer jacket 1778 and the inner liner 1775, but the outer sheath 1912 shown in FIG. 19 includes a plurality of pairs of attenuating members 1968 and intermediate layers 1970 between the outer jacket 1978 and the inner liner 1996. That is, the outer sheath 1912 shown in FIG. 19 includes a plurality of alternating attenuating members 1968 and intermediate layers 1970 between the outer jacket 1978 and the inner liner 1996, wherein the attenuating members 1968 have a higher density relative to the intermediate layers 1970.

As discussed above herein, smaller-sized blood vessels require a laser catheter having a reduced diameter for vessel entry, and the smaller-sized laser catheters may have limitations as to the amount of energy that can transfer. That is, the reduced sized laser catheters may have less optical fibers or smaller diameter optical fibers in comparison to larger sized laser catheters, thereby limiting the amount of energy that the reduced sized laser catheters can transfer before causing damage to the optical fibers. And if the optical fibers attempt to transfer too much energy, they will be damaged.

The amount of energy transmitted by the laser-induced pressure waves to the blood vessel (and the calcium included therein) is proportional to the amount of energy transmitted by the optical fibers to the liquid medium within the outer sheath. That is, the more energy transmitted by the optical fibers to the liquid medium, the greater the amount of energy transmitted by the laser-induced pressure waves to the blood vessel will be. Also, the less energy transmitted by the optical fibers to the liquid medium, the lesser the amount of energy transmitted by the laser-induced pressure waves to the blood vessel will be. Because smaller sized laser catheters may have less optical fibers or smaller diameter optical fibers, the amount of energy transferred by the optical fibers to the liquid medium, the amount of energy transmitted by the laser-induced pressure waves to the blood vessel is limited, thereby potentially limiting the ability to disrupt calcium in the blood vessel.

One way to compensate for the reduction in energy transferred through and emitted by the laser catheter includes increasing the amount of contrast in the liquid medium within the outers sheath. Transmitting pulses of light energy into the liquid medium produces vapor bubbles. Upon emitting light from an emitter, such as a laser catheter, within a sheath that contains an absorptive liquid medium, vapor bubbles may be produced within the interior of the sheath and/or exterior to the sheath. But increasing the amount of contrast may create an undesirably sized vapor bubble, either within or on the outside of the sheath, thereby potentially damaging the blood vessel.

Again, the outer sheath 1912 depicted in FIG. 19 includes a plurality of pairs of attenuating members 1968 and intermediate layers 1970 between the outer jacket 1978 and the inner liner 1996. That is, the outer sheath 1912 shown in FIG. 19 includes a plurality of alternating attenuating members 1968 and intermediate layers 1970 between the outer jacket 1978 and the inner liner 1996, wherein the attenuating members 1968 have a higher density relative to the intermediate layers 1970. The plurality of alternating attenuating members 1968 and intermediate layers 1970 increase(s) the reflective index of the pressure waves transmitted to the blood vessel without increasing the size of the undesirable vapor bubble. The plurality of alternating attenuating members 1968 and intermediate layers 1970 creates a composite structure that mimics anisotropic metamaterial to increase the amplitude of the pressure waves that transmit through the sheath because the attenuating members 1968 are harder than the intermediate layers 1970, and alternating hard and soft layers increase the amplitude of the pressure wave as it transmits through the alternating hard and soft layers.

Continuing to refer to FIG. 19, the outer sheath 1912 includes an outer jacket 1978, an inner liner 1996 and a plurality of alternating intermediate layers 1970 and attenuating members 1968 radially or concentrically disposed between the outer jacket 1978 and the inner liner 1996. The outer jacket 1978 and the inner liner 1996 in FIG. 19 may be constructed of the same materials as the outer jacket 1778 and the inner liner 1796 in FIG. 18, respectively. The outer jacket 1978 and the inner liner 1996 in FIG. 19 may be the same, similar or smaller sizes (e.g., smaller or reduced thickness) in comparison to the outer jacket 1778 and the inner liner 1776 in FIG. 18, respectively. The intermediate layers 1970 and attenuating members 1968 in FIG. 19 may be constructed of the same materials as the intermediate layers 1770 and attenuating members 1768 in FIG. 18, respectively. The intermediate layers 1970 and attenuating members 1968 in FIG. 19 may be the same, similar or smaller sizes or thicknesses in comparison the intermediate layers 1770 and attenuating members 1768 in FIG. 18, respectively.

Although the intermediate layers 1970 and attenuating members 1968 in FIG. 19 may have the same or similar thickness in comparison the intermediate layers 1770 and attenuating members 1768 in FIG. 18, respectively, it may be preferable for the intermediate layers 1970 and attenuating members 1968 in FIG. 19 to be smaller sized (e.g., smaller or reduced thickness) in comparison the intermediate layers 1770 and attenuating members 1768 in FIG. 18, respectively, because it is preferable to increase the number of intermediate layers 1970 and attenuating members 1968 while maintaining the overall diameter of the outer sheath 1912 in FIG. 19 the same as the overall size (e.g., diameter) of the outer sheath in FIG. 18. One of the keys to increasing the amplitude of the pressure waves that transmit through the sheath and minimizing the size of the undesirable vapor bubble is to increase the number of layers of pairs of alternating attenuating members 1968 and intermediate layers 1970 to create a composite structure that mimics anisotropic metamaterial.

Regarding the overall size of the outer sheath 1912, it may be desirable to use a 4 French, 5 French, 6 French, 7 French or 8 French sized catheter in the vasculature below the knee of the patient. In general, it is desirable for the alternating attenuating members 1968 to have a relatively higher density in comparison to the intermediate layers 1970, which will have a lower density in comparison to the attenuating members 1968. With respect to materials for catheters, such as polymers, elastomers, rubbers and plastics, density may be correlated to hardness. For the purposes of this disclosure, assuming that the intermediate layer and the attenuating member made of different types of these materials and the intermediate layer and the attenuating member are solid, the higher the density that the material is, the higher the hardness will likely be, and the lower the density that the material is, the lower the hardness will likely be. In other words, density and hardness for catheter materials are directly related-as one increases so does the other, and as one decreases so does the other. So, it is desirable to alternate a harder layer (e.g., the attenuating members 1968) with a relatively softer layer (e.g., intermediate layers 1970). Notwithstanding the foregoing, for the purposes of this disclosure, it shall be understood that if an attenuating member 1968 is constructed of metal and the intermediate layer 1970 is constructed of a polymer, elastomer, rubber or plastic, then the attenuating member 1968 shall be considered to have a greater density than the intermediate layer 1970, even if the attenuating member 1968 has openings, is porous or is coiled, such as the configurations illustrated in FIGs. 5 and 5A-5F.

Hardness may be tested using a durometer, which is a type of gauge to measure the resistance to surface penetration. There are several scales of durometer, used for materials with different properties. The two common scales, using slightly different measurement systems, are the ASTM D2240 type A and type D scales. The A scale is for softer ones, while the D scale is for harder ones. Higher numbers on the scales indicate a greater resistance to indentation and thus harder materials. Lower numbers indicate softer, more flexible materials, while higher numbers indicate harder and typically more durable materials.

Along with the number there is usually an alpha scale rating with "A" being softer materials and "D" for harder materials. This is scale is typically used for rubbers/elastomers and softer plastics such as polyolefins, fluoropolymers, and vinyls. The hardness value is determined by the penetration of the durometer indenter foot into the sample. Shore A is usually used for flexible materials and Shore D is used for semi-flexible materials.

Although Shore A is one scale, and Shore D is another scale, there may be overlap between these two scales. For example, the Shore A scale may have a hardness of 0 to 100, and the Shore D scale may have a hardness of 0 to 100, but a range of Shore 60A to Shore 100A may overlap with a range of Shore 0D to Shore 60D. That is, a hardness range of Shore 40A overlaps with a hardness range of Shore 60D.

Another hardness scale is the Rockwell scale. Rockwell hardness is generally chosen for 'harder' plastics such as nylon, polycarbonate, polystyrene, and acetal. Three common Rockwell scales are Rockwell A, Rockwell B, and Rockwell C. As with the Shore scale, a higher number indicates a harder material.

Although the Shore scale(s) is one scale, and the Rockwell scale(s) is another scale, there may be overlap between these two scales. For example, the Shore D scale may have a hardness of 0 to 100, and the Rockwell B scale may have a hardness of 0 to 110, but a range of Shore 75D to Shore 100D may overlap with a range of Rockwell 0B to Rockwell 40B. That is, a hardness range of Shore 25D overlaps with a hardness range of Rockwell 40B. However, due to differences in methods of measurement, it may not be possible to directly interconvert between the scales.

If an outer jacket 1978 is included in the construction of the outer sheath 1912, it may be desirable for the durometer and/or density of the outer jacket 1978 to be the same or relatively similar to the durometer and/or density of the intermediate layer 1970 or it may be desirable for the durometer and/or density of the outer jacket 1978 to be between the durometers and/or densities of the attenuating members 1968 and intermediate layers 1970. That is, it is desirable for the durometer and/or density of the outer jacket 1978 to be less than the durometer and/or densities of the attenuating members 1968, and the durometer and/or density of the outer jacket 1978 may be the same or greater than the durometer and/or density of the intermediate layers 1970.

As mentioned above, one of the keys to increasing the amplitude of the pressure waves that transmit through the sheath and minimizing the size of the undesirable vapor bubble is to increase the number of layers of pairs of alternating attenuating members 1968 and intermediate layers 1970 creates a composite structure that mimics anisotropic metamaterial. And the alternating durometers of the plurality of attenuating members 1968 and intermediate layers 1970 creates this composite structure that amplifies the pressure waves.

For example, it may be desirable for the differences in durometer (or hardness) between the attenuating members 1968 and the intermediate layers 1970 to be a difference of about Shore 5A, Shore 10A, Shore 15A, Shore 20A, Shore 25A, Shore 30A, Shore 35A, Shore 40A, Shore 45A, Shore 50A, Shore 55A, Shore 60A, Shore 65A or Shore 70A. It may be also desirable for the differences in durometer (or hardness) between the attenuating members 1968 and the intermediate layers 1970 to be a difference of about Shore 5D, Shore 10D, Shore 15D, Shore 20D, Shore 25D, Shore 30D, Shore 35D, Shore 40D, Shore 45D, Shore 50D, Shore 55D, Shore 60D, Shore 65D or Shore 70D. It may also be desirable for the differences in durometer (or hardness) between the attenuating members 1968 and the intermediate layers 1970 to be a difference of about Rockwell 5B, Rockwell 10B, Rockwell 15B, Rockwell 20B, Rockwell 25B, Rockwell 30B, Rockwell 35B or Rockwell 40B.

Because the attenuating member 1968 may be constructed of a material the hardness of which may be better suited to be determined using one scale, and the intermediate layer 1970 may be constructed of a different material the hardness of which may be better suited to be determined using a different scale, then the hardness difference between materials may be determined using two or more scales. For example, if the attenuating member 1968 has a hardness of Shore 80D and the intermediate layer has a hardness of Shore 85A, which is similar to Shore 35D, then the difference in hardness is about Shore 45D. Additionally, if the attenuating member 1968 has a hardness of Shore 90D and the intermediate layer has a hardness of Shore 40A, and there is no overlap between the Shore A scale (which has a range of 0-100) at the relevant hardness and the Shore D scale, then the difference in hardness is Shore 60A plus Shore 30D (which has range of 0-100 and Shore 100A overlaps at Shore 60D).

The attenuating member1968 may be between Shore hardness 80D and Rockwell 80B, and potentially more desirable to be between Rockwell 20B and Rockwell 70B. It may be desirable for the durometer of the intermediate layers 1970 to be between Shore 20D and Shore 80D, and potentially more desirable to be between Shore hardness 35D and Shore 75D. It may be desirable for the durometer of the outer jacket 1978 to be similar to the intermediate layer 1970.

The densities of the attenuating member1968 and the intermediate layer 1970 may be different solely due to the differences in materials while the thicknesses for the attenuating member1968 and the intermediate layer 1970 are substantially the same. Additionally or alternatively, the densities of the attenuating member1968 and the intermediate layer 1970 may be different due to the differences in both the materials and thicknesses for the attenuating member1968 and the intermediate layer 1970.

Referring again to FIG. 19, the outer jacket 1978 may be constructed of various materials, such as nylon, pebax, polysulfone, HDPE, LDPE, UHMWPE, polypropylene, polyolefins, carbothane, polyurethane, Suralyn, ionomers, Estane, EPTFE, PTFE, or FEP. If a 6 French sized laser catheter of the laser catheter assembly is to be used, the outer jacket 1978 may provide the sheath 1912 with an outer diameter between 0.058 and 0.098 inches, such as 0.058, 0.062, 0.066, 0.070, 0.074. 0.078, 0.082, 0.086, 0.090, 0.094, and 0.098 inches. If a 7 French sized laser catheter of the laser catheter assembly is to be used, the outer jacket 1978 may provide the sheath 1912 with an outer diameter between 0.071 and 0.111 inches, such as 0.071, 0.075, 0.079, 0.083, 0.087, 0.091, 0.095, 0.099, 0.103, 0.107, and 0.111 inches. If an 8 French sized laser catheter of the laser catheter assembly is to be used, the outer jacket 1978 may provide the sheath 1912 with an outer diameter between 0.082 and 0.122 inches, such as 0.082, 0.086, 0.090, 0.094, 0.098, 0.102, 0.106, 0.110, 0.114, 0.118, and 0.122 inches. The outer jacket 1978 may have a wall thickness between 0.0002 and 0.0035 inches, such as 0.0002, 0.0004, 0.0006, 0.0008, 0.001, 0.0012, 0.0015, 0.0017, 0.0019, 0.0021, 0.0023, 0.0025, 0.0027, 0.0029, 0.0031, 0.0033, and 0.0035.

The attenuating members 1968 may be a solid or porous structure such as those described in this disclosure, including those constructed of various metallic and polymeric materials. If the attenuating members 1968 are porous, they may be a braided or coiled structure. If the attenuating members 1968 are braided, the braided structure may include between 4 and 28 carriers and more particularly between 12 and 20 carriers, such as 12, 13, 14, 15, 16, 17, 18, 19, or 20 carriers. Each carrier may include between 1 and 10 wires, such as 1, 2, 3, 4, 5, 6, 7, 8, 9, and 10 wires. Each wire may have a cross-sectional height between 0.0002 and 0.005 inches, such as 0.0002, 0.0003, 0.0005, 0.0007, 0.0009, 0.001, 0.002, 0.003, 0.004, and 0.005 inches. Each wire may be a flat wire having a cross-sectional width between 0.0002and 0.003 inches, such as 0.0002, 0.0003, 0.0005, 0.0007, 0.0009, 0.001, 0.002, and 0.003 inches. Each wire may be constructed of stainless steel, such as 304 stainless steel, or other type of metal or metal alloy. The braided structure may have a braid density between 20 and 200 picks per inch (PPI) and particularly between 40 and 80 PPI, such as 40, 44, 48, 52, 56, 60, 64, 68, 72, 76, and 80 PPI. The relationship between the open area and the closed area (or the ratio of the open area to the overall area) within the braided structure 1968 should be such that a sufficient amount of laser-induced pressure waves pass through the braided structure 1968, and the open area should allow the laser-induced pressure waves to pass therethrough, while also limiting the size of the vapor bubbles that can form on the exterior of the sheath 1912. The braided structure 1968 may have an open area between 45 percent and 85 percent, and possibly between 55 percent and 75 percent, such as 55, 56, 57, 58, 59, 60, 61, 62, 63, 64, 65, 66, 67, 68, 69, 70, 71, 72, 73, 74, or 75 percent. The open area of the braided structure depends on the braid density, the number of carriers in the braided structure, the number of wires in each carrier, and the dimensions of the wires.

The thickness of the attenuating members 1968 may be between 0.0002 and 0.0030 inches, such as 0.0002, 0.0005, 0.00075, 0.0010, 0.0015, 0.0020, 0.0025, and 0.0030 inches. Each attenuating member 1968 in the outer sheath 1912 may have the same (or similar) or different thickness and porosity. For example, each attenuating member 1968 in the outer sheath 1912 may increase in size or decrease in size as the attenuating members progress from the inner liner 1996 to the outer jacket 1978.

Similar to the attenuating members 1968, the intermediate layers 1970 may have the same (or similar) or different thickness. For example, each intermediate layer 1970 may increase or decrease proportionately to the adjustment in thickness of the attenuating member 1968 in the outer sheath 1912 as the attenuating members progress from the inner liner 1996 to the outer jacket 1978. Or each intermediate layer 1970 may increase or decrease inversely in thickness to the adjustment in thickness of the attenuating member 1968 in the outer sheath 1912 as the attenuating members progress from the inner liner 1996 to the outer jacket 1978.

The intermediate layers 1970 may be constructed of various materials, such as nylon, pebax, polysulfone, HDPE, LDPE, UHMWPE, polypropylene, polyolefins, carbothane, polyurethane, Suralyn, ionomers, Estane, EPTFE, PTFE, or FEP. The intermediate layers 1970 may have a wall thickness between 0.0002 and 0.0030 inches, such as 0.0002, 0.0005, 0.00075, 0.0010, 0.0015, 0.0020, 0.0025, and 0.0030 inches.

The inner liner 1996 may be constructed of various materials, such as a polyamide or a fluoropolymer, specifically Neoflon FEP NP-101 available from Daikin America, PTFE, FEP, PFA, ETFE, EFEP. If a 6 French sized laser catheter of the laser catheter assembly is to be used, the inner liner 1996 may provide the sheath 1912 with an inner diameter between 0.040 and 0.080 inches, such as 0.044, 0.048, 0.052, 0.056, 0.060, 0.064, 0.068, 0.072, 0.076 and 0.080 inches. If a 7 French sized laser catheter of the laser catheter assembly is to be used, the inner liner 1996 may provide the sheath 1912 with an inner diameter between 0.053 and 0.093 inches, such as 0.053, 0.057, 0.061, 0.065, 0.069, 0.073, 0.077, 0.081, 0.085, 0.089, and 0.093 inches. If an 8 French sized laser catheter of the laser catheter assembly is to be used, the inner liner 1996 may provide the sheath 1912 with an inner diameter between 0.064 and 0.104 inches, such as 0.064, 0.068, 0.072, 0.076, 0.080, 0.084, 0.088, 0.092, 0.096, 0.100, and 0.104 inches. The inner liner 1996 may have a wall thickness between 0.0002 and 0.0025 inches, such as 0.0005, 0.0007, 0.0009, 0.0011, 0.0013, 0.0015, 0.0017, 0.0019, 0.0021, 0.0023, and 0.0025.

Although FIG. 19 illustrates the pattern of a pair of attenuating members and intermediate layers arranged such that the attenuating members 1968 is radially outward of the intermediate layers 1970, the pattern of a pair of attenuating members and intermediate layers may be arranged such that the attenuating members 1968 is radially inward of the intermediate layers 1970. Continuing to refer to FIG. 19, an outer jacket 1978 and an inner liner 1996, the outer sheath 1912 may exclude either the outer jacket 1978, an inner liner 1996 or both because doing so may decrease the overall cross-sectional diameter and/or size of the outer sheath 1912 and/or allowing the outer sheath 1912 to have a larger number of alternating pairs of attenuating members 1968 and intermediate layers 1970.

The outer sheath 1912 depicted in FIG. 19 can replace any sheath discussed in this disclosure and/or depicted in any of the figures. The outer sheath depicted in FIG. 19 can replace any sheath used to perform any method discussed and/or illustrated in the representative flow diagram to treat a subject. For example, the outer sheath 1912 depicted in FIG. 19 can replace the sheath 250 depicted in FIGs. 3A-3C and be used to perform the method illustrated in the representative flow diagram of FIG. 4 to treat a subject. The outer sheath 1912 depicted in FIG. 19 can replace the sheath 250" depicted in FIGs. 5 and 5A-5F and be used to perform the method illustrated in the representative flow diagram of FIG. 6 to treat a subject. The outer sheath 1912 depicted in FIG. 19 can replace the sheath used to perform the method illustrated in the representative flow diagram of FIG. 9A and 9B to treat a subject. The outer sheath 1912 depicted in FIG. 19 can replace the sheath used to perform the method illustrated in the representative flow diagram of FIG. 11 to treat a subject. The outer sheath 1912 depicted in FIG. 19 can replace the sheath 1212 depicted in FIGs. 12, 12A, 12B, 12B', 13, 13A, 13B, 13C and 13C' and be used to perform the method illustrated in the representative flow diagram of FIG. 15 to treat a subject. The outer sheath 1912 depicted in FIG. 19 can replace the sheath 1212 depicted in FIGs. 17, 17A and 18.

Replacing the sheaths discussed elsewhere in this disclosure with the outer sheath 1912 depicted in FIG. 19 should increase the amplitude of the pressure waves transmitted through the sheath. The plurality of pairs of attenuating members 1968 and intermediate layers 1970 increase(s) the reflective index of the pressure waves transmitted to the blood vessel without increasing the size of the undesirable vapor bubble, thereby potentially increasing the overall effectiveness of the therapeutic method while increasing patient safety.

Although a large portion of this disclosure includes a discussion of laser ablation catheters used in conjunction with a sheath assembly to perform CAD and PAD procedures, other the laser ablation catheter and sheath assembly may be used to perform other types of medical and/or surgical procedures. Laser catheters typically transmit laser energy through optical fibers housed in a relatively flexible tubular catheter inserted into a body lumen, such as a blood vessel, ureter, fallopian tube, cerebral artery and the like to remove obstructions or restrictions in the lumen. Catheters used for laser angioplasty and other procedures may have a central passageway or tube which receives a guide wire inserted into the body lumen (for example, vascular system) prior to catheter introduction. The guide wire facilitates the advancement and placement of the catheter to the selected portion(s) of the body lumen for laser ablation of tissue.

The present disclosure, in various aspects, embodiments, and configurations, includes components, systems and/or apparatus substantially as depicted and described herein, including various aspects, embodiments, configurations, sub combinations, and subsets thereof, and additionally methods and processes are described as exemplification. Those of skill in the art will understand how to make and use the various aspects, aspects, embodiments, and configurations, after understanding the present disclosure. The present disclosure, in various aspects, embodiments, and configurations, includes providing devices and processes in the absence of items not depicted and/or described herein or in various aspects, embodiments, and configurations hereof, including in the absence of such items as may have been used in previous devices or processes, for example, for improving performance, achieving ease and\or reducing cost of implementation.

The foregoing discussion of the disclosure has been presented for purposes of illustration and description. In the foregoing Detailed Description for example, various features of the disclosure are grouped together in one or more, aspects, embodiments, and configurations for the purpose of streamlining the disclosure. The features of the aspects, embodiments, and configurations of the disclosure may be combined in alternate aspects, embodiments, and configurations other than those discussed above. The method described is given as example. The following claims are hereby incorporated into this Detailed Description, with each claim standing on its own as a separate preferred embodiment of the disclosure.

Moreover, though the description of the disclosure has included description of one or more aspects, embodiments, or configurations and certain variations and modifications, other variations, combinations, and modifications are within the scope of the disclosure, for example, as may be within the skill and knowledge of those in the art, after understanding the present disclosure.

## Claims

1. A catheter system comprising:
a laser catheter comprising a proximal end, a distal end and at least one emitter disposed adjacent the distal end;
a sheath configured to be disposed over the laser catheter and configured to receive a liquid medium, the sheath comprising a proximal end and a distal end, wherein the sheath comprises a first layer forming a lumen, a plurality of pairs of attenuating members and intermediate layers radially exterior the first layer, wherein the attenuating members are constructed of a first material having a first thickness and first durometer, and the intermediate layers are constructed of a second material having a second thickness and a second durometer, wherein the first durometer is greater than the second durometer;
wherein the sheath comprises the plurality of the attenuating members and the intermediate layers in an alternating manner.

2. The catheter system of claim 1, wherein the first thickness and the second thickness are different.

3. The catheter system of claim 1, wherein the first thickness and the second thickness are substantially equal.

4. The catheter system of claim 1, wherein a difference between the first durometer and the second durometer is about Shore 10D.

5. The catheter system of claim 1, wherein a difference between the first durometer and the second durometer is about Shore 20D.

6. The catheter system of claim 1, wherein a difference between the first durometer and the second durometer is about Shore 30D.

7. The catheter system of claim 2,
wherein the laser catheter is configured to be positioned within a vasculature of a subject after locating a calcified portion in a media of a blood vessel of the subject;
wherein the sheath is configured to be positioned over the laser catheter within the vasculature of the subject;
wherein the sheath is configured to be positioned such that the attenuating member is disposed adjacent a portion of the calcified portion in the media of the blood vessel;
wherein the laser catheter is configured to be positioned within the vasculature such that the at least one emitter is positioned within the attenuating member and adjacent the portion of the calcified portion in the media of the blood vessel;
wherein the sheath is configured to introduce a liquid medium into the sheath and to the at least one emitter; and
wherein the emitter is configured to emit a plurality of pulses of light energy from the at least one emitter into the liquid medium, wherein the plurality of pulses of light energy are provided to react with the liquid medium and to generate a plurality of propagating laser-induced pressure waves that disrupt the calcified portion of media, thereby improving the compliance of the blood vessel.

8. The catheter system of claim 7, wherein the sheath is configured to be re-positioned such that the attenuating member is adjacent another calcified portion of the media.

9. The catheter system of claim 7, wherein the laser catheter is configured to be re-positioned within the sheath such that the one or more emitters is adjacent another calcified portion of the media.

10. The catheter system of claim 7, wherein the laser catheter is configured to be re-positioned within the sheath.

11. The catheter system of claim 9 or 10, wherein the laser catheter is configured to be re-positioned within the attenuating member.

12. The catheter system of claim 7, wherein the laser catheter and the sheath are configured to be removed from the vasculature.

13. The catheter system of claim 3, further comprising a guidewire;
wherein the guidewire is configured to be inserted through a vascular occlusion within a vasculature of a subject;
wherein the laser catheter is configured to be introduced into the vasculature and over the guidewire;
wherein the laser catheter is configured to ablate at least a portion of the vascular occlusion;
wherein the sheath is configured to be introduced into the vasculature and over the laser catheter,
wherein the sheath is configured to be positioned within the vasculature such that the attenuating member is disposed radially adjacent a calcified portion within the vasculature;
wherein the laser catheter is configured to be positioned within the vasculature such that the at least one emitter is positioned within the attenuating member and radially adjacent the calcified portion;
wherein the sheath is configured to introduce a liquid medium into the sheath and to the at least one emitter; and
wherein the emitter is configured to emit a plurality of pulses of light energy from the at least one emitter into the liquid medium, wherein the plurality of pulses of light energy are provided to react with the liquid medium and to generate a plurality of propagating laser-induced pressure waves that disrupt the calcified portion.

14. The catheter system of claim 13, wherein the laser catheter is configured to be extended distally of the sheath and to ablate another portion of a second vascular occlusion;
wherein the sheath is configured to be positioned within the vasculature such that the attenuating member is disposed radially adjacent a second calcified portion of the second vascular occlusion;
wherein the laser catheter is configured to be positioned within the vasculature such that the at least one emitter is positioned within the attenuating member and radially adjacent the second calcified portion;
wherein the plurality of pulses of light energy are provided to generate a plurality of propagating laser-induced pressure waves that disrupt the second calcified portion.

## Patentansprüche

1. Kathetersystem, umfassend:
einen Laserkatheter, der ein proximales Ende, ein distales Ende und mindestens einen Emitter umfasst, der an das distale Ende angrenzend angeordnet ist;
eine Hülle, die konfiguriert ist, um über dem Laserkatheter angeordnet zu werden und konfiguriert ist, um ein flüssiges Medium aufzunehmen, wobei die Hülle ein proximales und ein distales Ende aufweist, wobei die Hülle eine erste Schicht umfasst, die ein Lumen bildet, eine Vielzahl von Paaren von Dämpfungselementen und Zwischenschichten radial außerhalb der ersten Schicht, wobei die Dämpfungselemente aus einem ersten Material aufgebaut sind, das eine erste Dicke und eine erste Härte aufweist und die Zwischenschichten aus einem zweiten Material aufgebaut sind, das eine zweite Dicke und eine zweite Härte aufweist, wobei die erste Härte größer als die zweite Härte ist;
wobei die Hülle abwechselnd eine Vielzahl der Dämpfungselementen und der Zwischenschichten umfasst.

2. Kathetersystem nach Anspruch 1, wobei die erste Dicke und die zweite Dicke unterschiedlich sind.

3. Kathetersystem nach Anspruch 1, wobei die erste Dicke und die zweite Dicke im Wesentlichen gleich sind.

4. Kathetersystem nach Anspruch 1, wobei ein Unterschied zwischen der ersten Härte und der zweiten Härte etwa Shore 10D beträgt.

5. Kathetersystem nach Anspruch 1, wobei ein Unterschied zwischen der ersten Härte und der zweiten Härte etwa Shore 20D beträgt.

6. Kathetersystem nach Anspruch 1, wobei ein Unterschied zwischen der ersten Härte und der zweiten Härte etwa Shore 30D beträgt.

7. Kathetersystem nach Anspruch 2,
wobei der Laserkatheter konfiguriert ist, um innerhalb eines Gefäßsystems eines Subjekts, nach dem Orten eines verkalkten Abschnitts in einem Medium eines Blutgefäßes des Subjekts positioniert zu werden;
wobei die Hülle konfiguriert ist, um über dem Laserkatheter innerhalb des Gefäßsystems des Patienten positioniert zu werden;
wobei die Hülle konfiguriert ist, um positioniert zu werden, sodass das Dämpfungselement angrenzend an einen Abschnitt des verkalkten Abschnitts in dem Medium des Blutgefäßes angeordnet ist;
wobei der Laserkatheter konfiguriert ist, um innerhalb des Gefäßsystems positioniert zu werden, sodass der mindestens eine Emitter innerhalb des Dämpfungselements und angrenzend an den Abschnitt des verkalkten Abschnitts in dem Medium des Blutgefäßes positioniert ist;
wobei die Hülle konfiguriert ist, um ein flüssiges Medium in die Hülle und in den mindestens einen Emitter einzuleiten; und
wobei der Emitter konfiguriert ist, um eine Vielzahl von Lichtenergieimpulsen von dem mindestens einen Emitter in das flüssige Medium zu emittieren, wobei die Vielzahl von Lichtenergieimpulsen bereitgestellt werden, um mit dem flüssigen Medium zu reagieren, und um eine Vielzahl von sich ausbreitender laserinduzierter Druckwellen zu erzeugen, die den verkalkten Abschnitt des Mediums aufbrechen, wodurch sich die Nachgiebigkeit des Blutgefäßes verbessert.

8. Kathetersystem nach Anspruch 7, wobei die Hülle konfiguriert ist, um neu positioniert zu werden, sodass das Dämpfungselement an einen anderen verkalkten Abschnitt des Mediums angrenzt.

9. Kathetersystem nach Anspruch 7, wobei der Laserkatheter konfiguriert ist, um innerhalb der Hülle neu positioniert zu werden, sodass sich der eine oder mehrere Emitter an einen anderen verkalkten Abschnitt des Mediums angrenzt.

10. Kathetersystem nach Anspruch 7, wobei der Laserkatheter konfiguriert ist, um innerhalb der Hülle neu positioniert zu werden.

11. Kathetersystem nach Anspruch 9 oder 10, wobei der Laserkatheter konfiguriert ist, um innerhalb des Dämpfungselements neu positioniert zu werden.

12. Kathetersystem nach Anspruch 7, wobei der Laserkatheter und die Hülle konfiguriert sind, um aus dem Gefäßsystem entfernt zu werden.

13. Kathetersystem nach Anspruch 3, das weiter einen Führungsdraht umfasst;
wobei der Führungsdraht konfiguriert ist, um durch einen Gefäßverschluss innerhalb eines Gefäßsystems eines Subjekts hindurch eingeführt zu werden;
wobei der Laserkatheter konfiguriert ist, um über den Führungsdraht in das Gefäßsystem eingeführt zu werden;
wobei der Laserkatheter konfiguriert ist, um mindestens einen Abschnitt des Gefäßverschlusses abzutragen;
wobei die Hülle konfiguriert ist, um in das Gefäßsystem und über den Laserkatheter eingeführt zu werden,
wobei die Hülle konfiguriert ist, um innerhalb des Gefäßsystems positioniert zu werden, sodass das Dämpfungselement radial angrenzend an einen verkalkten Abschnitt innerhalb des Gefäßsystems angeordnet ist;
wobei der Laserkatheter konfiguriert ist, um innerhalb des Gefäßsystems positioniert zu werden, sodass der mindestens eine Emitter innerhalb des Dämpfungselements und radial angrenzend an den verkalkten Abschnitt positioniert ist;
wobei die Hülle konfiguriert ist, um ein flüssiges Medium in die Hülle und in den mindestens einen Emitter einzuleiten; und
wobei der Emitter konfiguriert ist, um eine Vielzahl von Lichtenergieimpulsen von dem mindestens einen Emitter in das flüssige Medium zu emittieren, wobei die Vielzahl von Lichtenergieimpulsen bereitgestellt werden, um mit dem flüssigen Medium zu reagieren, und um eine Vielzahl von sich ausbreitender laserinduzierter Druckwellen zu erzeugen, die den verkalkten Abschnitt aufbrechen.

14. Kathetersystem nach Anspruch 13, wobei der Laserkatheter konfiguriert ist, um sich distal zur Hülle zu erstrecken, und einen weiteren Abschnitt eines zweiten Gefäßverschlusses abzutragen;
wobei die Hülle konfiguriert ist, um innerhalb des Gefäßsystems positioniert zu werden, sodass das Dämpfungselement radial angrenzend an einen zweiten verkalkten Abschnitt des zweiten Gefäßverschlusses angeordnet ist;
wobei der Laserkatheter konfiguriert ist, um innerhalb des Gefäßsystems positioniert zu werden, sodass der mindestens eine Emitter innerhalb des Dämpfungselements und radial angrenzend an den zweiten verkalkten Abschnitt positioniert ist;
wobei die Vielzahl von Lichtenergieimpulsen bereitgestellt werden, um eine Vielzahl von sich ausbreitenden laserinduzierten Druckwellen zu erzeugen, die den zweiten verkalkten Abschnitt aufbrechen.

## Revendications

1. Système de cathéter comprenant :
un cathéter laser comprenant une extrémité proximale, une extrémité distale et au moins un émetteur disposé à proximité de l'extrémité distale ;
une gaine configurée pour être disposée sur le cathéter laser et configurée pour recevoir un milieu liquide, la gaine comprenant une extrémité proximale et une extrémité distale, dans lequel la gaine comprend une première couche formant une lumière, une pluralité de paires d'éléments atténuants et de couches intermédiaires radialement à l'extérieur de la première couche, dans lequel les éléments atténuants sont construits d'un premier matériau présentant une première épaisseur et une première dureté, et les couches intermédiaires sont construites d'un second matériau présentant une seconde épaisseur et une seconde dureté, dans lequel la première dureté est supérieure à la seconde dureté ;
dans lequel la gaine comprend la pluralité des éléments atténuants et des couches intermédiaires de manière alternée.

2. Système de cathéter selon la revendication 1, dans lequel la première épaisseur et la seconde épaisseur sont différentes.

3. Système de cathéter selon la revendication 1, dans lequel la première épaisseur et la seconde épaisseur sont sensiblement égales.

4. Système de cathéter selon la revendication 1, dans lequel la différence entre la première dureté et la seconde dureté est d'environ 10D Shore.

5. Système de cathéter selon la revendication 1, dans lequel la différence entre la première dureté et la seconde dureté est d'environ 20D Shore.

6. Système de cathéter selon la revendication 1, dans lequel la différence entre la première dureté et la seconde dureté est d'environ 30D Shore.

7. Système de cathéter selon la revendication 2,
dans lequel le cathéter laser est configuré pour être positionné dans le système vasculaire d'un sujet après avoir localisé une portion calcifiée dans le milieu d'un vaisseau sanguin du sujet ;
dans lequel la gaine est configurée pour être positionnée sur le cathéter laser à l'intérieur du système vasculaire du sujet ;
dans lequel la gaine est configurée pour être positionnée de telle sorte que l'élément atténuant soit disposé à proximité d'une partie de la portion calcifiée dans le milieu du vaisseau sanguin ;
dans lequel le cathéter laser est configuré pour être positionné dans le système vasculaire de telle sorte que le au moins un émetteur soit positionné dans l'élément atténuant et à proximité de la partie de la portion calcifiée dans le milieu du vaisseau sanguin ;
dans lequel la gaine est configurée pour introduire un milieu liquide dans la gaine et vers le au moins un émetteur; et
dans lequel l'émetteur est configuré pour émettre une pluralité d'impulsions d'énergie lumineuse à partir du au moins un émetteur dans le milieu liquide, dans lequel la pluralité d'impulsions d'énergie lumineuse est destinée à réagir avec le milieu liquide et à générer une pluralité d'ondes de pression induites par laser se propageant et perturbant la portion calcifiée du milieu, améliorant ainsi la compliance du vaisseau sanguin.

8. Système de cathéter selon la revendication 7, dans lequel la gaine est configurée pour être repositionnée de telle sorte que l'élément atténuant soit adjacent à une autre portion calcifiée du milieu.

9. Système de cathéter selon la revendication 7, dans lequel le cathéter laser est configuré pour être repositionné à l'intérieur de la gaine de sorte que les un ou plusieurs émetteurs soient adjacents à une autre portion calcifiée du milieu.

10. Système de cathéter selon la revendication 7, dans lequel le cathéter laser est configuré pour être repositionné à l'intérieur de la gaine.

11. Système de cathéter selon la revendication 9 ou 10, dans lequel le cathéter laser est configuré pour être repositionné à l'intérieur de l'élément atténuant.

12. Système de cathéter selon la revendication 7, dans lequel le cathéter laser et la gaine sont configurés pour être retirés du système vasculaire.

13. Système de cathéter selon la revendication 3, comprenant en outre un fil guide ;
dans lequel le fil guide est configuré pour être inséré à travers une occlusion vasculaire au sein du système vasculaire d'un sujet ;
dans lequel le cathéter laser est configuré pour être introduit dans le système vasculaire et sur le fil guide ;
dans lequel le cathéter laser est configuré pour ablater au moins une partie de l'occlusion vasculaire ;
dans lequel la gaine est configurée pour être introduite dans le système vasculaire et par-dessus le cathéter laser,
dans lequel la gaine est configurée pour être positionnée à l'intérieur du système vasculaire de telle sorte que l'élément atténuant soit disposé radialement à proximité d'une portion calcifiée à l'intérieur du système vasculaire ;
dans lequel le cathéter laser est configuré pour être positionné dans le système vasculaire de telle sorte que le au moins un émetteur soit positionné dans l'élément atténuant et radialement adjacent à la portion calcifiée ;
dans lequel la gaine est configurée pour introduire un milieu liquide dans la gaine et vers le au moins un émetteur; et
dans lequel l'émetteur est configuré pour émettre une pluralité d'impulsions d'énergie lumineuse à partir du au moins un émetteur dans le milieu liquide, dans lequel la pluralité d'impulsions d'énergie lumineuse est destinée à réagir avec le milieu liquide et à générer une pluralité d'ondes de pression induites par laser se propageant et perturbant la portion calcifiée.

14. Système de cathéter selon la revendication 13, dans lequel le cathéter laser est configuré pour être étendu distalement à la gaine et pour ablater une autre partie d'une seconde occlusion vasculaire ;
dans lequel la gaine est configurée pour être positionnée à l'intérieur du système vasculaire de telle sorte que l'élément atténuant soit disposé radialement à proximité d'une seconde portion calcifiée de la seconde occlusion vasculaire ;
dans lequel le cathéter laser est configuré pour être positionné dans le système vasculaire de telle sorte que le au moins un émetteur soit positionné dans l'élément atténuant et radialement adjacent à la seconde portion calcifiée ;
dans lequel la pluralité d'impulsions d'énergie lumineuse sont fournies pour générer une pluralité d'ondes de pression induites par laser se propageant et perturbant la seconde portion calcifiée.
